# EUROPEAN PATENT APPLICATION

(11) **EP 1 826 199 A1**
(43) Date of publication of application: **29.08.2007**
(21) Application number: 06450025.9
(22) Date of filing: 27.02.2006
(51) Int. Cl.: C07C 243/28, C07D 209/12, C07D 207/16, C07D 255/02, C07K 5/08

(54) **Modified amino acids**

(71) Applicant: Technische Universität Wien, 1040 Wien (AT)
(72) Inventor: Jordis, Ulrich, 1180 Vienna (AT); Phopase, Jaywant B., 1180 Vienna (AT)
(74) Representative: Sonn & Partner Patentanwälte

(57) **Abstract**

The present invention provides a compound of the general formula 1, 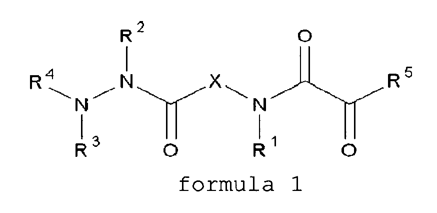 wherein X is the connection between the CO-hydrazine and the NR¹-oxalic acid or ester group, and uses and synthesis methods. These compounds represent amino acid derivatives, wherein the amine group is turned into an acidic group by the oxalic acid group and the carboxylic acid is turned into an amine functionality by the hydrazine group; as well as peptidomimetics comprising the compound and methods for their synthesis.

## Description

The present invention relates to modified amino acids and their use as building blocks for peptide and amino acid mimetics or analogs.

Amino acid derivatives are used or have been used as chemical moieties which mimic the biological function of an amino acid with modified stability, degradation or reactivity properties, especially in peptide mimics such modified amino acids or amino acids analogs are used. For example, amino acid derivatives have applications in the synthesis and manufacture of a wide range of pharmaceutical products and therapeutic agents used for the treatment of human or animal diseases or crop protecting agents such as herbicides, insecticides or fungicides.

One particular aspect in the design of modified peptides is the goal to obtain products that are metabolically stable and still exert their desired properties by providing a three dimensional arrangement of their natural or unnatural amino acid residues for interaction with and/or binding to their biological targets like receptors, enzymes, proteins and other macro- or small molecules. As an example the secondary structure peptidomimetical approach is a rational way to develop novel nonpeptide pharmaceutical agents based upon biologically significant proteinaceous leads (Eguchi et al., Mini-Reviews in Medicinal Chemistry (2002), 2(5): 447-462). Amino acid derivatives include ketones, aldehydes, acetals, esters, ethers, etc. and are designed for increased stability to prolong bioavailability of e.g. a peptide pharmaceutical or to increase its reactivity with a specific target for the targets inhibition.

In some areas lead compounds are derived by testing libraries of natural or unnatural peptides. As typically such leads cannot be used as drugs or other bioactive substances as they are either metabolically unstable or exert other unfavourable physicochemical properties, such leads are then altered in various ways, e.g. by preparing retro, inverso-, and retro-inverso analogs, by introducing additional conformational fixations, insertions or deletions of the original peptide motif, the compounds of the present invention can be used either as building blocks and sub-structures to arrive at biological active moieties or are biologically active themselves (WO 94/05311 A1).

According to the US 5,618,914 B a peptide mimetic is disclosed forming a beta turn motif. Therein modular components or building blocks for the synthesis of the mimetic are provided, which can be assembled to a variety of three dimensionally constrained beta turn motifs. These building blocks are amino acid derivatives, wherein a linker group is ligated to the amino group of the template amino acid.

A peptidomimetic is a small protein-like chain, ring or ring/chain combination that contains both natural and nonnatural amino acids. It is designed and synthesized with the purpose of binding to target proteins in order to induce virous biological effects thus mimicking key interactions in the cell.

One example of such an effect is to induce cancer cells into a form of programmed cell death called apoptosis. All healthy cells in multi-celled organisms are subject to programmed cell death when they are no longer wanted; but cancer cells have the ability to evade apoptosis and the body's attempts to get rid of them. So peptidomimetics are part of the wide effort by researchers, research labs and institutions to create cures for cancer by means of restoring or activating apoptotic pathways in specific cells.

In the WO 2005/103012 A a hydrazino-substituted heterocyclic nitrile amino acid derivative is disclosed, among many other substances, which functions as cysteine protease inhibitor.

Methods for the synthesis of aromatic hydrazines are disclosed in the DE 1150391. These compounds are used as UV absorbents in dyes.

Compounds that mimic the structures and hydrogen-bonding patterns of protein β-sheets, but have not specifically been targeted toward binding proteins, have been reported. Kemp and co-workers described a 2,8-diaminoepindolidione molecular template that mimics the hydrogen-bonding functionality of one edge of a peptide β-strand and have coupled this β-strand mimic to peptides to generate intramolecularly hydrogen-bonded β-sheet like structures (Kemp et al. J. Org. Chem. (1990) 55: 4650-4657).

A beta sheet mimetic is disclosed in the WO 01/14412 and in an article of Nowick et al. (J.Am.Chem.Sec.(2000) 122:7654-7661). Therein the C-alpha atom is replaced by a 5-amino-2-methoxybenzoic acid, wherein the methoxy group forms a hydrogen bond with the amide group thus forming a rigid structure, which imposes the three dimensional structure of flat beta sheet motif on the peptide. This amino acid analogue has been investigated in molecular dynamics simulations to study dimerisation and beta-sheet folding mechanisms (Yu et al., Proteins: Structure, Function, and Bioinformatics (2004) 54: 116-127).

The goal of the present invention is to provide new amino acid mimetics with a broad range of applications, especially as building blocks and substructures in the synthesis of modified peptides to prepare molecules with desired properties.

Therefore a compound of the general formula 1 is provided, 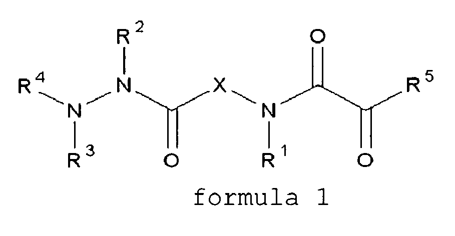 wherein X is the connection between the CO-hydrazine and the NR¹-oxalic acid, oxalic ester or oxalic amide group and is either a bond, 5-20-membered heteroaryl, or an optionally substituted group selected from C₃₋₂₀-cycloalkyl, 3-20-membered heterocyclyl, and linear or branched C₁₋₂₀-alkyl, C₂₋₂₀-alkenyl or C₂₋₂₀-alkinyl; and R⁵ is selected from -OR¹⁰ and -NR¹⁰R¹¹, or R⁵ can cooperate with R² or R³ to form a bond or a 8 to 10 membered heterocyclic ring; and R³ and R⁴ together may constitute a double bond to a group R¹²; R¹, R², R³, R⁴, R¹⁰, R¹¹ and R¹² are optionally substituted and independently selected from H, C₃₋₁₄-cycloalkyl, C₅₋₁₄ aryl, 3-14-membered heterocyclyl or heteroaryl, linear or branched C₁₋₁₄-alkyl, C₂₋₁₄-alkenyl, C₂₋₁₄-alkinyl; and optionally at least two of R¹, R², R³, R⁴ and X can cooperate to form a 3 to 12-membered cycloalkyl or heterocycloalkyl ring; or an ester, amide, salt, stereoisomer or racemate therefrom, with the proviso that in the case of X being CH₂ R³ and R⁴ each are bound by single bonds and R² is selected from H, C₃₋₂₀-cycloalkyl, C₅₋₂₀-aryl, 3-20-membered heterocyclyl or heteroaryl, linear or branched C₂₋₂₀-alkenyl, C₂₋₂₀)-alkinyl, or unsubstituted C₁₋₄-alkyl, and in the case of X being a bond one of R³ or R⁴ forms an amide, and in the case of X being heteroaryl X does not cooperate to form a heterocycloalkyl ring with R³, R⁴ or R⁵, and in the case of X and R¹ cooperating to form a cycloalkyl ring R⁵ does not comprise a further hydrazine group, R² does not form an aromatic ring with neither R³ or R⁴ and neither R³ nor R⁴ comprise sulphur. Preferably, X is part of a 3, 4, 5 or 6 membered ring in the case of X and R₁ forming a cycloalykl ring. These provisos can also be generalized for other compounds of formula 1 in other embodiments. Preferably X is 3, 4, 5, 6, 7, 8, 9, 10 or 10-15 membered. In the case of X being a bond and at least one of R³ or R⁴ forms an amide, the amide is preferably to an acidic group, preferably a protecting group. These building blocks that take the place of one or more amino acids can be used for peptidomimetics, to modify various peptides and proteins. Also comprised are pharmaceutical salts of the compound. An overview of pharmaceutical salts is given in the Handbook of Pharmaceutical Salts: Properties, Selection, and Use, P. H. Stahl and G. Wermuth (editors), publisher: Helvetica Chimica Acta, Zürich 2002.

Any aryl or heteroaryl group is preferably 5-20, more preferred 5-15 or 6 to 10, especially 6 membered, any cycloalkyl or heterocyclyl is preferably 3-20, more preferred 5-15 or 5 to 10, especially 6-8 membered, any alkyl, alkenyl or alkinyl group, optionally main chain hetero substituted, is preferably 2 to 20, more preferred 3-15 or 4 to 10, especially 5-8 membered.

The present invention provides certain novel derivatives of amino acids as shown in a compound of formula 1, wherein in an original template amino acids (exemplified by, but not restricted to, natural or unnatural, optionally substituted, alpha, beta, gamma, etc. to omega amino acids) are in carbocylic aromatic or heterocyclic compounds that are substituted by a carboxylic acid and bear an amino acid group as substituents or as part of the ring, the amino- and carboxylic acid functionalities are formally reversed in such a way that the original basic amino group is substituted by an oxalic acid function, thus rendering this end of the molecule acidic, and the originally acidic end is converted into a hydrazide, thus rendering this end of the molecule basic.

Preferably, in the compounds according to formula 1 the alkyl group is methyl, ethyl, propyl, isopropyl, n-butyl, sec-butyl, tert-butyl, pentyl, 2-pentyl, isopentyl, neopentyl, hexyl, 2-hexyl, 3-hexyl or 3-methylpentyl. Further, the alkenyl group is preferably ethenyl, propenyl, 1-but-3-enyl, 1-pent-3-enyl or 1-hex-5-enyl. The alkynyl group is preferably ethynyl, propynyl, butynyl or pentyn-2-yl. Also preferably, the alkoxy group (or -O-alkyl group) is methoxy, ethoxy, propoxy, isopropoxy, n-butoxy, sec-butoxy, tert-butoxy, pentoxy, isopentoxy, neopentoxy, hexoxy or 3-methylpentoxy. The cycloalkyl group is preferably cyclopropyl, cyclobutyl, cyclopentyl or cyclohexyl.

The aryl group is preferably phenyl, 1-naphthyl, 2-naphthyl, indanyl, indenyl, dihydronaphthyl, tetralinyl or 6,7,8,9-tetrahydro-5H-benzo[a]cycloheptenyl.

The heteroaryl group is preferably pyridinyl, pyrimidinyl, quinolinyl, benzothienyl, indolyl, indolinyl, pyridazinyl, pyrazinyl, isoindolyl, isoquinolyl, quinazolinyl, quinoxalinyl, phthalazinyl, imidazolyl, isoxazolyl, pyrazolyl, oxazolyl, thiazolyl, indolizinyl, indazolyl, benzothiazolyl, benzimidazolyl, benzofuranyl, furanyl, thienyl, pyrrolyl, oxadiazolyl, thiadiazolyl, triazolyl, tetrazolyl, oxazolopyridinyl, imidazopyridinyl, isothiazolyl, naphthyridinyl, cinnolinyl, carbazolyl, beta-carbolinyl, isochromanyl, chromanyl, tetrahydroisoquinolinyl, isoindolinyl, isobenzotetrahydrofuran- yl, isobenzotetrahydrothienyl, isobenzothienyl, benzoxazolyl, pyridopyridinyl, benzotetrahydrofuranyl, benzotetrahydrothienyl, purinyl, benzodioxolyl, triazinyl, phenoxazinyl, phenothiazinyl, pteridinyl, benzothiazolyl, imidazopyridinyl, imidazothiazolyl, dihydrobenzisoxazinyl, benzisoxazinyl, benzoxazinyl, dihydrobenzisothiazinyl, benzopyranyl, benzothiopyranyl, coumarinyl, isocoumarinyl, chromonyl, chromanonyl, pyridinyl-N-oxide, tetrahydroquinolinyl, dihydroquinolinyl, dihydroquinolinonyl, di-hydroisoquinolinonyl, dihydrocoumarinyl, dihydroisocoumarinyl, isoindolinonyl, benzodioxanyl, benzoxazolinonyl, pyrrolyl N-oxide, pyrimidinyl N-oxide, pyridazinyl N-oxide, pyrazinyl N-oxide, quinolinyl N-oxide, indolyl N-oxide, indolinyl N-oxide, isoquinolyl N-oxide, quinazolinyl N-oxide, quinoxalinyl N-oxide, phthalazinyl N-oxide, imidazolyl N-oxide, isoxazolyl N-oxide, oxazolyl N-oxide, thiazolyl N-oxide, indolizinyl N-oxide, indazolyl N-oxide, benzothiazolyl N-oxide, benzimidazolyl N-oxide, pyrrolyl N-oxide, oxadiazolyl N-oxide, thiadiazolyl N-oxide, triazolyl N-oxide, tetrazolyl N-oxide, benzothiopyranyl S-oxide or benzothiopyranyl S,S-dioxide.

In another embodiment, the heterocyclyl or heterocycloalkyl group is preferably a carbocyclic ring system of 4-, 5-, 6-, or 7-membered rings, which includes fused ring systems of 8-18 atoms containing at least one and up to four heteroatoms selected from nitrogen, oxygen, or sulfur. More preferably, the heterocycloalkyl or heterocyclyl group is morpholinyl, thiomorpholinyl, thiomorpholinyl S-oxide, thiomorpholinyl S,S-dioxide, piperazinyl, homopiperazinyl, pyrrolidinyl, pyrrolinyl, tetrahydropyranyl, piperidinyl, tetrahydrofuranyl, tetrahydrothienyl, homopiperidinyl, homomorpholinyl, homothiomorpholinyl, homothiomorpholinyl S,S-dioxide, oxazolidinonyl, dihydropyrazolyl, di-hydropyrrolyl, dihydropyrazinyl, dihydropyridinyl, di-hydropyrimidinyl, dihydrofuryl, dihydropyranyl, tetrahydrothienyl S-oxide, tetrahydrothienyl S,S-dioxide and homothiomorpholinyl S-oxide.

Halogen is preferably F, Cl, Br or I.

The chemical groups in a compound of formula 1 are preferably substituted by 1, 2, 3, 4, 5 or 6 substituents. The term "optionally substituted" refers to a substitution of at least one hydrogen atom of the respective chemical group, wherein the substituent is selected from the group of -OH, -SH, -NH₂, -SO₂, -SO₃, -PO₄, -O-C₁₋₄-alkyl, -S-C₁₋₈-alkyl, -NH-C₁₋₈-alkyl, -C₁₋₈-alkyl, -O-C₂₋₈-alkenyl, -S-C₂₋₈-alkenyl, -NH-C₂₋₈-alkenyl, -C₂₋₈-alkenyl, -O-C₂₋₈-alkynyl, -S-C₂₋₈-alkynyl, -NH-C₂₋₈-alkynyl, -C₂₋₈-alkynyl, -C₅₋₁₀-aryl or aryloxy, -C₅₋₁₀-hydroxyaryl, or 5 to 10 membered heteroaryl or heteroaryloxy, -C₅₋₁₀-cycloalkyl or -cycloalkenyl, 5 to 10 membered heterocycloalkyl, guanidinyl, a halogen atom, C₂₋₁₆-acyl, -acylamino or -acyloxy, amino, aminocarbonyl, alkoxycarbonylamino, azido, cyano, keto, thiocarbonyl, carboxy, carboxy-C₁₋₈-alkyl, C₅₋₁₀-arylthio, 5 to 10 membered heteroarylthio, 5 to 10 membered heterocyclyl, heterocyclylthio or heterocyclooxy, thiol, C₁₋₈-alkylthio, aminosulfonyl, aminocarbonylamino, hydroxyamino, alkoxyamino, nitro, -SO-C₁₋₈-alkyl, -SO-C₅₋₁₀-aryl, -SO-C₅₋₁₀-heteroaryl, -SO₂-C₁₋₈-alkyl, -SO₂-C₅₋₁₀-aryl and -SO₂-C₅₋₁₀-heteroaryl, carboxy, carboxy-C₁₋₈-alkyl, CF₃, substituted amino. The substituents are optionally further substituted.

Preferably R¹ or R² is hydrogen or methyl.

Heterosubstitutions provide a substitution of the main chain carbon atom by a heteroatom preferably selected from O, S, N or P. Such heterosubstitutions are included by the term "optionally substituted". These heteroatoms are also counted for group size. Hydrogen is not counted.

If X is an aryl or heteroaryl group, X is preferably not further substituted. In the case of rigid aromatic groups the flexibility of the X group can be maintained by preventing further constriction through side chain substituents, whereby the the compound of formula 1 can still adopt the necessary function as an universal amino acid derivative. If X is substituted aryl or heteroaryl the substituent is preferably selected from OH, O-, NH- or S- C₂₋₁₀-alkyl, C₀₋₁₀-alykl-5-14 membered (hetero) aryl or - (hetero)cycloalkyl, SH, NH₂.

The group X in formula 1 forms the connection group between the CO-hydrazine and the NR¹-oxalic acid or ester groups. The atom numbering for the X group is performed using standard locant nomenclature for amino acids using greek letters, i.e. the alpha position is the position of the atom next to the CO-hydrazine group within X (as in amino acids the atom next to the carboxylic acid group, from which the CO-hydrazine group can be derived). The beta group would be the next main chain atom after the alpha atom and so forth. The NR¹-oxalic acid or ester group can be bound to X either on the alpha, or the beta, gamma, etc. position as in alpha, beta, gamma, etc. amino acids.

The compounds are preferably L- or D-stereoisomers at the C-alpha position.

In a specific embodiment X in formula 1 is a chemical group of one of formulas 2-6, 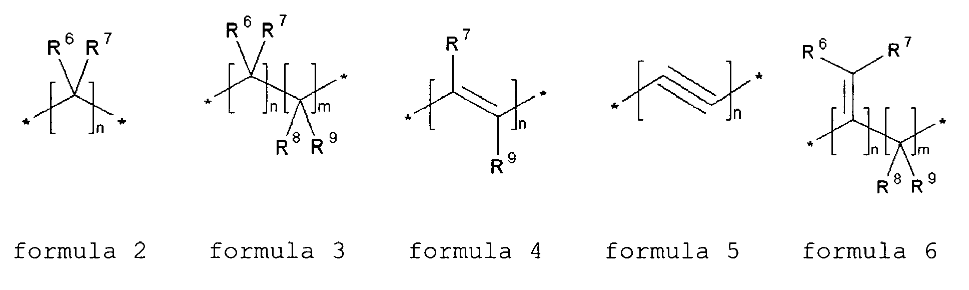 wherein R⁶, R⁷, R⁸ and R⁹ are optionally substituted and independently selected from the groups of H, C₃₋₁₄-cycloalkyl, C₅₋₁₂ aryl, 3-12-membered heterocyclyl or heteroaryl and linear or branched C₁₋₁₄-alkyl, C₂₋₁₄-alkenyl or C₂₋₁₄-alkynyl; and optionally at least two of R⁶, R⁷, R⁸ and R⁹ can cooperate to form a 3 to 12-membered cycloalkyl or heterocycloalkyl ring; and n and m are independent integers between 0 and 5.

Preferably X is a substitute for a natural or unnatural amino acid functionality. Accordingly, side chains of these amino acids are expressed by X. In a specific embodiment X is, a preferably alpha, beta or gamma NR¹-oxalic acid or ester bound group, selected from C₁₋₂-alkyl, guanidinylbutyl, 2-methyl-butyl, phenylethyl, p-hydroxyphenylethyl, indole-3-ylethyl, hydroxyethyl, methylthiopropyl, thioethyl, C₂₋₃-alkyl acid, C₂₋₃-alkyl acidamide, aminopentyl, 4-imidazolylethyl, or X and R¹ cooperate to form a butyl group forming a pyrrolidine ring with the nitrogen of the NR¹-oxalic acid or ester group.

In especially preferred embodiments X is an alpha -CHR¹³-group, wherein R¹³ is the side chain of an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine and valine, preferably the C-alpha L-enantiomer therefrom.

In another embodiment X is selected from O-, S-, N- or P-heterosubstituted 3-20-membered O-, S-, N- or P- heterosubstituted heteroaryl or from the group of optionally substituted heterocycloalkyl, and linear or branched 1-20-membered heteroalkyl, 2-20-membered heteroalkenyl or heteroalkinyl.

In another preferred embodiment R³ and R⁵ form a bond resulting in a heterocylic compound of the general (sub)formula 10, 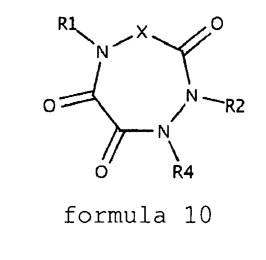 wherein X, R¹, R² and R⁴ are defined as given above. The compound of formula 10 resembles the 1,2,5-triazepine structure (especially if X is C₁-alkyl), known from e.g. Zaleska et al. (Pol. Synthesis 16 (2003): 2559-2563) and Lenman et al. (J. Chem. Soc. Perkin Trans. 1 (1997): 2297-2311). However according to the present invention all ring bonds are single bonds and the ring has an amide group and the oxalic group. As only relatively few substances of the 1,2,5-triazepine-type are known this method provides a new access to this ring system. In a further embodiment R² and R⁵ form a bond resulting in another heterocyclic compound.

Preferred are esters or amides of the present invention, preferably with protecting or activating groups. In a specific embodiment of the present invention the terminal amino group is substituted with a protecting group, preferably selected from Boc, Fmoc, CBz, Alloc, carbonyl, sulfonyl, sulfinyl, phosphoryl, phosphinyl. Another specific embodiment is characterized by an ester of the terminal carbonyl group in such a way, that this ester, preferably a benzyl- or t-butyl-ester, acts either as a protecting group, or as an activating group with enhanced reactivity compared to the underlying carboxylic acid. Further protecting or activating groups can be used as described in "Handbook of Reagents for Organic Synthesis: Activating and Agents and Protecting Groups. Pearson, Anthony J.; Roush, William R.; Editors. UK. (1999), 513 pp. Publisher: (Wiley, Chichester, UK) or Protective Groups in Organic Synthesis. 2nd Ed. Greene, Theodora W.; Wuts, Peter G. M. USA. (1991), 473 pp. Publisher: (John Wiley and Sons, Inc., New York, N.Y.).

A multitude of protecting groups is known in the state of the art. An example of an amine or OH protecting group is benzylcarboxycarbonyl (abbr. Z or Cbz) with the general reactivity: 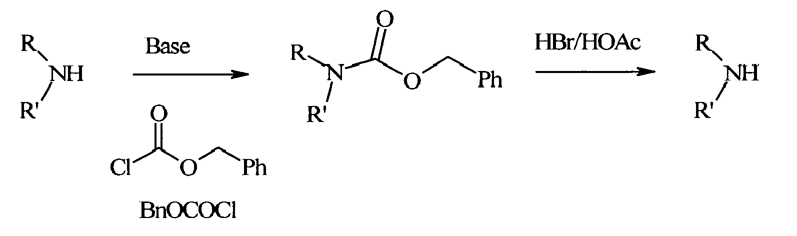 The aromatic phenyl (Ph) therein can optionally be substituted, e.g. by a halogen atom (see examples), or further varied as is known in the field of organic chemistry, e.g. by further substitution of one to three alkoxy (typically methoxy) groups allowing the deprotection steps to proceed under milder condition. Further preferred protecting groups include the t-Butoxycarbonyl (t-BOC) 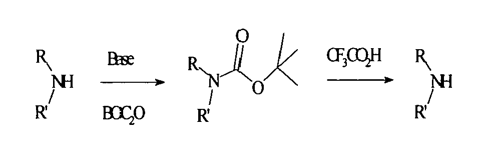 or the 9-Fluorenylmethoxycarbonyl (Fmoc) protecting group, 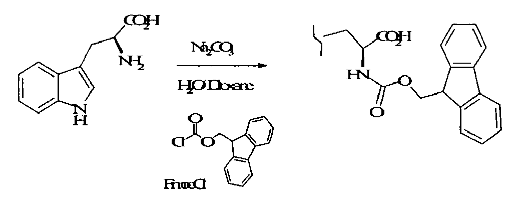 among others. Bound protecting groups in a compound according to the present invention preferably include 9-fluorenylmethyl carbamate (Fmoc-NRR'), t-butyl carbamate (Boc-NRR'), benzyl carbamate (Z-NRR', Cbz-NRR'), acetamide, trifluoroacetamide, phthalimide, benzylamine (Bn-NRR'), triphenylmethylamine (Tr-NRR'), optionally substituted with additional chlorine atoms, benzylideneamine, p-toluenesulfonamide (Ts-NRR'), N-allyloxycarbonyl (Alloc) ester, methyl ester, t-butyl ester, benzyl ester, S-t-butyl ester, 2-Alkyl-1,3-oxazoline, acetic acid ester, pivalic acid ester, benzoic acid ester, sulfonyl ester, sulfinyl ester, phosphonyl ester, and amino acid ester. An extensive overview of protecting groups, which can be used according to the present invention is given in "Synthesis of peptides and peptidomimetics" ed. M. Goodman, Vol. 22a and 22b (protecting and activating groups), 22c and 22d (peptide/peptidemimetic synthesis), Georg Thieme Verlag, Stuttgart, New York (2002). Volumes 22c and 22d also disclose methods for side chain modification, which can also be employed when synthesizing (derivative) compounds according to formula 1 and peptidomimetics of the present invention.

In a compound according to the present invention the protecting group preferably replaces R³, R⁴, R⁵ or R¹⁰ thus forming an ester or amide of the CO-hydrazine or the NR¹-oxalic acid group thus formally replacing R³, R⁴, R⁵or R¹⁰.

The present invention also provides a method for the manufacture of a compound of formula 1, defined above, wherein a compound or precursor according to formula 7, 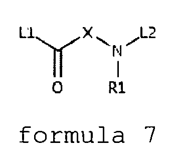 is contacted by a hydrazine or hydrazine derivative of formula 8 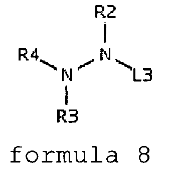 and an oxalic acid, ester or derivative of formula 9, 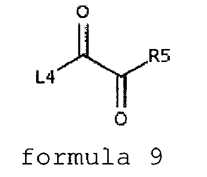 in any order, wherein X, R¹, R², R³, R⁴ and R⁵ are defined as given above for the final compound of formula 1, above, and L1, L2, L3 and L4 are independent selected arbitrary leaving groups, preferably L4 is Cl or OH or an activated ester, e.g. phenyl- or 4-nitrophenylester, or an anhydride including mixed anhydride, e.g. MeO-CO-CO-O-CO-CO-OMe or 1,4-dioxane-2,3,5,6-tetrone. Preferably the compound according to formula 9 is an oxalic acid ester chloride or oxalic acid anhydride. Further chemical bonds between two of X, R¹, R², R³, R⁴ and R⁵ can be formed by conventional chemical synthesis procedures, including ester, amide and protecting and activating group chemistry. Preferably, the compound of formula 9 can be added before or after the addition of the compound of formula 8. If all compounds are added in one step mixtures of all starting substances can result.

Leaving groups are well known in the field of organic chemistry and form preferably conjugate acids (see also "Synthesis of peptides and peptidomimetics", above). Their functionality is characterized by an inherent instability. Preferably L1, L2, L3 and L4 are independently selected from amine (-NH₂), methoxy (CH₃O-), hydroxyl (HO-), carboxylate (CH₃COO-), -NO₃, F-, Cl-, Br-, I-, azide (N₃-), thiocyanate (SCN-), nitro (-NO₂) and cyanide (-CN). L2 and L3 are preferably hydrogen.

In a most preferred embodiment R³ or R⁴ is a protecting group, preferably Fmoc, for example synthesized by reacting Fmoc-Cl anhydride with NH₂-NH₂, or more generally NHR³-R²L3.

The compound according to the present invention is preferably used to create a peptide mimetic with other amino acids or amino acid mimetics. Such a peptide mimetic comprises the compound according to the general formula 1 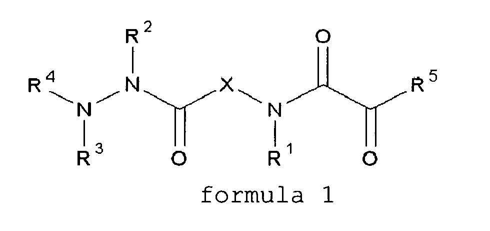 wherein X is the connection between the CO-hydrazine and the NR¹-oxalic acid or ester group and is either a bond, 5-20 membered heteroaryl or aryl, or an optionally substituted group selected from C₃-₂₀-cycloalkyl, 3-20 membered heterocyclyl, and linear or branched C₁₋₂₀-alkyl, C₂₋₂₀-alkenyl or C₂₋₂₀-alkinyl; and R⁵ is selected from -OR¹⁰ and -NR¹⁰R¹¹, or R⁵ can cooperate with R² or R³ to form a bond or a 8 to 10 membered heterocyclic ring; and R³ and R⁴ together may constitute a double bond to a group R¹²; R¹, R², R³, R⁴, R¹⁰, R¹¹ and R¹² are optionally substituted and independently selected from H, C₃₋₁₄-cycloalkyl, C₅₋₁₄-aryl, 3-14 membered heterocyclyl or heteroaryl, linear or branched C₁₋₁₄-alkyl, C₂₋₁₄-alkenyl, C₂₋₁₄-alkinyl; and optionally at least two of R¹, R², R³, R⁴ and x can cooperate to form a 3 to 12 membered cycloalkyl or heterocycloalkyl ring; or an ester, amide, salt, stereoisomer or racemate therefrom; as a molecular part, and a natural or unnatural amino acid or an additional amino acid mimetic, preferably bound by an amide bond. As in the case of the sole compound according to formula 1, in the case of X being aryl or heteroaryl X is preferably not substituted. The compound of formula 1 is most preferably linked to another main amino acid via its hydrazine or oxalic acid groups, preferably by both to different amino acids. The compound of formula 1 is therefore comprised in a peptide mimetic among other amino acids, peptides, or other amino acid analoga, preferably comprising 1, 2, 3, 4, 5, 6, 7, 8, 9 or 10 further natural or unnatural amino acids. Preferably at least one of these amino acids is selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or proline, most preferred connected at the R³, R⁹ or R⁵ position, replacing any possible substituents at these positions in the compound of formula 1. Another preferred additional compound or peptide mimetic substructure is phenyl ethylenediamine, which can be used to generate turn motifs. Such an amide can mimic the three dimension appearance of an amino acid, e.g. the hydrogen bonding acceptor and donor properties of the amide bond, found in proteins. Amazingly, with the compound of formula 1 most native protein structures can be created, including alpha-helices, and beta-sheets.

Therefore, in a preferred embodiment the peptide mimetic mimics the three dimensional structure of an alpha-helix, beta-sheet or turn motif. Through this three dimensional structure native protein motifs can be formed, which allows the use of the compound in substantial protein or peptide parts. It is thus possible to form peptide mimetics with the biological functionality of the respective templates, but with modified stability, bioavailability or distribution as examples of its modified chemical properties.

Interactions between the hydrogen-bonding edges of beta sheets occur widely in protein quaternary structure, protein-protein interactions, and protein aggregation and are involved in both healthy biological processes and in diseases ranging from cancer and AIDS to anthrax and Alzheimer's. These protein-protein interactions constitute a form of molecular recognition of great importance in biological processes and because of its fundamental nature. The compounds of the present invention can be used to synthesize modified peptides that recognize protein beta-sheets in a sequence-selective fashion. A database for beta-sheets, wherein the compounds of the present invention can be incorporated is established in the internet (http://www.igb.uci.edu/servers/icbs/).

Protein secondary structures such as α-helices, β-sheets, and β-turns are important features of the three-dimensional structure and biological activity of proteins. The mimicry of peptide and protein structures has emerged as a focal point of bioorganic and medicinal chemistry. Designing secondary structure mimics composed of short peptides has attracted much attention in the development of pharmacologically active compounds, artificial receptors, asymmetric catalyst and new materials. A substantive examination of artificial alpha-helices, beta-sheets and turns, as well as their design was reviewed by Rizo and Gierasch (Annu. Rev. Biochem 1992. 61:387-418). The chemical synthesis of such a mimetic can be performed as described herein, e.g. by solid phase synthesis using a compound of formula 1. The prognosis of a fold for alpha-helices, beta-sheets and (beta-) turns can be designed according to Rizo and Gierasch, or Loughlin et al. (Chem Rev 2004, 104:6085-6117).

Compounds that mimic the structure and hydrogen-bonding patterns of protein β-sheets are of interest as drug candidates and as model systems with which to study protein structure and stability. β-sheet formation plays a critical role in many biological processes associated with diseases and normal functions. β-sheet interactions between proteins have been shown or hypothesized to be involved in cell signalling and oncogene expression associated with the binding of Ras and Rap by the serine/threonine kinase Raf, the clustering of membrane ion channels by PDZ domains, the binding of lymphocyte function-associated antigen-1 (LFA-1), by the intercellular adhesion molecule-1 (ICAM-1), and the interaction between the CD4 receptor and the HIV viral protein gp120, to cleave peptides, proteolytic enzymes, such as HIV-1 proteases or renin, form β-sheet-like networks of hydrogen bonds with their peptide substrates. HIV-1 protease dimerizes through four-interchelating β-strands. The met repressor, a protein involved in gene regulation, also functions as a β-sheet dimer; in this case, the β-sheet that forms is directly involved in binding to the major groove of DNA. Many proteins aggregate to form insoluble β-sheet structures that are associated with Alzheimer's disease, Kreutzfeld-Jacob disease and other prion diseases, and progressive neurodegenerative disorders that are associated with trinucleotide (CAG) repeats.

The compounds of the current invention can provide alternating arrays of hydrogen-bond donors and acceptor patterns like a tripeptide β-strand and can be used as β-strand mimics to form β-sheet-like structures in combination with an appropriate molecular scaffold. The application of a compound according to formula one in a beta-sheet is realized by hydrogen bond donor and acceptor functionalities, for example according to the following scheme:

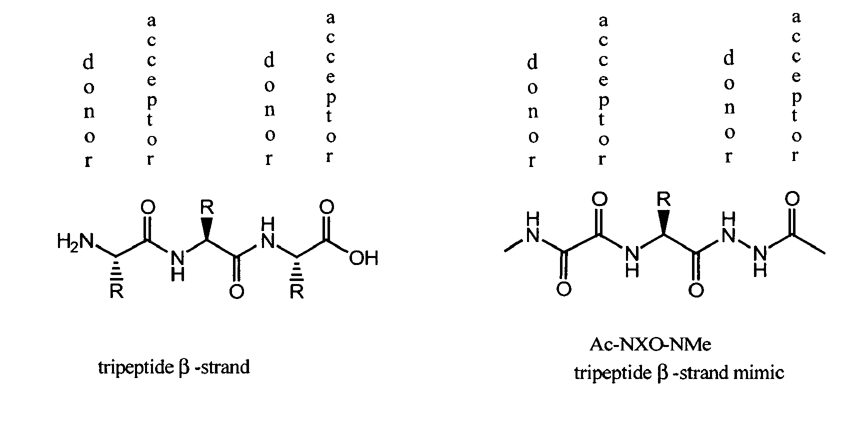

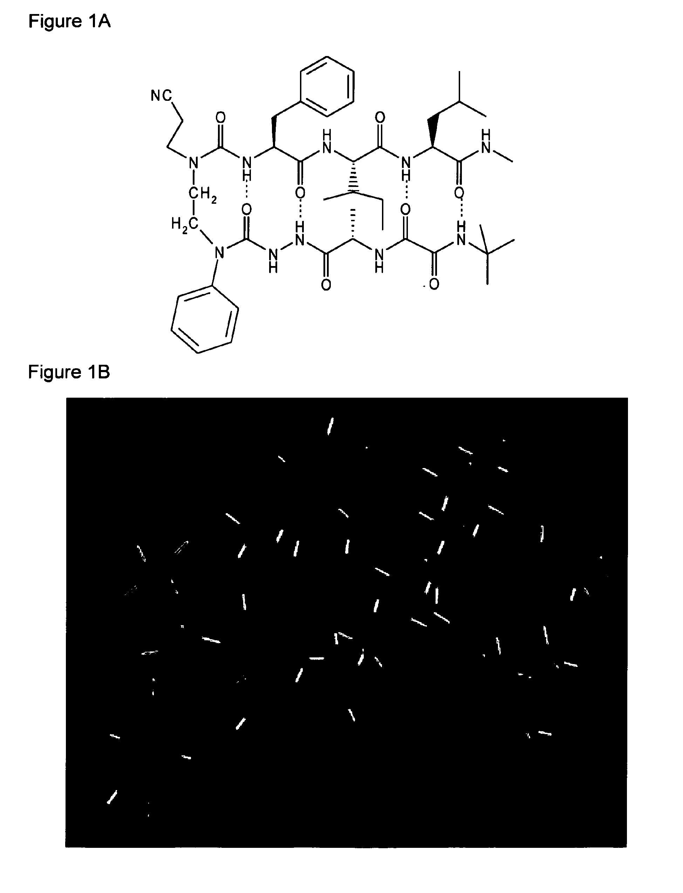

2D and 3D structures of a specific example of the compound in a β-strand are given in figure 1.

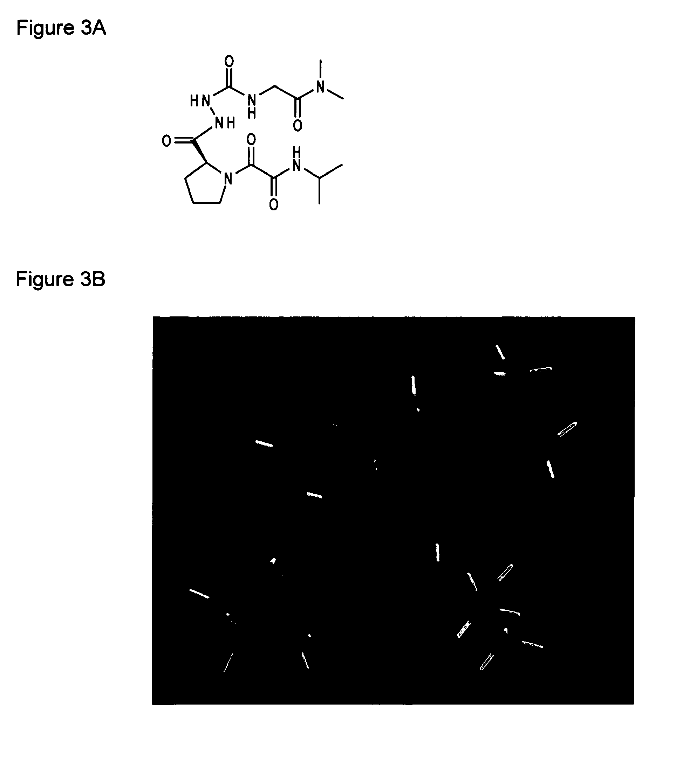

In the 20 proteinic amino acids, proline is the only amino acid whose Cα-N bond is a part of the pyrrolidine ring. This cyclic side chain imposes strong restrains on peptide conformation. Proline if quite often observed at the (i + 1) position of β-turn structure. The proline derivatives (wherein X cooperates with R¹ to form a ring structure) can also be used as beta turn mimics, as an example figure 3 is a structure of a turn-motif.

A compound of formula 1 can e.g. be derived from a natural amino acid, wherein the amino functionality of the amino acid was transformed into a carboxylic functionality through the oxalic acid ligation, and the carboxylic acid functionality was transformed into an amidic functionality by the hydrazine. These reversed functionalities can, in analogy to the amine and carboxylic functionalities of an amino acid, be used for the same purposes, like liquid or solid phase protein synthesis using the same or similar protective groups developed for amino acids.

The present invention provides a method for the synthesis of a peptide mimetic using a compound of the general formula 1, wherein a reaction target comprising an amine is contacted with the compound according to formula 1, and R³ or R⁴ constitutes an amide with a protecting group, preferably Fmoc. Through this step a peptide bond is formed under standard conditions for peptide synthesis. In this step of common peptide synthesis procedures the amino acid to be bound is replaced by the compound according to formula 1. The amino group containing reaction target can be an amino acid or a derivative or another target, like a solid resin. The Fmoc and Boc protecting groups are especially preferred, since they can again be removed by an irreversible process, wherein the carboxylic group of Fmoc or Boc is removed through CO₂ elimination, preferably under acidic conditions by trifluoroacetic acid. This has the advantage of high yield, especially in combination with solid phase synthesis, wherein a peptide is stepwise synthesized onto a solid resin. Contrary to natural biosynthesis at the ribosome, solid-phase peptide synthesis preferably proceeds in a C-terminal to N-terminal fashion, wherein the amino group is protected by an arbitrary protecting group. In a next step this protecting group is removed, e.g. the amide with the R³ or R⁴ protecting group of the compound according to formula 1. Amino acid chain elongation, or amino acid mimetic ligation, can then proceed by binding the carboxylic functionality of the next amino acid or an amino acid mimetic to the now unprotected amino functionality. The compound according to the present invention is an example of such an amino acid mimetic, among others known in the state of the art.

In a further aspect of the method provided herein an amino acid or amino acid mimetic, preferably with a protected amino group is contacted with the compound according to formula 1, preferably after the step of removing the R³ or R⁴ protecting group.

Preferably, the method for peptide mimetic synthesis is a solid phase synthesis method, i.e. a reaction target is solid, preferably a solid resin, e.g. the Merrifield resin, a copolymer of styrene and chloromethylstyrene in bead form (Steward and Young, Solid Phase Peptides Synthesis, Pierce, Rockford, IL (1984)). This includes cases where amino acids or other chemical compounds have already been ligated to the resin and the compound of formula 1 is ligated to the amine groups of these immobilized amino acids or compounds. Of course, further amino acids and other chemical compounds can be ligated to the compound of formula 1 afterwards.

In the examples several solid phase synthesis methods have been applied with the incorporation of building blocks of formula 1 into artificial peptides. These building blocks can also be used by peptide synthesis robots. The term "protecting groups" also comprises the solid phase versions of protecting groups. As these building blocks can be integrated into solid phase synthesis and automated peptide synthesizers, there will be numerous applications of the compounds of formula 1.

In another aspect the present invention provides a protein or protein fragment comprising the compound of the general formula 1 as covalently bound insert at any position of the protein amino acid sequence. To this end the protein can be synthesized using artificial, biological or microbiological methods. The compound of formula 1 can be attached to such a protein or protein fragment or two fragments, for example two fragments of one protein, whereby the compound of formula 1 is inserted into the amino acid sequence of the protein.

To this end a method for the manufacture of a protein or protein fragment is provided, comprising the step of ligating a compound of formula 1 to a peptide, protein or protein fragment. As mentioned above, the advantage of a compound of formula 1 lies in the usability of standard protein and peptide synthesis methods.

Preferably, the method further comprises the step of ligating a further peptide, protein or protein fragment to the compound of formula 1. Preferred is the use of the compound of formula 1 as amino acid substitute (wherein one or more amino acids of the protein is/are substituted) in a protein, e.g. an affinity peptide, wherein the compound stabilizes the peptide in a specific three dimensional structure.

A preferred use for the compound according to the invention is as a spacer moiety, especially as a hydrophilic spacer. Spacer moieties have a wide range of application. For example in biochemistry spacers are used for the immobilization of pharmacologic agents on biomolecules, e.g. antibodies, or for the immobilization of affinity targets on a resin for the purposes of chromatography. The compounds disclosed herein have the amino and carboxylic features of a natural amino acid, and can be handled by standard protein chemistry techniques. Therefore, the compounds of formula 1 can be used as linker moieties, wherein the length of the linker can be determined by the size of the X group. Especially in the field of bioaffinity purification such a linker is excelled by low unspecific binding of unwanted biomolecules.

The present invention is further illustrated by the following figures and examples, without being limited thereto.

### Figures:

Figure 1: A: 2D structure of a beta-sheet model comprising a modified Alanin (X in formula 1 is a C₁-alkylated C-alpha), lower strand. B: 3D-structure of a beta-sheet stabilized by hydrogen bonds.

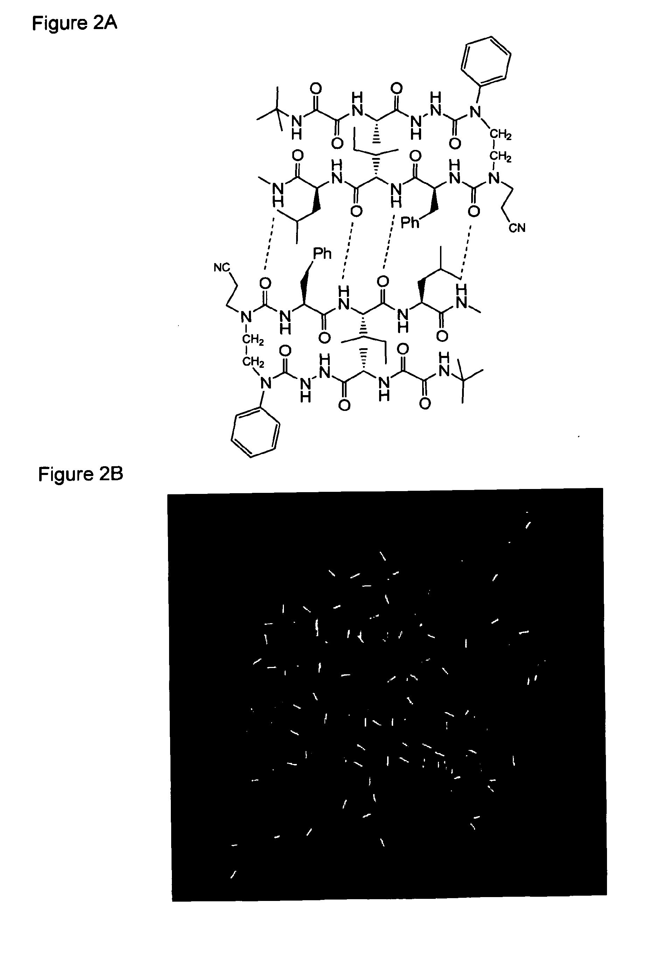

Figure 2: Structures of a hydrogen bonded beta-sheet dimer compound, A: 2D, B: 3D.

Figure 3: Structures of beta-turns of a prolin amino acid analogon, A: 2D, B: 3D.

### Examples:

### Abbreviations

- Boc: t-Butyloxycarbonyl
- DCC: Dicyclohexylcarbodiimide
- DCM: Dichloromethane
- DCHU: Dicyclohexylurea
- DIPA: Diisopropylethylamine (=Hünig's base)
- DIC: Diisopropylcarbodiimide
- EDCI.HCl: Ethyldiisopropylcarbodiimide hydrochloride
- Fmoc: Fluorenylmethyloxycarbonyl
- HOBt: 1-Hydroxybenzotriazole
- THF: Tetrahydrofuran
- Z: Benzyloxycarbonyl

### Example 1: N'-((S)-2-Benzyloxycarbonylamino-3-phenyl-propionyl)-hydrazine carboxylic acid tert-butyl ester:

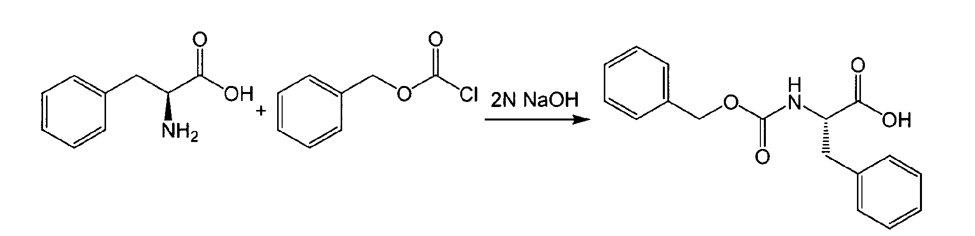

### Step 1: Z- L -Phenylalanine:

L- Phenylalanine (2.0 g,12 mmol) was dissolved in 2 N NaOH (10 mL), freshly distilled benzyloxy carbonyl chloride (2.07 mL, 14.54 mmol) was added drop wise with stirring at 0°C to the reaction mixture and stirred further at 0°C for 30 min and at room temperature for 1h. The reaction mixture was washed with diethyl ether (10 mL), acidified to pH=4 with 2 N HCl and extracted with ethyl acetate (3 x 10 mL), the combined organic layer was washed with water (10 mL), brine (10 mL), dried (Na₂SO₄) and concentrated to give 3.6 g (99%) of the product as a viscous oil which on standing overnight at room temperature gives white solid; mp. 83 - 84°C (Lit.: 85 - 87°C).

¹H NMR (200 MHz, CDCl3) δ (ppm) 7.04-7.26 (m, 10H), 5.11 (d, *J* = 8.02 Hz, 1H), 5.02 (s, 2H), 4.63 (m, 1H), 2.97-3.19 (m, 2H).

### Step 2:

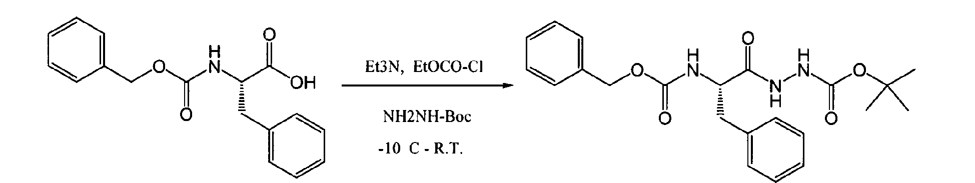

To a solution of Z- L -Phenylalanine (3.6 g, 12 mmol) in dry THF (30 mL) was added slowly with stirring triethylamine (1.85 mL,13.23 mmol) at -10°C, followed by ethylchloroformate (1.26 mL,13.23 mmol). The reaction mixture was stirred at same temperature for 30 min under argon and the solution of *t*-butyl carbazate (1.59 g, 12.03 mmol) in dry THF (20 mL) was added slowly with stirring, the reaction mixture was stirred further at room temperature for 1 h.

The mixture was diluted with ethyl acetate (50 mL) and washed with 1N HCL (10 mL), followed by 10% NaHCO₃(10 mL) and brine (10 mL). The ethyl acetate layer was dried (Na₂SO₄) and concentrated to give 5.2 g crude compound which was purified by column chromatography to give 3.6 g (72%) of pure product as a colourless viscous oil which, on standing overnight at room temp., crystallized as a white solid, mp 104 -105°C.

¹H NMR ( 200 MHz, CDCl₃) δ (ppm) 8.13(br s, 1H), 7.09-7.25 (m, 10H), 6.51 (br s, 1H), 5.43 (d, *J* = 6.98 Hz, 1H), 4.95 (dd, *J* = 12.34, 12.36 Hz, 2H), 4.28-4.59 (m, 1H), 3.1 (dd, *J* = 5.9, 6.44 Hz, 1H), 2.93 (dd, *J* = 7.92 Hz, 1H), 1.38(s, 9H).

### Example 2: (S)-2-(methoxyoxalyl-amino)-3-phenyl-propionic acid

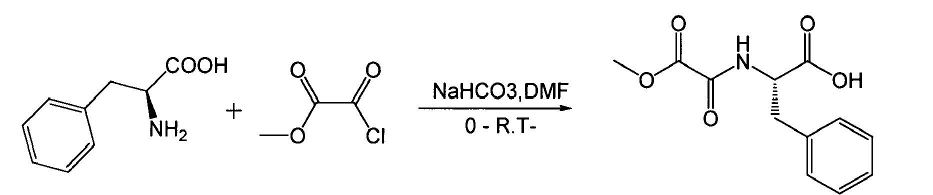

Mono- methyl oxalyl chloride (3.34 mL, 36.32 mmol) was added drop wise at 0°C to the solution of L-phenylalanine (3.0 g, 18.16 mmol) and NaHCO₃ (7.63 g, 90.81 mmol) in DMF (15 mL). The reaction mixture was stirred further at room temperature for 2 h. DMF was removed by evaporation, the residue obtained was dissolved in water (50 mL),washed with diethyl ether (2 x 10mL), acidified (pH 4) with 1 N HCL and extracted with ethyl acetate (3 x 15 mL). The combined organic layer was washed with brine (2 x 5 mL), dried (Na₂SO₄) and concentrated to give 2.58 g (56 %) of desired compound as yellowish viscous oil.

¹H NMR (200 MHz, DMSO-d₆) δ (ppm) 9.06 (d, J = 9.2 Hz, 1H), 7.15-7.31 (m, 5H), 4.43-4.54 (m, 1H), 3.76 (s, 3H), 2.97-3.21 (m, 2H).

### Example 3: N-[(S)-1-Benzyl-2-(N'-tert-butoxycarbonyl-hydrazino)-2-oxo-ethyl]-oxalamic acid methyl ester

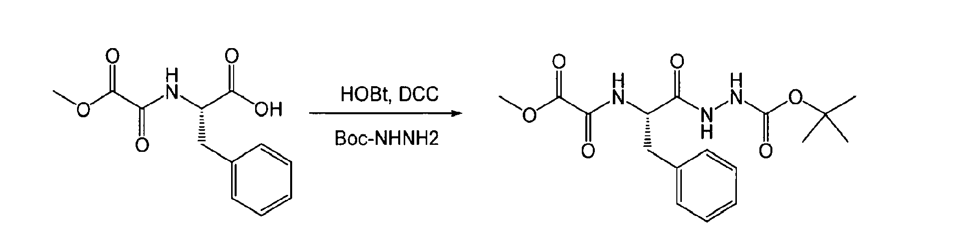

To the solution of (S)-2-(methoxy-oxalyl-amino)-3-phenylpropionic acid (example 2) (1.6 g, 6.37 mmol) in DCM (30 mL) was added HOBt (903.6 mg, 6.69 mmol) followed by DCC (1.38 g, 6.69 mmol) and stirred for 30 min at room temperature. To this Boccarbazate (883.8 mg, 6.69 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (10 mL),10 % NaHCO₃ (10 mL), brine (10 mL), dried (Na₂SO₄) and concentrated to give 2.1g of crude compound which was purified by column chromatography to give 1.64 g (70 %) of product as a white solid; mp. 72-73°C.

[α]_{D} -28.6 (c 0.5, MeOH)

¹H NMR (200 MHz, CDCl₃) δ (ppm) 8.2 (br s, 1H), 7.8 (br d, *J* = 9.98 Hz, 1H), 7.19-7.35 (m, 5H), 6.56 (br s,1H), 4.74-4.86 (m, 1H), 3.85 (s, 3H), 3.06-3.32 (m,2H), 1.45 (s,9H).

¹³C NMR (200 MHz, CDCl₃) δ (ppm) 169.53, 160.21, 156.38, 155.23, 135.75, 129.24, 128.69, 127.16, 82.04, 53.66, 53.2, 37.55, 28.06.

IR (KBr) ν 4000 - 3292 (br.), 3031, 2980, 1742, 1688 (br.) cm⁻¹.

### Example 4: N-[(S)-1-Benzyl-2-(N'-tert-butoxycarbonyl-hydrazino)-2-oxo-ethyl]-oxalamic acid

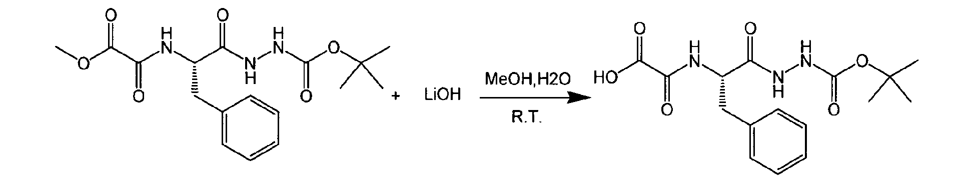

To the solution of *N*-[(S)-1-benzyl-2-(*N*'-*tert*-butoxycarbonyl-hydrazino)-2-oxo-ethyl]-oxalamic acid methyl ester (example 3) (520 mg, 1.42 mmol) in methanol (10 mL) was added lithium hydroxide (38 mg, 1.57 mmol) followed by 2 drops of water. The reaction mixture was stirred at room temperature for 1h. Methanol was evaporated and the residue obtained was dissolved in water (30 mL), washed with ether (2 x 10 mL). The aqueous layer was cooled in ice bath, acidified (pH = 4) with 1N HCl and extracted with ethyl acetate (3 x 10 mL). The combined organic layer was washed with brine (10 mL), dried (Na₂SO₄) and concentrated to give 333 mg (66 %) of desired compound as off- white solid; mp. 82-85°C.

¹H NMR (200 MHz, CDCl₃) δ (ppm) 9.32 (br, 1H), 8.9 (br s, 1H), 8.1 (br d, *J* = 7.24 Hz, 1H), 7.14 (br, 5H), 6.96 (br, 1H), 4.75 (m, 1H), 2.95-3.23 (m,2H), 1.36 (s,9H).

IR (KBr) ν 4000 - 3300 (br.), 3031, 2981, 2934, 1689 (br.) cm⁻¹.

### Example 5: N-{(S)-1-Benzyl-2-[N'-(9H-fluoren-9-ylmethoxy-carbonyl)-hydrazino]-2-oxo-ethyl}-oxalamic acid methyl ester

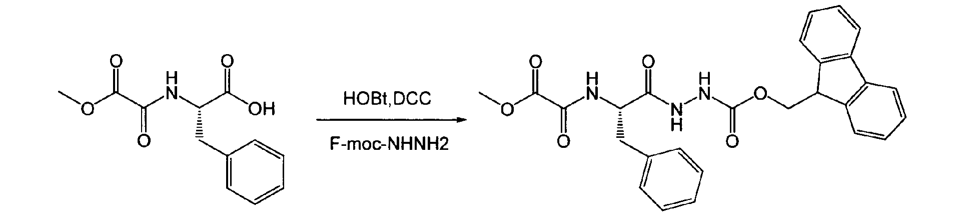

To the solution of (S)-2-(methoxyoxalyl-amino)-3-phenyl-propionic acid (Example 2) (812 mg, 3.23 mmol) in DCM (15 mL) was added HOBt (459 mg, 3.39 mmol) followed by DCC (700 mg, 3.39 mmol) and stirred for 30 min at room temperature. To this Fmoc-carbazate (822 mg, 3.23 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (10 mL),10 % NaHCO₃ (10 mL), brine (10 mL), dried (Na₂SO₄) and concentrated to give 1.3 mg of crude compound which was purified by column chromatography to give 975 mg (61%) of product as a white solid; mp. 91-93°C.

[α]_{D} -19.4 (c 0.5, MeOH)

¹H NMR ( 200 MHz, DMSO-d₆) δ (ppm) 10.08 (br s, 1H), 9.38 (br s, 1H), 8.99 (d, *J* = 8.4 Hz, 1H), 7.89 (d, *J* = 7.04 Hz, 2H) 7.72 (d, *J* = 6.26 Hz, 2H), 7.17-7.46 (m, 9H), 4.53-4.64 (m, 1H), 4.26-4.34 (m, 3H), 3.73 (s, 3H), 2.93-3.15 (m, 2H).

¹³C NMR (200 MHz, DMSO-d₆) δ (ppm) 169.98, 160.67, 156.82, 155.94, 143.61, 140.74, 137.44, 129.08, 128.19, 127.65, 126.99, 126.43, 125.11, 120.05, 66.24, 53.13, 52.70, 46.42, 36.62. IR (KBr) ν 4000 - 3275 (br.), 3030, 2954, 1734, 1704 (br.) cm⁻¹.

### Example 6: (S)-2-tert-Butoxycarbonylamino-3-phenyl-propionic acid

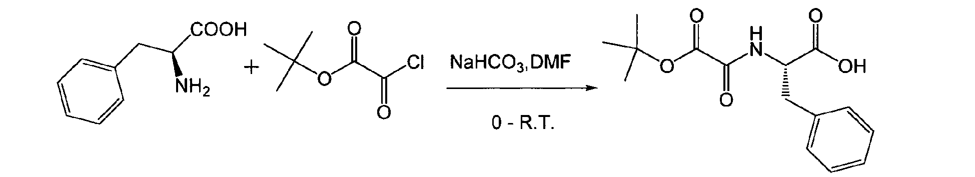

Mono- tert-butyl oxalyl chloride (407 mg, 2.47 mmol) was added drop wise at 0°C to the solution of L-phenylalanine (204 mg, 1.24 mmol) and NaHCO₃ (519 mg, 6.18 mmol) in DMF (5 mL). The reaction mixture was stirred further at room temperature for 2 h. DMF was removed by evaporation, the residue obtained was dissolved in water (20 mL),washed with diethyl ether (2 x 5 mL), acidified (pH 4) with 1 N HCL and extracted with ethyl acetate (3 x 10 mL). The combined organic layer was washed with brine (10 mL), dried (Na₂SO₄) and concentrated to give 250 mg of the crude compound as a colourless viscous oil.

Conclusion: The side product (mono-tert - butyl oxalic acid ester) was not separable from desired compound by column chromatography so the crude compound was used as such for further reaction.

### Example 7: N'-((S)-2-tert-Butoxycarbonylamino-3-phenyl-propionyl)-hydrazine carboxylic acid 9H-fluoren-9-ylmethyl ester

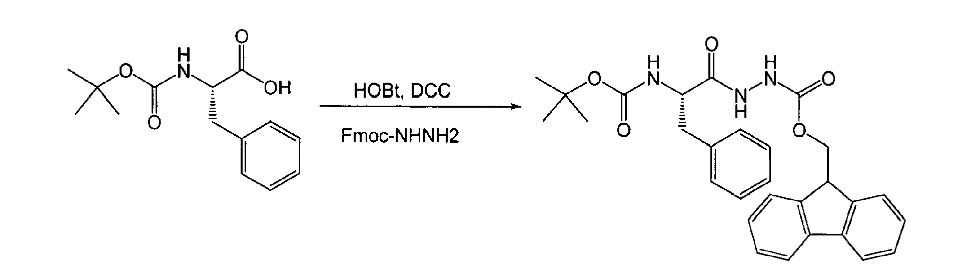

To the solution of Boc-L-phenylalanine (2.0 g, 7.54 mmol) in DCM (50 mL ) was added HOBt (1.07 g, 7.92 mmol) followed by DCC (1.63 g, 7.92 mmol) and stirred for 30 min at room temperature. To this F-moc- hydrazine (1.91 g, 7.54 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (30 mL),10 % NaHCO₃ (30 mL), brine (30 mL), dried (Na₂SO₄) and concentrated to give 4.4 g of crude compound which was crystallised from chloroform - pet ether to give 3.6 g (95%) of product as a white solid; mp. 160 - 161°C.

R*_{f}* = 0.26 (1.5 : 3.5 EtOAc/petrol ether)

[α]_{D} -11.2 (c 0.5, MeOH)

¹H NMR (200 MHz, DMSO-d₆) δ (ppm) 9.94 (br s, 1H, *d₂o exch*.), 9.35 (br s, 1H, *d₂o exch.*)*,* 7.88 (d, *J =* 6.86 Hz, 2H), 7.73 (d, *J* = 7.04 Hz, 2H), 7.17 - 7.45 (m, 9H), 6.93 (d, *J =* 8.6 Hz, 1H, *d₂o exch*.), 4.28 - 4.31 (m, 4H), 2.69 - 3.0 (m, 2H), 1.27 (s, 9H).

¹³C NMR (200 MHz, DMSO-d₆) δ (ppm) 171.67, 155.95, 155.13, 143.58, 140.65, 137.94, 129.15, 127.95, 127.64, 127.06, 126.16, 125.24, 120.05, 79.09, 77.93, 66.11, 54.16, 46.44, 28.05.

IR (KBr) ν 4000 - 3364, 3309, 3250, 3037, 3003, 2980, 1757, 1692, 1675, 1517 cm⁻¹.

### Example 8: N-{(S)-1-Benzyl-2-[N'-(9H-fluoren-9-ylmethoxycarbonyl)-hydrazino]-2-oxo-ethyl}-oxalamic acid tert-butyl ester

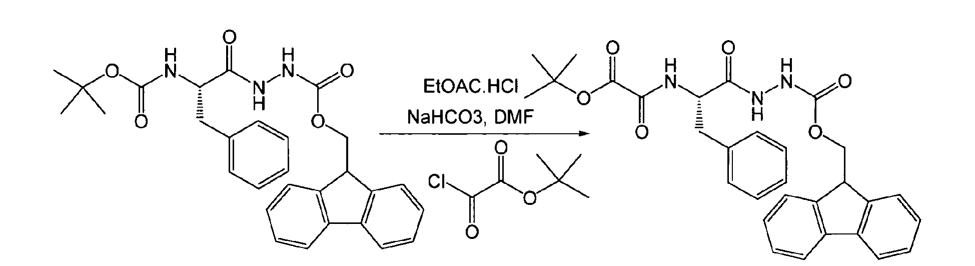

To the solution of *N*'-((S)-2-tert-Butoxycarbonylamino-3-phenyl-propionyl)-hydrazine carboxylic acid 9*H*-fluoren-9-yl-methyl ester (Example 7 (1.2 g, 2.39 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (30 mL), NaHCO₃ (1.41 g, 16.75 mmol) was added to the reaction mixture, followed by *tert*-butyl oxalyl chloride (0.59 g, 3.59 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20mL). The ethyl acetate layer was dried (Na₂SO₄) and concentrated to give 770 mg (60 %) of product as a white powder; mp 106 - 108°C.

R*_{f}* = 0.29 ( 1.5 : 3.5 EtOAc/petrol ether)

[α]_{D} -18.0 (*c* 0.5, DMF)

¹H NMR (200 MHz, CDCl₃) δ (ppm) 8.49 (br s, 1H, *d₂O exch.),* 7.64 (d, *J* = 7.42 Hz, 3H, 1H, *d₂O exch.*)*,* 7.46 (d, *J =* 7.42 Hz, 2H), 7.14-7.31 (m, 9H), 6.96 (br s, 1H, *d₂O exch.),* 4.66-4.77 (m, 1H), 4.21-4.35 (m, 2H), 4.06-4.13 (m, 1H), 2.96-3.19 (m, 2H), 1.37 (s, 9H).

¹³C NMR (200 MHz, CDCl₃) δ (ppm) 170.02, 158.36, 157.78, 156.03, 143.46, 141.23, 135.69, 129.32, 128.74, 127.77, 127.22, 127.14, 125.14, 119.95, 85.01, 68.11, 53.35, 46.80, 37.59, 27.61.

IR (KBr) ν 4000 - 3285, 3065, 2980, 1751, 1732, 1692, 1701, 1517, 1451, 1371, 1219, 1155, 1031, 840, 758, 740, cm⁻¹.

### Example 9: N'-((S)-2-tert-Butoxycarbonylamino-3-phenyl-propionyl)-hydrazine carboxylic acid benzyl ester

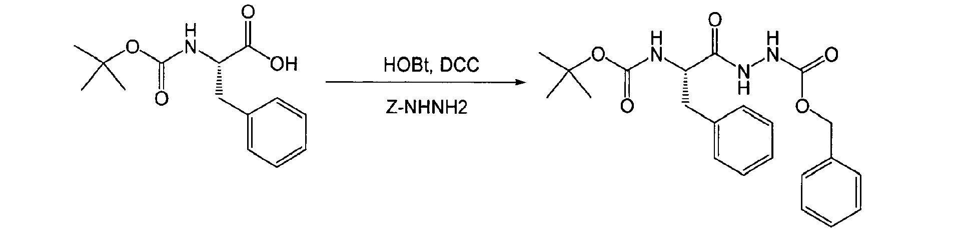

To the solution of Boc-L-phenylalanine (3.0 g, 11.31 mmol) in DCM (50 mL ) was added HOBt (1.6 g, 11.87 mmol) followed by DCC (2.45 g, 11.87 mmol) and stirred for 30 min at room temperature. To this Z- hydrazine (1.88 g, 11.31 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (30 mL),10 % NaHCO₃ (30 mL), brine (30 mL), dried (Na₂SO₄) and concentrated to give 6.4 g of crude compound which was purified by column chromatography to give 4.2 g (89%) of product as a off - white solid; mp. 112 - 113°C.

R*_{f}* = 0.23 (1.5 : 3.5 EtOAc/petrol ether)

[α]_{D} -10.2 (c 0.5, MeOH)

¹H NMR ( 200 MHz, CDCl₃) δ (ppm) 8.65 (br s, 1H, *d₂o exch.),* 7.12-7.22 (m, 11H), 5.27 (br s, 1H, *d₂o exchangeable),* 5.03 (s, 2H), 4.45 (m,1H), 2.78-3.21 (m, 2H), 1.24 (s, 9H).

¹³C NMR (200 MHz, CDCl₃) δ (ppm) 171.65, 156.14, 155.79, 136.42, 135.64, 129.39, 128.50, 128.27, 128.14, 126.81, 80.42, 67.71, 53.99, 38.27, 28.2.

IR (KBr) ν 4000 - 3372, 3313, 3002, 2971, 2929, 1762, 1688, 1672, 1628, 1526 cm-¹.

### Example 10: N-[(S)-1-Benzyl-2-(N'-benzyloxycarbonyl-hydrazino)-2-oxo-ethyl]-oxalamic acid tert-butyl ester (Z-NPheO-O-t-Bu)

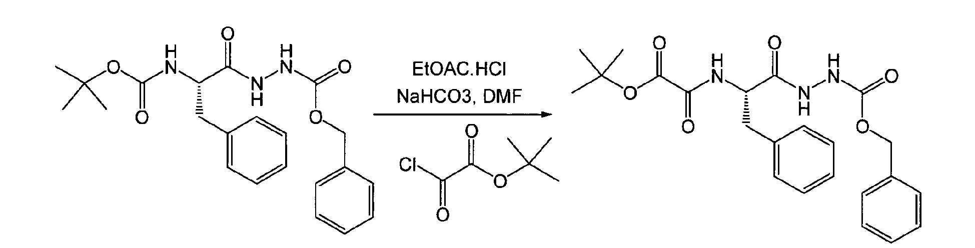

To the solution of *N*'-((S)-2-*tert*-Butoxycarbonylamino-3-phenyl-propionyl)-hydrazine carboxylic acid benzyl ester (Example 9) (1.2 g, 2.9 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (30 mL), NaHCO₃ (1.71 g, 20.32 mmol) was added to the reaction mixture, followed by tert-butyl oxalyl chloride (0.72 g, 4.35 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 770 mg (60 %) of product as a colourless viscous oil which after triturating with pentane gives white powder; mp. 72 - 7°C.

R*_{f}* = 0.29 ( 1.5 : 3.5 EtOAc/petrol ether)

[α]_{D} -23.0 (c 0.5, MeOH)

¹H NMR (200 MHz, CDCl₃) δ (ppm) 8.51 (br s, 1H, *d₂O exch.),* 7.71 (d, *J* = 8.2 Hz, 1H, *d₂O exch.),* 7.24-7.31 (m, 10H), 6.95 (br s, 1H, *d₂O exch.),* 5.12 (s, 2H), 4.72-4.83 (m, 1H), 3.04-3.26 (m, 2H), 1.48 (s, 9H).

¹³C NMR (200 MHz, CDCl₃) δ (ppm) 169.97, 158.37, 157.70, 155.99, 135.70, 135.50, 129.33, 128.70, 128.52, 128.33, 128.14, 127.18, 84.94, 67.83, 53.29, 37.57, 27.60.

IR (KBr) ν 4000 - 3293, 3063, 3032, 2982, 2934, 1732 (br.), 1703 (br.), 1519, 1498, 1455, 1371, 1219, 1154, 1028, 840, 742, 698 cm⁻¹.

### Example 11: N-[(S)-1-Benzyl-2-(N'-benzyloxycarbonyl-hydrazino)-2-oxo-ethyl]-oxalamic acid methyl ester (Z-NPheO-OMe)

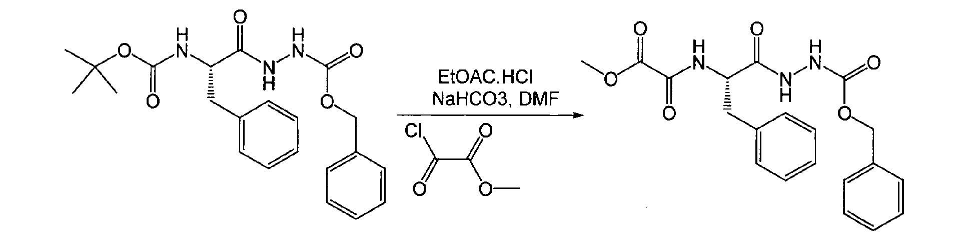

To the solution of *N*-[(S)-1-Benzyl-2-(*N*'-benzyloxycarbonyl-hydrazino)-2-oxo-ethyl]-oxalamic acid *tert*-butyl ester (Example 10) (1.2 g, 2.9 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (30 mL), NaHCO₃ (1.71 g, 20.32 mmol) was added to the reaction mixture, followed by methyl oxalyl chloride (0.4 mL, 4.35 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 1.1g (94 %) of product as a colourless viscous oil which after triturating with pentane gives white powder; mp. 87-89°C.

R*_{f}* = 0.42 (1:1 petrol ether/EtOAc)

[α]_{D} -24.6 (c 0.5, MeOH)

¹H NMR ( 200 MHz, CDCl₃) δ (ppm) 8.52 (br s, 1H), 7.77 (br s, 1H), 7.14-7.22 (m, 11H), 5.02 (s, 2H), 4.73-4.76 (m,1H), 3.67 (s, 3H), 2.85-3.36 (m, 2H).

¹³C NMR (200 MHz, CDCl₃) δ (ppm) 169.92, 160.07, 156.47, 156.07, 135.64, 135.47, 129.25, 128.68, 128.52, 128.36, 128.15, 127.16, 67.88, 53.63, 53.25, 37.59.

IR (KBr) ν 4000 - 3285 (br.), 3031, 2953, 1742, 1683, 1525, 1498, 1456, 1218, 1028, 980, 741, 697 cm⁻¹.

### Example 12: N'-[(S)-2-Benzyloxycarbonylamino-3-(1H-indol-3-yl)-propionyl]-hydrazine carboxylic acid 9H-fluoren-9-ylmethyl ester

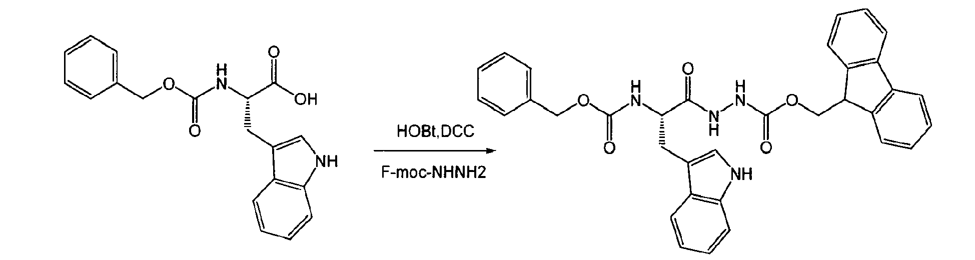

To the solution of Z-L-tryptophan (1.0 g, 2.96 mmol) in DCM (20 mL) was added HOBt (419.3 mg, 3.1 mmol) followed by DCC (640.3 mg, 3.1 mmol) and stirred for 30 min at room temperature. To this a suspension of F-moc- hydrazine (751.5 mg, 2.96 mmol) in DCM (10 mL) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (15 mL),10 % NaHCO₃ (15 mL), brine (15 mL), dried (Na₂SO₄) and concentrated to give 1.7 g of crude compound which was purified by column chromatography to give 1.39 g (81%) of product as a white solid; mp. 191-192°C.

[α]_{D} -30.2 (c 0.5, MeOH)

¹H NMR ( 200 MHz, DMSO-d₆) δ (ppm) 10.82 (s, 1H), 10.08 (br s, 1H), 9.37 (br s, 1H), 7.89 (d, *J* = 7.04 Hz, 2H), 7.67-7.77 (m, 3H), 6.94-7.46 (m, 14H), 4.93 (s, 2H), 4.34 (m, 4H), 2.88-3.19 (m, 2H).

¹³C NMR ( 200 MHz, DMSO-d₆) δ (ppm) 171.79, 155.99, 155.76, 143.62, 140.68, 136.88, 136.04, 128.22, 127.67, 127.41, 127.09, 125.27, 123.99, 120.82, 120.08, 118.48, 118.2, 111.26, 109.87, 66.15, 65.21, 53.93, 46.46, 27.84.

IR (KBr) ν 4000 - 3385, 3276, 3062, 2949, 1730, 1708, 1694, 1681, 1668, 1623 cm⁻¹.

### Example 13: N'-[(S)-2-tert-Butoxycarbonylamino-3-(1H-indol-3-yl)-propionyl]-hydrazine carboxylic acid 9H-fluoren-9-ylmethyl ester

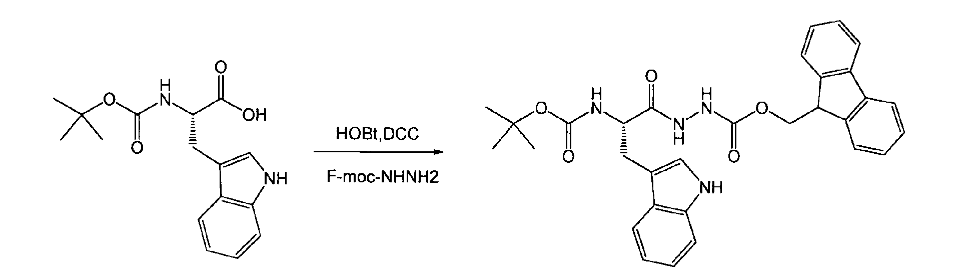

To the solution of Boc-L-tryptophan (1.0 g, 3.29 mmol) in DCM ( 20 mL ) was added HOBt (466.2 mg, 3.45 mmol) followed by DCC (711.8 mg, 3.45 mmol) and stirred for 30 min at room temperature. To this a suspension of F-moc- hydrazine (835.5 mg, 3.29 mmol) in DCM (10 mL) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (15 mL),10 % NaHCO₃ (15 mL), brine (15 mL), dried (Na₂SO₄) and concentrated to give 1.8 g of crude compound which was purified by column chromatography to give 1.56 g (87%) of product as a white solid, mp. 181-182°C.

R_{f} = 0.36 (1:1 petrol ether/EtOAc)

[α]_{D} -13.0 (*c* 0.5, MeOH)

¹H NMR ( 200 MHz, DMSO-d₆) δ (ppm) 10.8 (s, 1H), 9.97 (br s, 1H), 9.36 (br s, 1H), 7.89 (d, *J* = 7.04 Hz, 2H), 7.74 (d, *J* = 8.0 Hz, 2H) 7.65 (d, *J* = 7.24 Hz, 1H), 7.3-7.46 (m, 5H), 6.94-7.19 (m, 3H), 6.76 (d, *J* = 8.8 Hz, 1H), 4.28 (m, 4H), 2.85-3.16 (m, 2H), 1.29 (s,9H).

¹³C NMR ( 200 MHz, DMSO-d₆) δ (ppm) 171.96, 155.98, 155.10, 143.61, 140.69, 135.98, 127.67, 127.29, 127.08, 125.28, 123.80, 120.75, 120.11, 118.51, 118.11, 111.25, 109.93, 77.90, 59.72, 53.41, 46.48, 28.08, 27.79.

IR (KBr) ν 4000 - 3328, 3052, 2977, 2933, 1748, 1674, 1626 cm⁻¹.

### Example 14: N-[(S)-2-[N'-(9H-Fluoren-9-ylmethoxycarbonyl)-hydrazino]-1-(1H-indol-3-ylmethyl)-2-oxo-ethyl]-oxalamic acid tert-butyl ester (Fmoc-NTrpO-O-t-Bu)

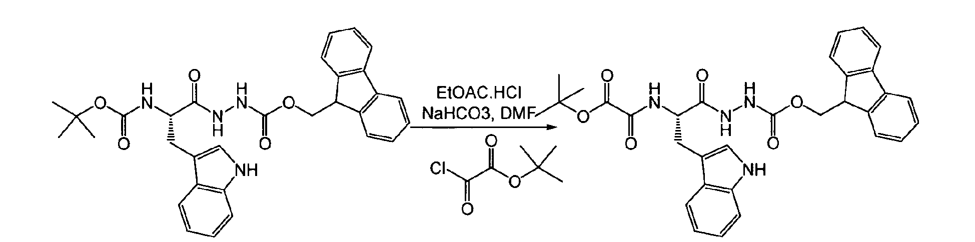

To the solution of N'-[(S)-2-tert-Butoxycarbonylamino-3-(1H-indol-3-yl)-propionyl]-hydrazine carboxylic acid 9H-fluoren-9-ylmethyl ester (Example 13) (1.0 g, 1.85 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (1.09 g, 12.95 mmol) was added to the reaction mixture, followed by *tert*-butyl oxalyl chloride (0.46 g, 2.78 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 882 mg (83 %) of product as a colourless viscous oil which after triturating with pentane gives off-white powder; mp. 157-159°C.

R*_{f}* = 0.24 (1:1 petrol ether/EtOAc)

[α]_{D} -37.2 (c 0.5, MeOH)

¹H NMR ( 200 MHz, CDCl₃) δ (ppm) 8.4 (br s, 1H), 8.19 (br s, 1H), 7.65-7.81 (m, 4H), 7.52 (d, *J* = 7.24 Hz, 2H), 6.96 - 7.4 (m, 9H), 4.72 - 4.82 (m, 1H), 4.12 - 4.41 (m, 3H), 3.28 (m, 2H), 1.46 (s, 9H).

¹³C NMR (200 MHz, CDCl₃) δ (ppm) 170.4, 158.43, 157.58, 156.06, 143.46, 143.39, 141.21, 136.12, 127.79, 127.16, 125.13, 123.95, 122.18, 119.97, 119.76, 118.53, 111.43, 109.23, 85.04, 68.01, 52.9, 46.76, 27.96, 27.59.

IR (KBr) ν 4000 - 3336 (br.), 2979, 1695, 1507, 1451, 1371, 1219, 1153, 758, 740 cm⁻¹.

### Example 15: (S)-3-(1H-Indol-3-yl)-2-(methoxyoxalyl-amino)-propionic acid

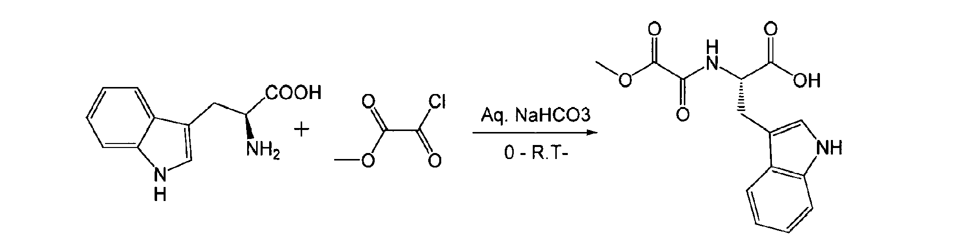

To the solution of L-tryptophan (200 mg, 0.98 mmol) in aq. NaHCO₃ (329.1 mg, 3.92 mmol, in 5 mL H₂O) was added drop wise a solution of mono-methyl oxalyl chloride (0.1 mL, 1.08 mmol) in THF (5 mL) at 0°. The reaction mixture was stirred at room temperature for 2 h. THF was removed by evaporation. The aqueous layer was washed with diethyl ether (2 x 10mL), acidified (pH 4) with 1 N HCL and extracted with ethyl acetate (3 x 10 mL). The combined organic layer was washed with brine (2 x 5 mL ), dried (Na₂SO₄) and concentrated to give 185 mg (65 %) of desired compound as a viscous oil.

¹H NMR ( 200 MHz, DMSO-d₆) δ (ppm) 10.83 (s, 1H), 8.93 (d, *J* = 8.6 Hz, 1H), 7.52 (d, *J* = 8.02 Hz,1H) 7.33 (d, *J* = 7.24 Hz, 1H), 6.93-7.14 (m, 3H), 4.47-4.58 (m, 1H), 3.74 (s, 3H), 3.13-3.33 (m, 2H).

### Example 16: N-[(S)-2-[N'-(9H-Fluoren-9-ylmethoxycarbonyl)-hydrazino]-1-(1H-indol-3-ylmethyl)-2-oxo-ethyl]-oxalamic acid methyl ester (Fmoc-NTrpO-OMe)

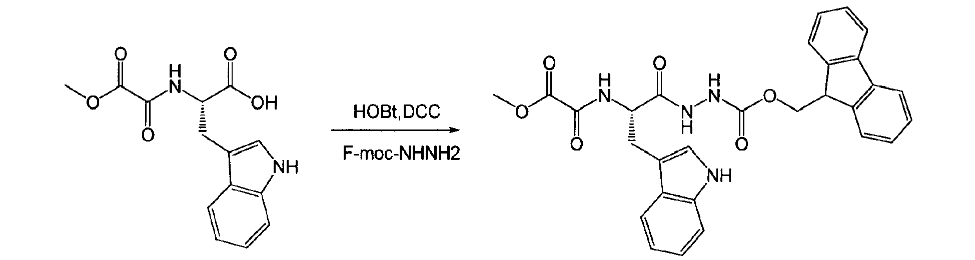

To the solution of (S)-3-(1H-Indol-3-yl)-2-(methoxyoxalyl-amino)-propionic acid (Example 15) (160 mg, 0.55 mmol) in DCM (10 mL) was added HOBt (78.21 mg, 0.58 mmol) followed by DCC (119.4 mg, 0.58 mmol) and stirred for 30 min at room temperature. To this F-moc- hydrazine (140.2 mg, 0.55 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (10 mL), 10 % NaHCO₃ (10 mL), brine (10 mL), dried (Na₂SO₄) and concentrated to give 265 mg of crude compound which was purified by column chromatography to give 135 mg (46%) of product as a white solid, mp. 141-142°C.

[α]_{D} -37.2 (c 0.5, MeOH)

¹H NMR ( 200 MHz, DMSO-d₆) δ (ppm) 10.86 (s, 1H), 10.18 (br s, 1H), 9.43 (br s, 1H), 8.88 (d, *J =* 7.62 Hz, 1H), 7.95 (d, *J* = 7.64 Hz, 2H), 7.78 (d, *J* = 7.04 Hz, 2H), 7.68 (d, *J* = 7.62 Hz, 1H), 7.35-7.51 (m, 5H), 6.98-7.23 (m, 3H), 4.67-4.74 (m, 1H), 4.32-4.43 (m, 3H), 3.77 (s, 3H), 3.13-3.32 (m, 2H).

¹³C NMR (200 MHz, DMSO-d₆) δ (ppm) 170.30, 160.75, 156.85, 155.98, 143.61, 140.69, 136.03, 127.67, 127.13, 125.29, 123.88, 120.94, 120.08, 118.35, 111.29, 109.34, 66.25, 52.80, 52.37, 46.40, 27.03.

IR (KBr) ν 4000 - 3389 (br.), 3040 (br.), 1741, 1680 cm-¹.

### Example 17: N'-[(S)-2-tert-Butoxycarbonylamino-3-(1H-indol-3-yl)-propionyl]-hydrazine carboxylic acid benzyl ester

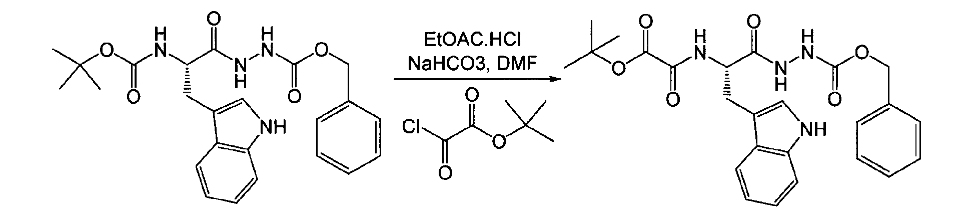

To the solution of Boc-L-tryptophan (1.5 g, 4.93 mmol) in DCM (30 mL) was added HOBt (0.7 g, 5.18 mmol) followed by DCC (1.07 g, 5.18 mmol) and stirred for 30 min at room temperature. To this Z- hydrazine (0.86 g, 5.18 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (30 mL), 10 % NaHCO₃ (30 mL), brine (30 mL), dried (Na₂SO₄) and concentrated to give 2.16 g (96%) of product as a viscous oil which after triturating with pentane gives off-white powder; mp. 88-89°C.

R*_{f}* = 0.42 (1:1 petrol ether/EtOAc)

[α]_{D} -18.2 (c 0.5, MeOH)

¹H NMR (200 MHz, CDCl₃) δ (ppm) 8.35 (br s, 2H), 7.49 (d, *J* = 7.44 Hz, 1H), 6.91-7.18 (m, 10H), 5.25 (br d, 1H), 4.94 (s, 2H), 4.45 (m, 1H), 3.1 (m, 2H), 1.26 (s, 9H).

¹³C NMR (200 MHz, CDCl₃) δ (ppm) 171.94, 156.21, 155.71, 135.97, 135.56, 128.52, 128.31, 128.1, 127.54, 123.67, 121.96, 119.47, 118.51, 111.29, 109.53, 80.51, 67.72. 53.79, 34.11, 28.2.

IR (KBr) ν 4000 - 3303 (br.), 3036, 2977, 1687 (br.), 1499, 1457, 1393, 1367, 1226, 1165, 1049, 741, 697, cm⁻¹.

### Example 18: N-[(S)-2-(N'-Benzyloxycarbonyl-hydrazino)-1-(1H-indol-3-ylmethyl)-2-oxo-ethyl]-oxalamic acid tert-butyl ester (Z-NTrpO-O-t-Bu)

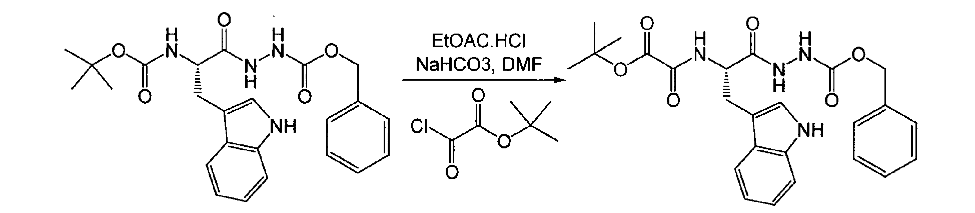

To the solution of *N*'-[(S)-2-*tert*-Butoxycarbonylamino-3-(1*H-*indol-3-yl)-propionyl]-hydrazine carboxylic acid benzyl ester (Example 17)(1.0 g, 2.21 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (1.3 g, 15.47 mmol) was added to the reaction mixture followed by tert-butyl oxalyl chloride (0.55 g, 3.32 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na2SO4) and concentrated to give 0.85 g (80 %) of product as a colourless viscous oil which after triturating with pentane gives off-white powder.

R*_{f}* = 0.32 (2:1 petrol ether/EtOAc)

[α]_{D} -56.8 (c 0.5, MeOH)

¹H NMR (200 MHz, CDCl₃) δ (ppm) 8.55 (br s, 1H), 8.26 (br s, 1H), 7.77 (d, *J* = 8.02 Hz, 1H), 7.63 (d, *J* = 7.42 Hz, 1H), 7.03-7.31 (m, 10H), 5.05 (s, 2H), 4.68-4.78 (m, 1H), 3.25 (m, 2H), 1.45 (s, 9H).

¹³C NMR (200 MHz, CDCl₃) δ (ppm) 170.47, 158.42, 157.53, 156.12, 136.12, 135.48, 128.54, 128.35, 128.10, 127.34, 124.05, 122.04, 119.66, 118.45, 111.47, 109.06, 85, 67.80, 52.92, 27.96, 27.58.

IR (KBr) v 4000 - 3328 (br.), 3058, 2981, 2934, 1736 (br.), 1692 (br.), 1517, 1457, 1371, 1219, 1153, 838, 742 cm⁻¹.

### Example 19: N-[(S)-2-(N'-Benzyloxycarbonyl-hydrazino)-1-(1H-indol-3-ylmethyl)-2-oxo-ethyl]-oxalamic acid methyl ester (Z-NTrpO-OMe)

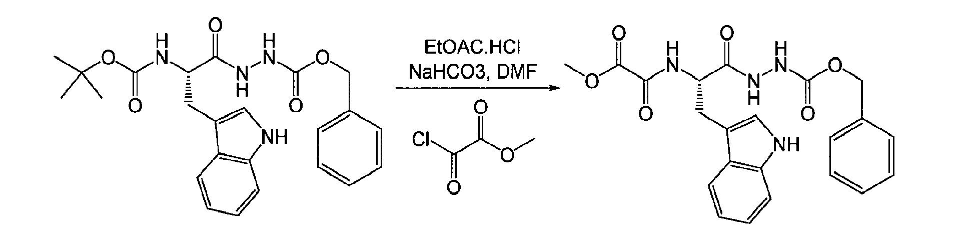

To the solution of *N*'-[(S)-2-*tert*-Butoxycarbonylamino-3-(1*H-*indol-3-yl)-propionyl]-hydrazine carboxylic acid benzyl ester (Example 17) (1.0 g, 2.21 mmol) in ethyl acetate (15 mL), ethyl acetate saturated with HCl (15 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (1.30 g, 15.47 mmol) was added to the reaction mixture, followed by methyl-oxalyl chloride (0.41 mL, 4.42 mmol) at 0°C under argon. The reaction mixture was stirred at room temperature for 30 min. Solvent was evaporated under high vacuum, the residue obtained was dissolved in water (50 mL) and extracted with ethyl acetate (3 x 20 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 569 mg (58 %) of product as a white solid; mp. 97-99°C.

R*_{f}* = 0.12 (1:1 EtOAc / Pentane)

[α]_{D} -37.4 (c 0.5, MeOH)

¹H NMR (200 MHz, DMSO-d₆) δ (ppm) 10.81 (s, 1H), 10.13 (s, 1H), 9.31 (s, 1H), 8.82 (d, *J* = 7.24 Hz, 1H), 6.94-7.63 (m, 10H), 5.1 (s, 2H), 4.63 (m, 1H), 3.72 (s, 3H), 3.19 (m, 2H).

¹³C NMR (200 MHz, DMSO-d₆) δ (ppm) 170.25, 160.75, 156.77, 156, 136.53, 135.99, 128.33, 127.93, 127.7, 127.06, 123.79, 120.88, 118.28, 111.27, 109.38, 65.95, 52.77, 52.36, 27.13.

IR (KBr) ν 4000 - 3299 (br), 3034, 2954, 1739, 1702, 1679, 1524, 1457, 1436, 1342, 1266, 1221, 1027, 1010, 743, 697 cm⁻¹.

### Example 20: Carbonic acid 2-bromo-benzyl ester 4-{(S)-2-tert-butoxycarbonylamino-3-[N'-(9H-fluoren-9-ylmethoxycarbonyl)-hydrazino]-3-oxo-propyl}-phenyl ester

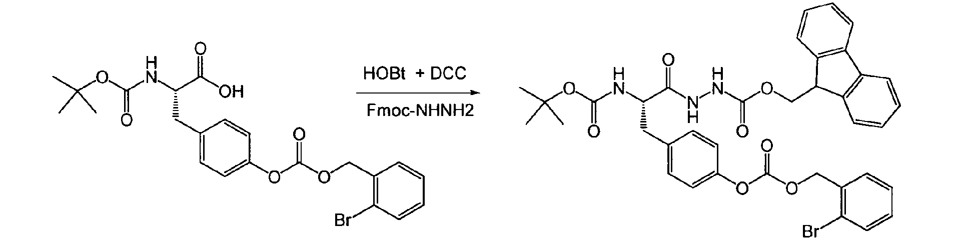

To the solution of Boc-L-Tyr(2-Br-Z)-OH (1.5 g, 3.03 mmol) in DCM (20 mL) was added HOBt (431 mg, 3.19 mmol) followed by DCC (657 mg, 3.19 mmol) and stirred for 30 min at room temperature. To this a suspension of F-moc- hydrazine (772 mg, 3.29 mmol) in DCM (10 mL) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (15 mL), 10 % NaHCO₃ (15 mL), brine (15 mL), dried (Na₂SO₄) and concentrated to give 2.5 g of crude compound which was purified by column chromatography to give 2.1 g (94 %) of product as a white solid; mp. 77-79°C.

R*_{f}* = 0.57 (1:1 petrol ether/EtOAc)

[α]_{D}, -5.8 (c 0.5, MeOH)

¹H NMR ( 200 MHz, DMSO-d₆) δ (ppm) 9.95 (br s, 1H), 9.37 (br s, 1H), 7.89 (d, *J* = 7.04 Hz, 2H), 7.68-7.75 (m, 3H), 7.55-7.67 (m, 1H), 7.33-7.54 (m, 8H),7.16 (d, *J* = 8.4 Hz, 2H), 6.98 (d, *J* = 8.02 Hz, 1H), 5.31 (s, 2H), 4.22-4.36 (m, 4H), 2.7-3.04 (m, 2H), 1.29 (s, 9H).

¹³C NMR ( 200 MHz, DMSO-d₆) δ (ppm) 171.56, 155.96, 155.17, 152.80, 149.25, 143.59, 140.69, 134.05, 132.66, 130.69, 130.29, 128.05, 127.66, 127.13, 125.29, 122.99, 120.68, 120.09, 78.04, 69.22, 63.25, 54.11, 46.44, 33.30, 28.10.

IR (KBr) ν 4000 - 3309, 3250, 2976, 1763, 1717, 1684, 1627 cm⁻¹.

### Example 21: N-{(S)-1-[4-(2-Bromo-benzyloxycarbonyloxy)-benzyl]-2-[N'-(9H-fluoren-9-ylmethoxycarbonyl)-hydrazino]-2-oxo-ethyl}-oxalamic acid methyl ester (Fmoc-NTyr(2-Br-Z)O-OMe)

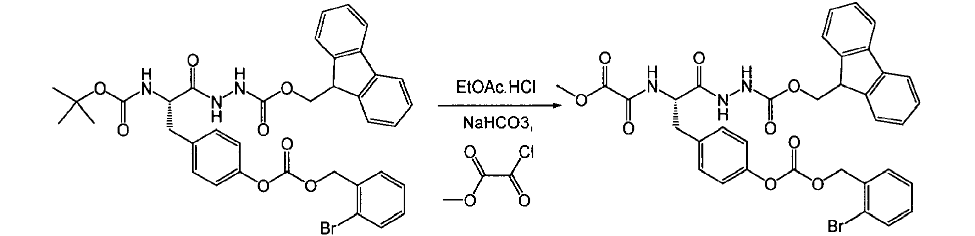

To the solution of Carbonic acid 2-bromo-benzyl ester 4-{(*S*)-2-*tert*-butoxycarbonylamino-3-[*N*'-(9*H*-fluoren-9-ylmethoxy-carbonyl)-hydrazino]-3-oxo-propyl}-phenyl ester (Example 20) (1.1 g, 1.51 mmol) in ethyl acetate (15 mL), ethyl acetate saturated with HCl (15 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (0.63 g, 7.53 mmol) was added to the reaction mixture, followed by methyl oxalyl chloride (0.15 mL, 0.35 mmol) at 0° under argon. The reaction mixture was stirred for 30 min at 0° and further at room temperature for 30 min. The mixture was diluted with water (50 mL) and extracted with ethyl acetate (3 x 30 mL), the combined organic layer was washed with 1N HCl (30 mL) followed by 10% NaHCO₃ (30 mL) and water (3 x 50 mL) followed by brine (20 mL). The ethyl acetate layer was dried (Na₂SO₄) and concentrated to give crude compound which was purified by column chromatography (silica gel) to give 957 mg (88 %) of product as a white powder; mp 205 - 206°C.

[α]_{D} -10.2 (c 0.5, DMF)

¹H NMR ( 200 MHz, DMSO-d₆) δ (ppm) 10.10 (br s, 1H), 9.38 (br s, 1H), 9.04 (br d, J= 9.2 Hz, 1H), 7.88 (d, J = 7.62 Hz, 2H), 7.67-7.70 (m, 3H), 7.32-7.57 (m, 9H), 7.14 (d, J = 9.18 Hz, 2H), 5.30 (s, 3H), 4.53-4.66 (m, 1H), 4.16-4.34 (m, 3H), 3.73 (s, 3H), 2.96-3.15 (m, 2H).

¹³C NMR ( 200 MHz, DMSO-d₆) δ (ppm) 169.82, 160.59, 156.87, 155.94, 152.76, 149.35, 143.61, 140.67, 135.47, 134.08, 132.76, 130.74, 130.20, 128.02, 127.64, 127.02, 125.24, 122.99, 120.82, 120.12, 69.21, 66.19, 53.01, 52.77, 46.49, 35.83.

IR (KBr) ν 4000 - 3382, 3296, 3019, 2955, 1765, 1735, 1709, 1683, 1509 cm⁻¹.

### Example 22: N-{(S)-1-[4-(2-Bromo-benzyloxycarbonyloxy)-benzyl]-2-[N'-(9H-fluoren-9-ylmethoxycarbonyl)-hydrazino]-2-oxo-ethyl}-oxalamic acid tert-butyl ester (Fmoc-NTyr (2-Br-Z) O-O-t-Bu)

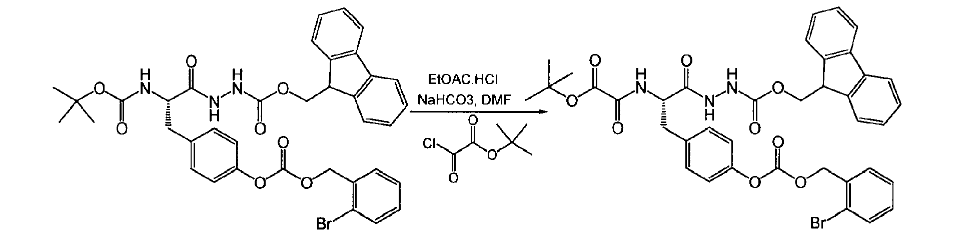

To the solution of Carbonic acid 2-bromo-benzyl ester 4-{(S)-2-*tert*-butoxycarbonylamino-3-[*N*'-(9*H*-fluoren-9-ylmethoxy-carbonyl)-hydrazino]-3-oxo-propyl}-phenyl ester (Example 20) (0.5 g, 0.68 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (0.4 g, 4.79 mmol) was added to the reaction mixture, followed by tert-butyl oxalyl chloride (0.17 g, 1.03 mmol) at 0°C under argon. The reaction mixture was stirred at room temperature for 30 min. The mixture was diluted with water (50 mL) and extracted with ethyl acetate (3 x 20 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 440 mg (84 %) of product as a colourless viscous oil which after triturating with pentane gives white powder; mp. 124-126°C.

R*_{f}* = 0.38 (1:1 petrol ether / EtOAc)

[α]_{D} -12.4 (*c* 0.5, MeOH)

¹H NMR (200 MHz, CDCl₃) δ (ppm) 8.53 (br s, 1H), 7.71-7.75 (m, 3H), 7.47-7.62 (m, 4H), 7.18-7.41 (m, 8H), 7.04-7.13 (m, 3H), 5.35 (s, 2H), 4.73 - 4.83 (m, 1H), 4.16 - 4.47 (m, 3H), 3.05-3.27 (m, 2H), 1.48 (s, 9H).

¹³C NMR (200 MHz, CDCl₃) δ (ppm) 169.82, 158.38, 157.78, 156.01, 153.35, 150.21, 143.44, 141.23, 134.19, 133.66, 132.93, 130.48, 130.17, 130.08, 127.78, 127.63, 127.14, 125.12, 123.45, 121.22, 119.96, 85.14, 69.63, 68.1, 53.17, 46.81, 36.92, 27.61.

IR (KBr) ν 4000 - 3291 (br.), 2979, 1761, 1695, 1509, 1451, 1372, 1220, 1153, 758, 740 cm⁻¹.

### Example 23: Carbonic acid 4-[(S)-3-(N'-benzyloxycarbonyl-hydrazino)-2-tert-butoxycarbonylamino-3-oxo-propyl]-phenyl ester 2-bromo-benzyl ester

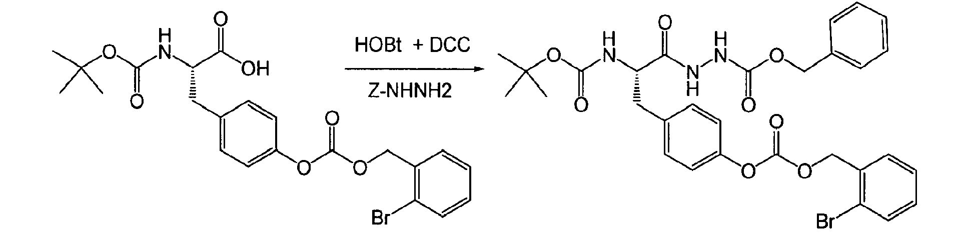

To the solution of Boc-L-Tyr(2-Br-Z)-OH (1.0 g, 2.02 mmol) in DCM (15 mL) was added HOBt (0.29 g, 2.12 mmol) followed by DCC (0.44 g, 2.12 mmol) and stirred for 30 min at room temperature. To this Z- hydrazine (0.35 g, 2.12 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (15 mL), 10 % NaHCO₃ (15 mL), brine (15 mL), dried (Na₂SO₄) and concentrated to give 1.26 g (96%) of product as a viscous oil which after triturating with pentane gives off-white powder; mp. 64-66°C.

R*_{f}* = 0.78 (1:1 petrol ether / EtOAc)

[α]_{D} -15.6 (c 0.5, MeOH)

¹H NMR (200 MHz, CDCl₃) δ (ppm) 8.61 (br s, 1H), 7.61 (d, *J* = 7.82 Hz, 1H), 7.5 (dd, *J =* 1.36, 1.18 Hz,1H), 7.07-7.38 (m, 12H), 5.36 (br s, 3H), 5.13 (s, 2H), 4.51 (m, 1H), 2.89-3.19 (m, 2H), 1.36 (s, 9H).

¹³C NMR (200 MHz, CDCl₃) δ (ppm) 171.39, 156.1, 155.74, 153.41, 150.02, 135.57, 134.29, 134.23, 132.93, 130.5, 130.16, 130.08, 128.52, 128.32, 128.15, 127.63, 123.46, 121, 80.62, 69.59, 67.78, 53.9, 37.48, 28.20.

IR (KBr) ν 4000 - 3287 (br), 3034, 2978, 2932, 1764, 1683 (br), 1509, 1379, 1368, 1219, 1163, 1028, 1018, 751 cm⁻¹ .

### Example 24: N-{(S)-2-(N'-Benzyloxycarbonyl-hydrazino)-1-[4-(2-bromo-benzyloxycarbonyloxy)-benzyl]-2-oxo-ethyl}-oxalamic acid tert-butyl ester (Z-NTyr(2-Br-Z)O-O-t-Bu)

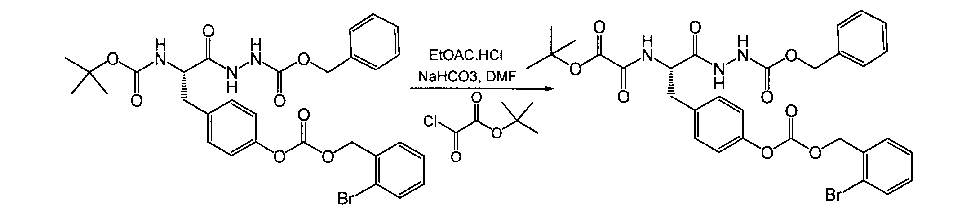

To the solution of Carbonic acid 4-[(S)-3-(*N*'-benzyloxycarbonyl-hydrazino)-2-tert-butoxycarbonylamino-3-oxo-propyl]-phenyl ester 2-bromo-benzyl ester (Example 23) (0.5 g, 0.78 mmol) in ethyl acetate (5 mL), ethyl acetate saturated with HCl (5 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (0.46 g, 5.45 mmol) was added to the reaction mixture followed by *tert*-butyl oxalyl chloride (0.19 g, 1.17 mmol) at 0°C under argon. The reaction mixture was stirred at room temperature for 30 min. The mixture was diluted with water (50 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 0.49 g (94 %) of product as a colourless viscous oil which after triturating with pentane gives off-white powder; mp. 93-94°C.

R*_{f}* = 0.73 (1:1 Pentane/EtOAc)

[α]_{D} -10.4 (c 0.5, MeOH)

¹H NMR (200 MHz, CDCl₃) δ (ppm) 8.53 (br s, 1H), 7.74 (d, *J* = 8.2 Hz, 1H), 7.61 (dd, *J* = 1.16, 1.16 Hz,1H), 7.5 (dd, *J* = 1.56, 1.74 Hz,1H), 7.0-7.39 (m, 12H), 5.35 (s, 2H), 5.12 (s, 2H), 4.7-4.81 (m, 1H), 2.95-3.25 (m, 2H), 1.48 (s, 9H).

¹³C NMR (200 MHz, CDCl₃) δ (ppm) 169.85, 158.4, 157.73, 156.02, 153.34, 150.18, 135.49, 134.20, 133.68, 132.93, 130.47, 130.17, 130.1, 128.53, 128.35, 128.15, 127.64, 123.46, 121.16, 85.06, 69.61, 67.85, 53.12, 36.91, 27.61.

IR (KBr) ν 4000 - 3287 (br.), 3035, 2981, 1760 (br.), 1508, 1372, 1218, 838, 750, 696 cm-¹.

### Example 25: N-{(S)-2-(N'-Benzyloxycarbonyl-hydrazino)-1-[4-(2-bromo-benzyloxycarbonyloxy)-benzyl]-2-oxo-ethyl}-oxalamic acid methyl ester (Z-NTyr(2-Br-Z)O-OMe)

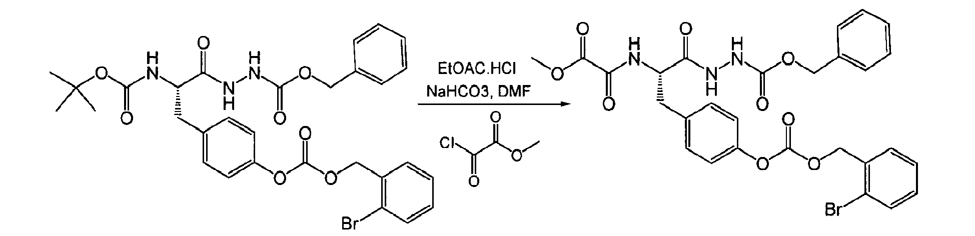

To the solution of Carbonic acid 4-[(S)-3-(N'-benzyloxycarbonyl-hydrazino)-2-tert-butoxycarbonylamino-3-oxo-propyl]-phenyl ester 2-bromo-benzyl ester (Example 23) (0.5 g, 0.78 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (0.46 g, 5.45 mmol) was added to the reaction mixture, followed by methyl-oxalyl chloride (0.14 mL, 1.56 mmol) at 0°C under argon. The reaction mixture was stirred at room temperature for 30 min. Solvent was evaporated under high vacuum, the residue obtained was dissolved in water (50 mL) and extracted with ethyl acetate (3 x 20 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 460 mg (94 %) of product as a white solid; mp.78-79°C.

R*_{f}* = 0.2 (1:1 EtOAc/Pentane)

[α]_{D} -9.0 (*c* 0.5, MeOH)

¹H NMR (200 MHz, CDCl₃) δ (ppm) 8.69 (br s, 1H), 7.92 (d, *J* = 7.82 Hz, 1H), 7.6 (dd, *J* = 0.98, 0.98 Hz, 1H), 7.49 (dd, *J* = 1.56, 1.58 Hz,1H), 7.06-7.38 (m, 12H), 5.34 (s, 2H), 5.10 (s, 2H), 4.75-4.82 (m, 1H), 3.76 (s, 3H), 3.0-3.26 (m, 2H).

¹³C NMR (200 MHz, CDCl₃) δ (ppm) 169.81, 160.05, 156.54, 156.12, 153.4, 150.15, 135.47, 134.17, 133.65, 132.93, 130.42, 130.19, 130.1, 128.53, 128.37, 128.13, 127.64, 123.46, 121.14, 69.64, 67.89, 53.7, 53.09, 36.93.

IR (KBr) ν 4000 - 3281 (br.), 3034, 2955, 1761, 1702, 1688, 1507, 1379, 1218, 1027, 1017, 750, 696 cm⁻¹.

### Example 26: (S)-2-(Methoxyoxalyl-amino)-4-methyl-pentanoic acid

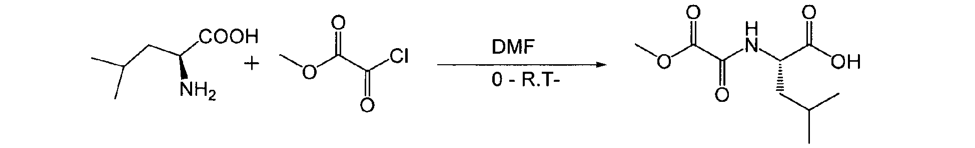

Mono- methyl oxalyl chloride (4.21 mL, 45.74 mmol) was added drop wise at 0°C to the solution of L-Leucine (2.0 g, 15.25 mmol) and NaHCO₃ (6.4 g, 76.24 mmol) in DMF (10 mL). The reaction mixture was stirred further at room temperature for 2 h. DMF was removed by evaporation, the residue obtained was dissolved in water (100 mL),washed with diethyl ether (2 x 30mL), acidified (pH 4) with 1 N HCL and extracted with ethyl acetate (3 x 30 mL). The combined organic layer was washed with brine (30 mL), dried (Na₂SO₄) and concentrated to give 3.2 g (96 %) of desired compound as a colourless viscous oil.

### Example 27: N-[(S)-1-(N'-tert-Butoxycarbonyl-hydrazinocarbonyl)-3-methyl-butyl]-oxalamic acid methyl ester (Boc-NLeuO-OMe)

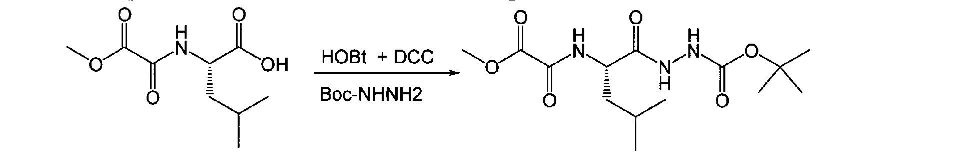

To the solution of (S)-2-(Methoxyoxalyl-amino)-4-methyl-pentanoic acid (Example 26) (3.2 g, 14.73 mmol) in DCM (50 mL) was added HOBt (2.09 g, 15.47 mmol) followed by DCC (3.19 g, 15.47 mmol) and stirred for 30 min at room temperature. To this Boc- carbazate (2.04 g, 15.47 mmol) was added and the mixture was stirred overnight at room temperature under argon.

The precipitated DCHU was removed by filtration and the filtrate was evaporated. The additional DCHU was removed by subsequent trituration with cold ethyl acetate and filtration. The ethyl acetate solution was washed with 1N HCl (20 mL), 10 % NaHCO₃ (20 mL), brine (20 mL), dried (Na₂SO₄) and concentrated to give 4.5g of crude compound which was purified by column chromatography to give 3.7 g (75 %) of product as a white solid; mp. soften at 71°C and melting at 83°C.

[α]_{D} - 43.4 (*c* 0.5, MeOH)

¹H NMR ( 200 MHz, CDCl₃) δ (ppm) 8.90 (br s, 1H), 7.87 (br d, *J* = 8.6 Hz, 1H), 6.89 (br s, 1H), 4.57-4.68 (m, 1H), 3.80 (s, 3H) 1.51-1.69 (m, 3H), 1.37 (s, 9H), 0.87 (dd, *J* = 5.66, 5.48 Hz, 6H).

¹³C NMR (200 MHz, CDCl₃) δ (ppm) 170.67, 160.43, 156.52, 155.36, 81.77, 53.64, 50.44, 40.64, 28.05, 24.54, 22.87, 21.66.

IR (KBr) ν 4000 - 3292 (br.), 2960, 2873, 1742, 1687 (br.) cm⁻¹.

### Example 28: N'-((S)-2-tert-Butoxycarbonylamino-4-methyl-pentanoyl)-hydrazine carboxylic acid 9H-fluoren-9-ylmethyl ester

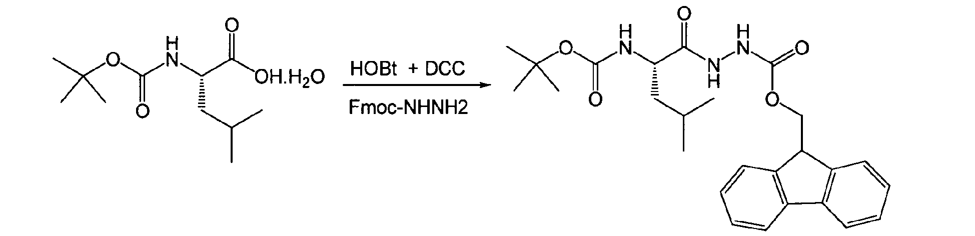

To the solution of Boc-L-Leucine monohydrate (4.0 g, 16.04 mmol) in DCM (50 mL) was added HOBt (2.28 g, 16.85 mmol) followed by DCC (3.48 g, 16.85 mmol) and stirred for 30 min at room temperature. To this a suspension of F-moc- hydrazine (4.08 g, 16.04 mmol) in DCM (20 mL) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (30 mL),10 % NaHCO₃ (30 mL), brine (30 mL), dried (Na₂SO₄) and concentrated to give 8.2 g of crude compound which was purified by column chromatography to give 7.2 g (95%) of product as a white solid; mp. soften at 82°C and melting at 92°C.

[α]_{D} -37.0 (c 0.5, MeOH)

¹H NMR ( 200 MHz, CDCl₃) δ (ppm) 9.04 (br s, 1H), 7.74 (d, *J* = 7.44 Hz, 2H), 7.58 (d, *J* = 7.22 Hz, 2H), 7.22-7.41 (m, 5H), 5.24-5.30 (m, 1H), 4.38 (d, *J* = 6.84 Hz, 2H), 4.18-4.32 (m, 1H), 1.58-1.84 (m, 3H), 1.54 (s, 9H), 0.95 (dd, *J* = 5.28, 5.48 Hz, 6H) .

¹³C NMR (200 MHz, CDCl₃) δ (ppm) 172.86, 156.21, 156.14, 143.54, 141.2, 127.72, 127.12, 125.2, 119.92, 80.51, 67.99, 51.3, 46.85, 41.15, 28.3, 24.57, 22.91, 21.84.

IR (KBr) ν 4000 - 3287 (br.), 2958, 2871, 1685 (br.) cm⁻¹.

### Example 29: N-{(S)-1-[N'-(9H-Fluoren-9-ylmethoxycarbonyl)-hydrazinocarbonyl]-3-methyl-butyl}-oxalamic acid methyl ester (Fmoc-NLeuO-OMe)

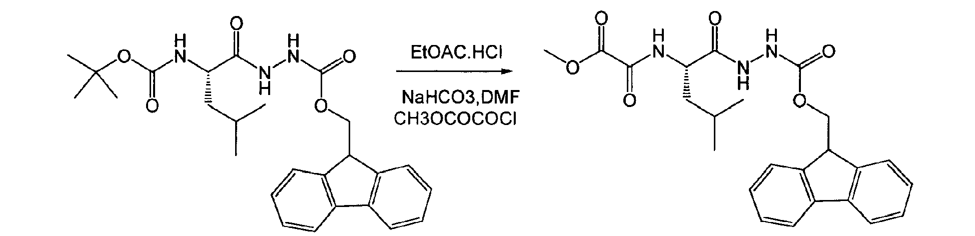

To the solution of *N*'-((S)-2-*tert*-Butoxycarbonylamino-4-methyl-pentanoyl)-hydrazine carboxylic acid 9H-fluoren-9-yl-methyl ester (Example 28) (3.87g, 8.28 mmol) in ethyl acetate (30 mL), ethyl acetate saturated with HCl (30 mL) was added slowly with stirring at 0°C. The reaction mixture was stirred at room temperature for 30 min. Solvent was evaporated and the reaction mixture was well dried under high vacuum. The residue obtained was dissolved in dry DMF (30 mL), NaHCO₃ (3.48 g, 41.39 mmol) was added to the reaction mixture, followed by methyl oxalyl chloride (0.84 mL, 9.11 mmol) at 0° under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 50 mL) followed by brine (20 mL). The ethyl acetate layer was dried (Na₂SO₄) and concentrated to give crude compound which was purified by column chromatography (silica gel) to give 3.4 g (90 %) of product as a white powder; mp. soften at 82°C and melting at 87.3°C .

[α]_{D} -42.0 (c 0.5, MeOH)

¹H NMR ( 200 MHz, CDCl₃) δ (ppm) 8.85 (s, 1H), 7.62-7.72 (m, 3H), 7.47 (d, *J* = 7.22 Hz, 2H), 7.13-7.32 (m, 5H), 4.56-4.68 (m, 1H), 4.29 (d, *J* = 7.24, 2H), 4.02-4.14 (m, 1H), 3.72 (s, 3H) 1.5-1.79 (m, 3H), 0.84 (dd, *J* = 5.48 Hz, 6H).

¹³C NMR (200 MHz, CDCl₃) δ (ppm) 170.95, 160.45, 156.47, 156.22, 143.55, 141.24, 127.79, 127.15, 125.23, 119.98, 68.11, 53.76, 50.68, 46.85, 40.7, 24.69, 22.9, 21.75.

IR (KBr) ν 4000 - 3283 (br.), 2956, 2925, 2854, 1739, 1684 (br.) cm⁻¹.

### Example 30: N-{(S)-1-[N'-(9H-Fluoren-9-ylmethoxycarbonyl)-hydrazinocarbonyl]-3-methyl-butyl}-oxalamic acid tert-butyl ester (Fmoc-NLeuO-O-t-Bu)

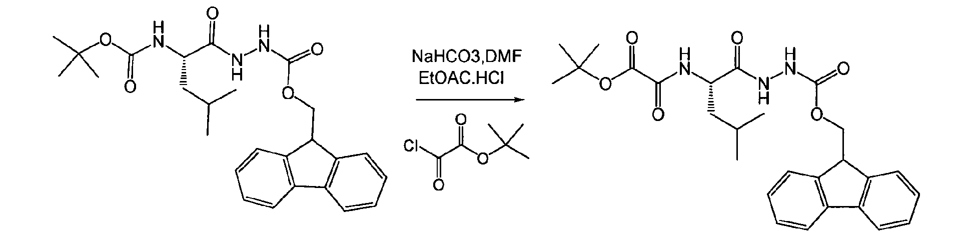

To the solution of *N*'-((S)-2-tert-Butoxycarbonylamino-4-methyl-pentanoyl)-hydrazine carboxylic acid 9H-fluoren-9-yl-methyl ester (Example 28) (1.16 g, 2.48 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (1.46 g, 17.37 mmol) was added to the reaction mixture, followed by *tert*-butyl oxalyl chloride (0.61 g, 3.72 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 1.0 g (88 %) of product as a colourless viscous oil which after triturating with pentane gives off-white powder; mp. 91-93°C.

*R_{f}* = 0.63 (1:1 petrol ether/EtOAc)

[α]_{D} -35.2 (c 0.5, MeOH)

¹H NMR (200 MHz, CDCl₃) δ (ppm) 8.91 (br s, 1H), 7.55-7.76 (m, 5H), 7.23-7.41 (m, 4H), 7.12 (br s, 1H), 4.66-4.74 (m, 1H), 4.17-4.45 (m, 3H), 1.19-1.84 (m, 12H), 0.86-0.97 (m, 6H).

¹³C NMR (200 MHz, CDCl₃) δ (ppm) 171.04, 158.71, 157.81, 156.1, 143.49, 141.21, 127.76, 127.13, 125.16, 119.94, 85.07, 68.18, 50.56, 46.8, 40.75, 27.62, 24.62, 22.76, 21.99.

IR (KBr) ν 4000 - 3286 (br.), 2958, 1703, 1684, 1520, 1451, 1370, 1304, 1217, 1155, 759, 740 cm⁻¹.

### Example 31: N'-((S)-2-tert-Butoxycarbonylamino-4-methyl-pentanoyl)-hydrazine carboxylic acid benzyl ester

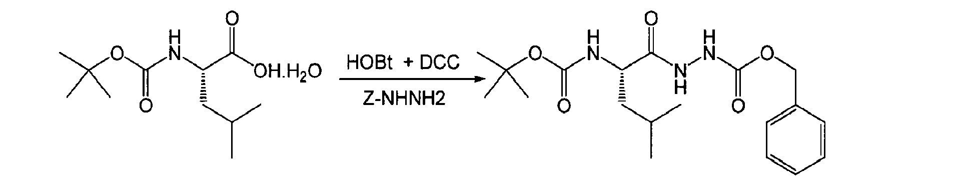

To the solution of Boc-L-Leucine (1.5 g, 6.02 mmol) in DCM (30 mL) was added HOBt (0.85 g, 6.32 mmol) followed by DCC (1.3 g, 6.32 mmol) and stirred for 30 min at room temperature. To this Z- hydrazine (1.05 g, 6.32 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (30 mL),10 % NaHCO₃ (30 mL), brine (30 mL), dried (Na₂SO₄) and concentrated to give 2.16 g (94%) of product as a viscous oil which after triturating with pentane gives off-white powder; mp. 57-59°C.

R_{f} = 0.76 (1:1 petrol ether/EtOAc)

[α]_{D} -42.6 (c 0.5, MeOH)

¹H NMR (200 MHz, CDCl₃) δ (ppm) 8.96 (br s, 1H), 7.31 (s, 6H), 5.21 (br s, 1H), 5.12 (s, 2H), 4.3 (m, 1H), 1.51-1.71 (m, 3H), 1.4 (s, 9H), 0.88-0.93 (m, 6H).

¹³C NMR (200 MHz, CDCl₃) δ (ppm) 172.85, 156.23, 156.04, 135.63, 128.46, 128.24, 128.12, 80.49, 67.64, 51.21, 41.16, 28.24, 24.5, 22.85, 21.82.

IR (KBr) ν 4000 - 3296 (br.), 2959, 1683 (br.), 1521, 1393, 1368, 1221, 1166, 1047, 741, 696, cm⁻¹.

### Example 32: N-[(S)-1-(N'-Benzyloxycarbonyl-hydrazinocarbonyl)-3-methyl-butyl]-oxalamic acid tert-butyl ester (Z-NLeuO-O-t-Bu

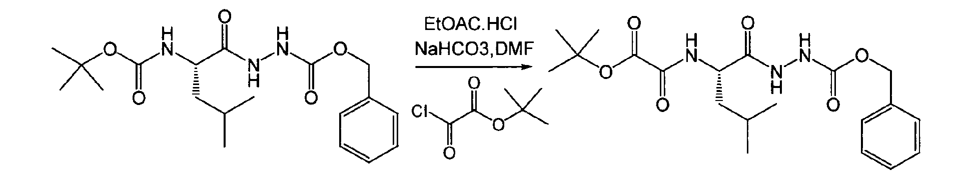

To the solution of *N*'-((S)-2-*tert*-Butoxycarbonylamino-4-methyl-pentanoyl)-hydrazine carboxylic acid benzyl ester (Example 31) (1.0 g, 2.64 mmol) in ethyl acetate (10 mL), ethyl acetate saturated with HC1 (10 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (1.55 g, 18.45 mmol) was added to the reaction mixture followed by tert-butyl oxalyl chloride (0.65 g, 3.95 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 0.84 g (78 %) of product as a colourless viscous oil which after triturating with pentane gives off-white powder; mp. 69-71°C.

R_{f} = 0.72 (1:1 Pentane/EtOAc)

[α]_{D} -41.6 (c 0.5, MeOH)

¹H NMR (200 MHz, CDCl₃) δ (ppm) 8.85 (br s, 1H), 7.66 (br s, 1H), 7.31 (s, 5H), 7.04 (br s, 1H), 5.18 (s, 2H), 4.59-4.63 (m, 1H), 1.61-1.82 (m, 3H), 1.51 (s, 9H), 0.90 (br s, 6H).

¹³C NMR (200 MHz, CDCl₃) δ (ppm) 171.02, 158.73, 157.72, 156.07, 135.52, 128.5, 128.29, 128.15, 84.95, 67.78, 50.51, 40.78, 27.61, 24.55, 22.71, 21.94.

IR (KBr) ν 4000 - 3294 (br.), 2960, 1685 (br.), 1523, 1370, 1306, 1218, 1156, 842, 741, 697 cm⁻¹.

### Example 33: N-[(S)-1-(N'-Benzyloxycarbonyl-hydrazinocarbonyl)-3-methyl-butyl]-oxalamic acid methyl ester (Z-NLeuO-OMe)

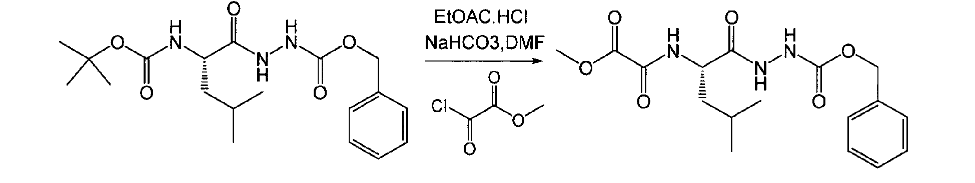

To the solution of *N*'-((S)-2-*tert*-Butoxycarbonylamino-4-methyl-pentanoyl)-hydrazine carboxylic acid benzyl ester (Example 31) (1.0 g, 2.64 mmol) in ethyl acetate (15 mL), ethyl acetate saturated with HCl (15 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (1.55 g, 18.45 mmol) was added to the reaction mixture, followed by methyl-oxalyl chloride (0.49 mL, 5.27 mmol) at 0°C under argon. The reaction mixture was stirred at room temperature for 30 min. Solvent was evaporated under high vacuum, the residue obtained was dissolved in water (50 mL) and extracted with ethyl acetate (3 x 20 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 771 mg (88 %) of product as a white solid.

R*_{f}* = 0.24 (1:1 EtOAc/Pentane)

[α]_{D} -43.8 (c 0.5, MeOH)

¹H NMR (200 MHz, CDCl₃) δ (ppm) 8.97 (br s, 1H), 7.82 (br s, 1H), 7.3 (br s, 6H), 5.1 (s, 2H), 4.63-4.66 (m, 1H), 3.79 (s, 3H), 1.68 (m, 3H), 0.89 (br s, 6H).

¹³C NMR (200 MHz, CDCl₃) δ (ppm) 171.99, 160.26, 156.62, 156.16, 135.5, 128.5, 128.31, 128.14, 67.8, 53.65, 50.55, 40.69, 24.55, 22.73, 21.74.

IR (KBr) ν 4000 - 3287 (br), 3036, 2958, 1686 (br), 1525, 1456, 1217, 1043, 986, 741, 697 cm⁻¹.

### Example 34: (S)-2-[N'-(9H-Fluoren-9-ylmethoxycarbonyl)-hydrazinocarbonyl]-pyrrolidine-1-carboxylic acid tert-butyl ester

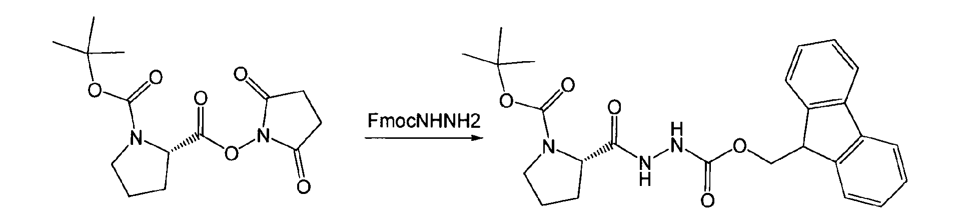

To the solution of Boc-L-Proline succinamide ester (0.5 g, 1.6 mmol) in DCM (15 mL) Fmoc- hydrazine (0.41 g, 1.6 mmol) was added and the mixture was stirred overnight at room temperature.

The reaction mixture was diluted with ethyl acetate (50 mL) and washed with 1N HCl (15 mL), 10% NaHCO₃ (15 mL), brine (15 mL), dried (Na₂SO₄) and concentrated to give 701 mg (96%) of product as a white solid; mp. 81-82°C.

R*_{f}* = 0.3 (1:1 EtOAc/Pentane)

[α]_{D} -59.8 (c 0.5, MeOH)

¹H NMR (200 MHz, CDCl₃) δ (ppm) 8.85 (br s, 1H), 7.65 (d, *J* = 7.24 Hz, 2H), 7.5 (d, *J* = 7.24 Hz, 2H), 7.11-7.33 (m, 5H), 4.25-4.34 (m, 3H), 4.1-4.17 (m, 1H), 3.37 (m, 2H), 1.69-2.08 (m, 4H), 1.38 (s, 9H).

¹³C NMR (200 MHz, CDCl₃) δ (ppm) 172.02, 171.17, 156.07, 143.56, 141.23, 127.73, 127.11, 125.15, 119.93, 80.85, 67.92, 58.38, 47.1, 46.9, 28.36, 24.42, 14.17.

IR (KBr) ν 4000 - 3277 (br), 2977, 1696 (br), 1451, 1405, 1366, 1246, 1164, 759, 740 cm⁻¹.

### Example 35: (S)-2-(N'-Benzyloxycarbonyl-hydrazinocarbonyl)-pyrrolidine-1-carboxylic acid tert-butyl ester

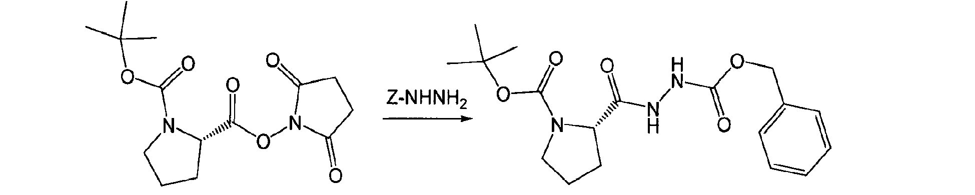

To the solution of Boc-L-Proline succinamide ester (1.0 g, 3.2 mmol) in DCM (15 mL) Fmoc- hydrazine (0.56 g, 3.2 mmol) was added and the mixture was stirred overnight at room temperature.

The reaction mixture was diluted with ethyl acetate (50 mL) and washed with 1N HCl (15 mL),10 % NaHCO₃ (15 mL), brine (15 mL), dried (Na₂SO₄) and concentrated to give 1.1 g (94%) of product as a sticky mass.

R*_{f}* = 0.28 (1:1 EtOAc/Pentane)

[α]_{D} -82.0 (c 0.5, MeOH)

¹H NMR (200 MHz, CDCl₃) δ (ppm) 8.76 (br s, 1H), 7.25 (s, 5H), 6.94 (br s, 1H), 5.07 (s, 2H), 4.22 (br s, 1H), 3.32 (br s, 2H), 1.68-2.21 (m, 4H), 1.37 (s, 9H).

¹³C NMR (200 MHz, CDCl₃) δ (ppm) 171.99, 156.02, 135.66, 128.48, 128.25, 128.1, 80.79, 67.64, 58.24, 47.03, 28.32, 24.41, 14.15.

IR (KBr) ν 4000 - 3280 (br), 2979, 1699 (br), 1456, 1404, 1367, 1219, 1164, 742, 697 cm⁻¹.

### Example 36: {(S)-2-[N'-(9H-Fluoren-9-ylmethoxycarbonyl)-hydrazinocarbonyl]-pyrrolidin-1-yl}-oxo-acetic acid tert-butyl ester (Fmoc-NProO-O-t-Bu)

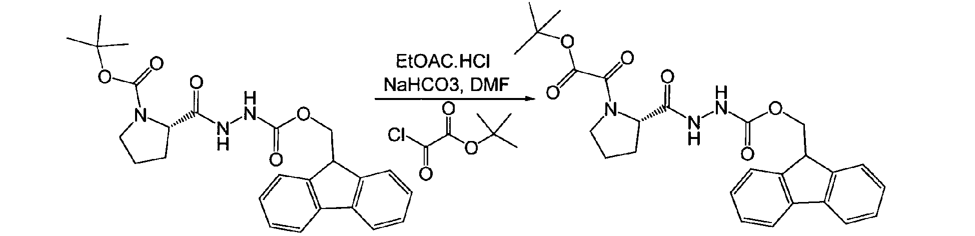

To the solution of (S)-2-[*N*'-(9*H*-Fluoren-9-ylmethoxycarbonyl)-hydrazinocarbonyl]-pyrrolidine-1-carboxylic acid *tert-*butyl ester (Example 34) (0.6 g, 1.33 mmol) in ethyl acetate (15 mL), ethyl acetate saturated with HCl (15 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (0.78 g, 9.3 mmol) was added to the reaction mixture, followed by *tert*-butyl oxalyl chloride (0.33 g, 1.99 mmol) at 0°C under argon. The reaction mixture was stirred at room temperature for 30 min. Solvent was evaporated under high vacuum, the residue obtained was dissolved in water (50 mL) and extracted with ethyl acetate (3 x 20 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 637 mg (99 %) of product as a white solid; mp. 86-87°C.

R*_{f}* = 0.13 (1:1 EtOAc/Pentane)

[α]_{D} -68.6 (c 0.5, MeOH)

¹H NMR (200 MHz, CDCl₃) δ (ppm) 8.83 (s, 1H), 7.73 (d, *J* = 7.04 Hz, 2H), 7.58 (d, *J* = 7.24 Hz, 2H), 7.17-7.41 (m, 5H), 4.57-4.74 (m, 1H), 4.37-4.41 (m, 2H), 4.19-4.26 (m, 1H), 3.63-3.81 (m, 2H), 1.94-2.4 (m, 4H), 1.53 (s, 9H). (a small percentage of other rotamer was also observed )

¹³C NMR (200 MHz, CDCl₃) δ (ppm) 170.47, 160.85, 160.56, 156.1, 143.59, 141.21, 127.71, 127.13, 125.21, 119.91, 84.81, 68.01, 58.49, 48.22, 46.85, 27.86, 27.74, 24.91.

IR (KBr) ν 4000 - 3282 (br), 2980, 1733, 1700, 1656, 1652, 1451,1370, 1252, 1149, 759, 740 cm⁻¹.

### Example 37: [(S)-2-(N'-Benzyloxycarbonyl-hydrazinocarbonyl)-pyrrolidin-1-yl]-oxo-acetic acid tert-butyl ester (Z-NProO-O-t-Bu)

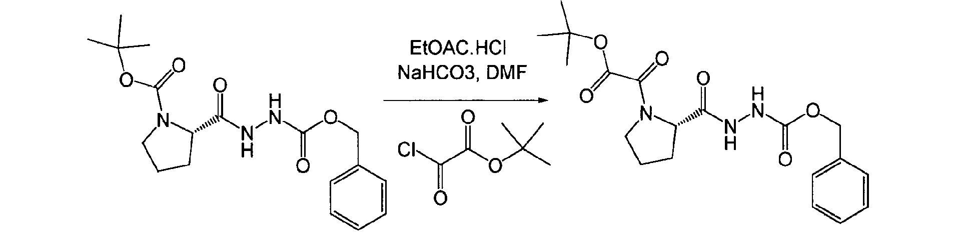

To the solution of (S)-2-(*N*'-Benzyloxycarbonyl-hydrazinocarbonyl)-pyrrolidine-1-carboxylic acid t*ert-*butyl ester (Example 35) (0.75 g, 2.06 mmol) in ethyl acetate (15 mL), ethyl acetate saturated with HCl (15 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (1.21 g, 14.45 mmol) was added to the reaction mixture, followed by *tert*-butyl oxalyl chloride (0.51 g, 3.1 mmol) at 0°C under argon. The reaction mixture was stirred at room temperature for 30 min. Solvent was evaporated under high vacuum, the residue obtained was dissolved in water (50 mL) and extracted with ethyl acetate (3 x 20 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 807 mg (99 %) of product as a white solid; mp. 51-52 °C.

R*_{f}* = 0.15 (1:1 EtOAc/Pentane)

[α]_{D} -70.8 (c 0.5, MeOH)

¹H NMR (200 MHz, CDCl₃) δ (ppm) 8.76 (br s, 1H), 7.31 (s, 5H), 7.12 (br s, 1H), 5.12 (s, 2H), 4.5-4.67 (m, 1H), 3.6-3.66 (m, 2H), 1.85-2.26 (m, 4H), 1.53 (s, 9H). **(Minor percentage of other rotamer was also observed )**

¹³C NMR (200 MHz, CDCl₃) δ (ppm) 170.47, 160.81, 160.39, 156.06, 135.73, 128.46, 128.18, 128.07, 84.69, 67.59, 58.46, 48.16, 27.85, 27.68, 24.81.

IR (KBr) ν 4000 - 3290 (br), 2982, 1733 (br), 1651(br), 1456,1371, 1253, 115, 742, 698 cm⁻¹.

### Example 38: [(S)-2-(N'-Benzyloxycarbonyl-hydrazinocarbonyl)-pyrrolidin-1-yl]-oxo-acetic acid methyl ester (Z-NProO-OMe)

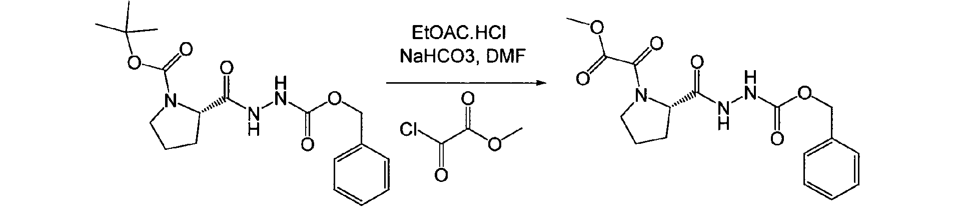

To the solution of (S)-2-(N'-Benzyloxycarbonyl-hydrazinocarbonyl)-pyrrolidine-1-carboxylic acid *tert*-butyl ester (Example 35) (0.75 g, 2.06 mmol) in ethyl acetate (15 mL), ethyl acetate saturated with HCl (15 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (1.21 g, 14.45 mmol) was added to the reaction mixture, followed by *mono*-methyl oxalyl chloride (0.38 mL, 4.13 mmol) at 0°C under argon. The reaction mixture was stirred at room temperature for 30 min. Solvent was evaporated under high vacuum, the residue obtained was dissolved in water (50 mL) and extracted with ethyl acetate (3 x 20 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 620 mg (86 %) of product as a thick sticky mass.

### Example 39: N'-((DL)-2-tert-Butoxycarbonylamino-3-phenyl-propionyl)-hydrazine carboxylic acid 9H-fluoren-9-ylmethyl ester

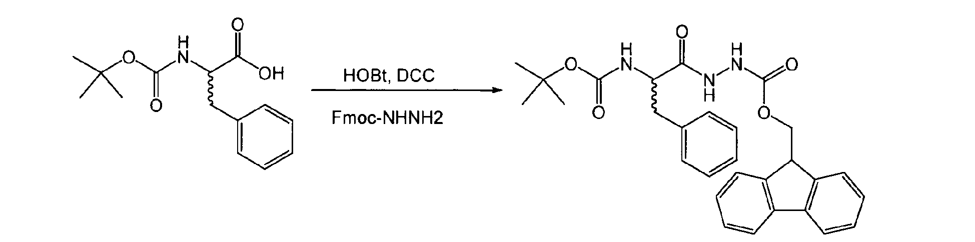

To the solution of Boc-L-phenylalanine (0.4 g, 1.51 mmol) in DCM (30 mL) was added HOBt (0.21 g, 1.58 mmol) followed by DCC (0.33 g, 1.58 mmol) and stirred for 30 min at room temperature. To this F-moc- hydrazine (0.4 g, 1.58 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (15 mL), 10 % NaHCO₃ (15 mL), brine (15 mL), dried (Na₂SO₄) and concentrated to give 730 mg of crude compound which was purified by column chromatography to give 514 mg (67%) of product as a white solid.

### Example 40: N'-((DL)-2-tert-Butoxycarbonylamino-3-phenyl-propionyl)-hydrazine carboxylic acid benzyl ester

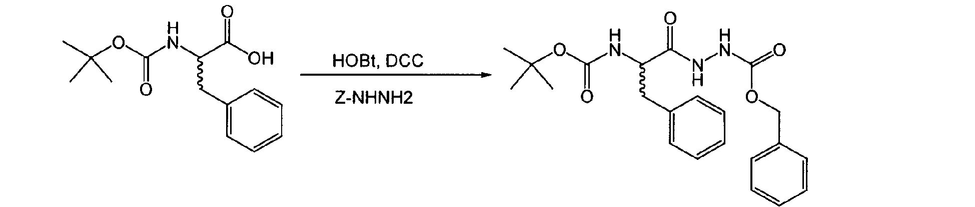

To the solution of Boc-L-phenylalanine (0.4 g, 1.51 mmol) in DCM (30 mL) was added HOBt (0.21 g, 1.58 mmol) followed by DCC (0.33 g, 1.58 mmol) and stirred for 30 min at room temperature. To this Z- hydrazine (0.26 g, 1.58 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (15 mL), 10 % NaHCO₃ (15 mL), brine (15 mL), dried (Na₂SO₄) and concentrated to give 650 mg of crude compound which was purified by column chromatography to give 617 mg (98%) of product as a white solid.

### Example 41: N-{(DL)-1-Benzyl-2-[N'-(9H-fluoren-9-ylmethoxycarbonyl)-hydrazino]-2-oxo-ethyl}-oxalamic acid tert-butyl ester (Fmoc-N(DL)PheO-O-O-tBu)

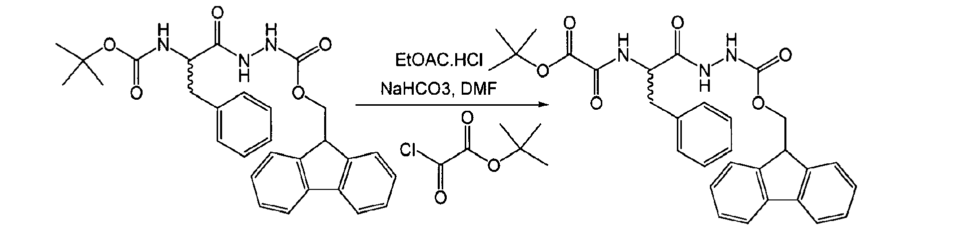

To the solution of *N*'-((DL)-2-*tert*-Butoxycarbonylamino-3-phenyl-propionyl)-hydrazine carboxylic acid 9H-fluoren-9-yl-methyl ester (Example 39) (0.5 g, 1.0 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (0.59 g, 6.98 mmol) was added to the reaction mixture, followed by *tert*-butyl oxalyl chloride (0.25 g, 1.5 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 450 mg of crude compound which was purified by column chromatography to give 278 mg (52%) of product as a white solid.

### Example 42: N-[(DL)-1-Benzyl-2-(N'-benzyloxycarbonyl-hydrazino)-2-oxo-ethyl]-oxalamic acid tert-butyl ester (Z-N(DL)PheO-O-O-tBu)

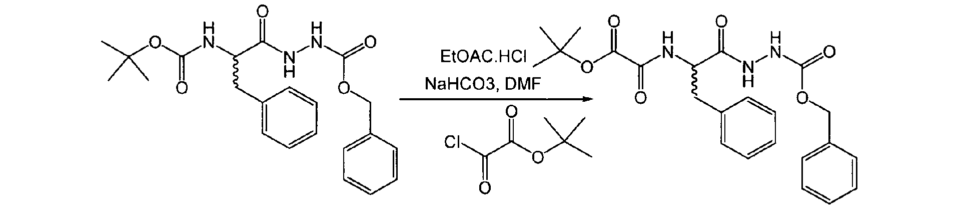

To the solution of N'-((DL)-2-tert-Butoxycarbonylamino-3-phenyl-propionyl)-hydrazine carboxylic acid benzyl ester (Example 40) (0.5 g, 1.21 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (10 mL), NaHCO₃ (0.71 g, 8.47 mmol) was added to the reaction mixture, followed by *tert*-butyl oxalyl chloride (0.3 g, 1.8 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 430 mg of crude compound which was purified by column chromatography to give 304 mg (56%) of product as a white solid.

### Example 43: N'-((R)-2-tert-Butoxycarbonylamino-3-phenyl-propionyl)-hydrazine carboxylic acid 9H-fluoren-9-ylmethyl ester

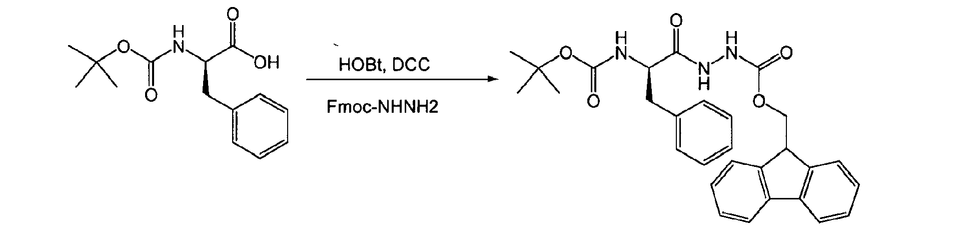

To the solution of Boc-D-phenylalanine (0.5 g, 1.89 mmol) in DCM (30 mL) was added HOBt (287 mg, 1.98 mmol) followed by DCC (408 g, 1.98 mmol) and stirred for 30 min at room temperature. To this F-moc- hydrazine (503 g, 1.98 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (15 mL),10 % NaHCO₃ (15 mL), brine (15 mL), dried (Na₂SO₄) and concentrated. The crude compound which was purified by column chromatography to give 710 mg (93%) of product as a white solid.

R*_{f}* = 0.26 ( 1.5 : 3.5 EtOAc/petrol ether)

[α]_{D} +10.8 (c 0.5, MeOH)

¹H NMR (200 MHz, DMSO-d₆) δ (ppm) 9.94 (br s, 1H, *d₂o exch.),* 9.35 (br s, 1H, *d₂o exch.),* 7.88 (d, *J* = 6.86 Hz, 2H), 7.73 (d, *J* = 7.04 Hz, 2H), 7.17 - 7.45 (m, 9H), 6.93 (d, *J =* 8.6 Hz, 1H, *d₂o exch*.), 4.28 - 4.31 (m, 4H), 2.69 - 3.0 (m, 2H), 1.27 (s, 9H).

¹³C NMR (200 MHz, DMSO-d₆) δ (ppm) 171.67, 155.95, 155.13, 143.58, 140.65, 137.94, 129.15, 127.95, 127.64, 127.06, 126.16, 125.24, 120.05, 79.09, 77.93, 66.11, 54.16, 46.44, 28.05.

### Example 44: N-{(R)-1-Benzyl-2-[N'-(9H-fluoren-9-ylmethoxycar-bonyl)-hydrazino]-2-oxo-ethyl}-oxalamic acid tert-butyl ester (Fmoc-N(D)PheO-O-O-tBu)

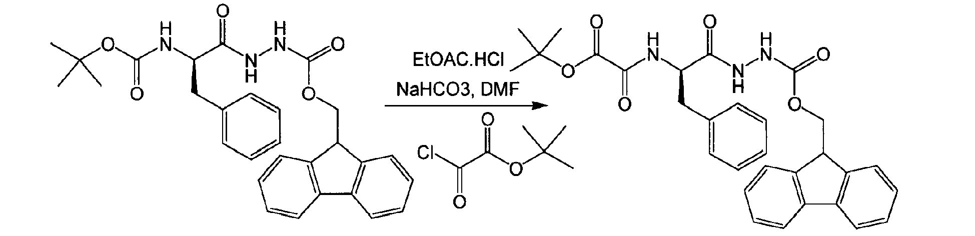

To the solution of *N*'-((R)-2-tert-Butoxycarbonylamino-3-phenyl-propionyl)-hydrazine carboxylic acid 9H-fluoren-9-yl-methyl ester (Example 43) (1.0 g, 1.99 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. The residue obtained was dissolved in dry DMF (25 mL), NaHCO₃ (1.17 g, 13.96 mmol) was added to the reaction mixture, followed by *tert*-butyl oxalyl chloride (492 mg, 2.99 mmol) at 0°C under argon. The reaction mixture was stirred at room temperature for 30 min. The mixture was diluted with water (100 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 835 mg (79 %) of product as a white powder.

R*_{f}* = 0.29 ( 1.5 : 3.5 EtOAc/petrol ether)

[α]_{D} +18.4 (c 0.5, DMF)

¹H NMR ( 200 MHz, CDCl₃) δ (ppm) 8.49 (br s, 1H, *d₂O exch.),* 7.64 (d, *J* = 7.42 Hz, 3H, 1H, *d₂O exch.),* 7.46 (d, *J* = 7.42 Hz, 2H), 7.14-7.31 (m, 9H), 6.96 (br s, 1H,*d₂*O *exch.),* 4.66-4.77 (m, 1H), 4.21-4.35 (m, 2H), 4.06-4.13 (m, 1H), 2.96-3.19 (m, 2H), 1.37 (s, 9H).

¹³C NMR (200 MHz, CDCl3) δ (ppm) 170.02, 158.36, 157.78, 156.03, 143.46, 141.23, 135.69, 129.32, 128.74, 127.77, 127.22, 127.14, 125.14, 119.95, 85.01, 68.11, 53.35, 46.80, 37.59, 27.61.

### Example 45: (S)-6-Benzyl-[1,2,5] triazepane-3,4,7-trione

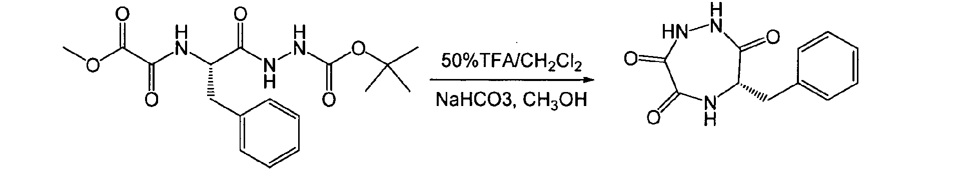

To the solution of *N*-[(S)-1-Benzyl-2-(*N*'-*tert*-butoxycarbonyl-hydrazino)-2-oxo-ethyl]-oxalamic acid methyl ester (Example 3) (1.0 g, 2.74 mmol) in CH₂Cl₂ (15 mL) was added TFA (15 mL) drop wise, the reaction mixture was further stirred at room temperature for 30 min under argon. The reaction mixture was then evaporated on rotavap and dried well under high vacuum. The residue obtained was dissolved in dry CH₂Cl₂ (15 mL) and washed with 10 % NaHCO₃. The aqueous solution was extracted with CH₂Cl₂ (3 x 15 mL), the combined organic layer was dried over Na₂SO₄ and evaporated under reduced pressure. The residue obtained was refluxed overnight in methanol (15 mL) under nitrogen. The solvent was evaporated and the crude compound obtained was purified by column chromatography to give 350 mg (54%) of desired compound as a white solid.

¹H NMR (200 MHz, CDCl₃) δ (ppm) 8.3 (br s, 1H), 8.1 (br s, 1H), 7.1-7.4 (m, 5H), 6.7 (br s,1H), 4.7-4.9 (m, 1H), 3.04-3.36 (m,2H).

¹³C NMR (200 MHz, CDCl₃) δ (ppm) 171.83, 159.2, 158.6, 135.7, 129.23, 128.67, 127.1, 53.1, 37.5.

### Example 46: N'-((S)-2-tert-Butoxycarbonylamino-propionyl)-hydrazine carboxylic acid 9H-fluoren-9-ylmethyl ester

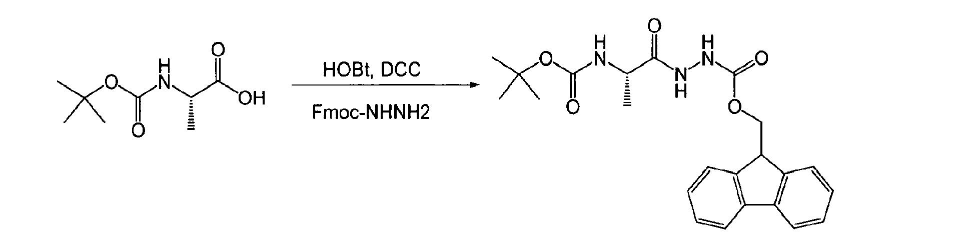

To the solution of Boc-L-alanine (2.0 g, 10.57 mmol) in DCM (30 mL ) was added HOBt (1.5 g, 11.10 mmol) followed by DCC (2.29 g, 11.10 mmol) and stirred for 30 min at room temperature. To this Fmoc- hydrazine (2.82 g, 11.10 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (30 mL), 10 % NaHCO₃ (30 mL), brine (30 mL), dried (Na₂SO₄) and concentrated to give 4.4 g (97 %) of product as a white solid; mp. 71-72°C.

R*_{f}* = (1:1 Pentane / EtOAc)

[α]_{D} -36.0 (c 0.5, MeOH)

¹H NMR ( 200 MHz, CDCl₃) δ (ppm) 8.78 (br s, 1H), 7.74 (d, *J* = 7.42 Hz, 2H), 7.57 (d, *J* = 7.44 Hz, 2H), 7.23-7.41 (m, 5H), 5.31 (br s, 1H), 4.39 (d, *J* = 7.02 Hz, 2H), 4.18-4.33 (m, 1H), 1.38-1.44 (m, 12H).

¹³C NMR (200 MHz, CDCl₃) δ (ppm) 172.88, 156.24, 155.77, 143.48, 141.22, 127.74, 127.11, 125.13, 119.93, 80.56, 67.98, 48.45, 46.85, 28.29, 18.09.

IR (KBr) ν 4000 - 3293 (br.), 2979, 2934, 1668 (br.), 1506, 1451, 1368, 1247, 1167, 1046, 759, 740 cm⁻¹.

### Example 47: N-{(S)-2-[N'-(9H-Fluoren-9-ylmethoxycarbonyl)-hydrazino]-1-methyl-2-oxo-ethyl}-oxalamic acid tert-butyl ester (Fmoc-NAlaO-O-O-tBu)

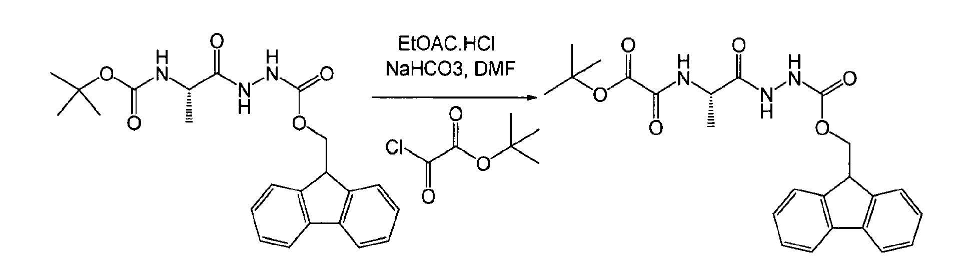

To the solution of N'-((S)-2-tert-Butoxycarbonylamino-propionyl)-hydrazine carboxylic acid 9H-fluoren-9-ylmethyl ester (Example 46) (3.8 g, 8.93 mmol) in ethyl acetate (20 mL), ethyl acetate saturated with HCl (20 mL) was added slowly with stirring at 0°C. The reaction mixture was stirred at room temperature for 30 min. Solvent was evaporated and the reaction mixture was well dried under high vacuum. The residue obtained was dissolved in dry DMF (30 mL), NaHCO₃ (5.25 g, 62.52 mmol) was added to the reaction mixture, followed by *tert*-butyl oxalyl chloride (2.21 g, 13.4 mmol) at 0°C under argon. The reaction mixture was stirred at room temperature for 30 min. Solvent was evaporated under high vacuum, the residue obtained was dissolved in water (50 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL). The ethyl acetate layer was dried (Na₂SO₄) and concentrated to give 3.8 g (93 %) of product as a white solid; mp. 79-80°C.

*R_{f}* = (1:1 Pentane/EtOAc)

[α]_{D} -31.4 (c 0.5, MeOH)

¹H NMR ( 200 MHz, CDCl₃) δ (ppm) 8.74 (br s, 1H), 7.63-7.72 (m, 3H), 7.47 (d, *J* = 7.24 Hz, 2H), 7.09-7.32 (m, 5H), 4.47-4.61 (m, 1H), 4.3 (d, *J* = 7.04 Hz, 2H), 4.1-4.15 (m, 1H), 1.37-1.43 (m, 12H).

¹³C NMR (200 MHz, CDCl₃) δ (ppm) 171.31, 158.62, 157.62, 156.17, 143.45, 141.22, 127.77, 127.12, 125.11, 119.95, 85.06, 68.08, 47.84, 46.81, 27.63, 17.65.

IR (KBr) ν 4000 - 3290 (br.), 2982, 2936, 1732, 1692 (br.), 1520, 1451, 1371, 1296, 1220, 1156, 759, 741 cm⁻¹.

### Example 48: N-{(S)-2-[N'-(9H-Fluoren-9-ylmethoxycarbonyl)-hydrazino]-1-methyl-2-oxo-ethyl}-oxalamic acid (Fmoc-NAlaOOH)

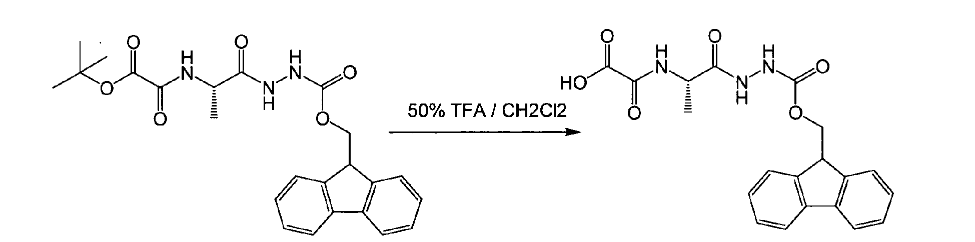

To the solution of N-{(S)-2-[N'-(9H-Fluoren-9-ylmethoxycarbonyl)-hydrazino]-1-methyl-2-oxo-ethyl}-oxalamic acid tert-butyl ester (Example 47) (1.0 g, 2.21 mmol) in dichloromethane (15 mL), TFA (15 mL) was added slowly with stirring at 0°C. The reaction mixture was stirred at room temperature for 30 min. Solvent was evaporated and was well dried under high vacuum to give 870 mg of crude compound which after crystallisation from ethylacetate - pet ether gives 752 mg (85 %) of desired compound as a white solid.

¹H NMR ( 200 MHz, DMSO-d₆) δ (ppm) 9.92 (s, 1H), 9.33 (s, 1H), 8.73 (d, *J* = 7.82 Hz, 1H), 7.87 (d, *J* = 7.02 Hz, 2H), 7.71 (d, *J* = 7.02 Hz, 2H), 7.28-7.44 (m, 4H), 4.27-4.4 (m, 4H), 1.33 (d, *J* = 6.84 Hz, 3H).

¹³C NMR (200 MHz, DMSO-d₆) δ (ppm) 171.08, 161.67, 157.86, 155.88, 143.56, 140.66, 127.65, 127.06, 125.21 120.07, 66.12, 47.22, 46.43, 17.72.

### Example 49: N-((S)-1-Hydrazinocarbonyl-ethyl)-oxalamic acid tert-butyl ester (NalaO-O-t-Bu)

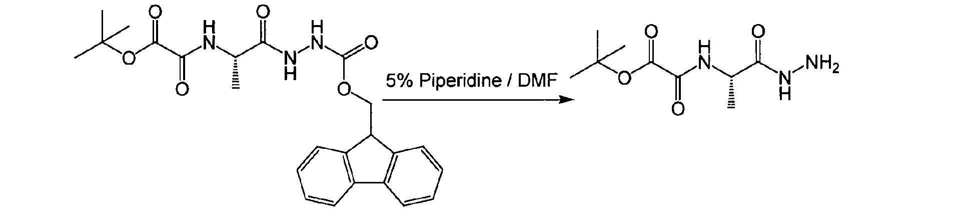

The solution of N-{(S)-2-[N'-(9H-Fluoren-9-ylmethoxycarbonyl)-hydrazino]-1-methyl-2-oxo-ethyl}-oxalamic acid tert-butyl ester (Example 47) (2.1 g, 4.63 mmol) in 5% piperidine in DMF (15 mL) was stirred at rt for 20 min. Solvent was evaporated and the reaction mixture was well dried under high vacuum. The crude compound obtained was purified by column chromatography to give 137 mg (12 %) of product thick sticky mass.

Note: The other side product obtained was the hydrazone of desired compound with DMF due to high reactivity of hydrazide. Yield of the desired compound was very poor, several other spots were seen on TLC but were not separable.

¹H NMR (200 MHz, CDCl₃) δ (ppm) 7.81 (d, *J =* 7.82 Hz, 1H), 4.37-4.51 (m, 1H), 4.06 (br s, 2H), 1.48 (s, 9H), 1.36 (d, *J* = 7.04 Hz, 3H).

### Example 50: N'-((S)-2-tert-Butoxycarbonylamino-propionyl)-hydrazine carboxylic acid benzyl ester

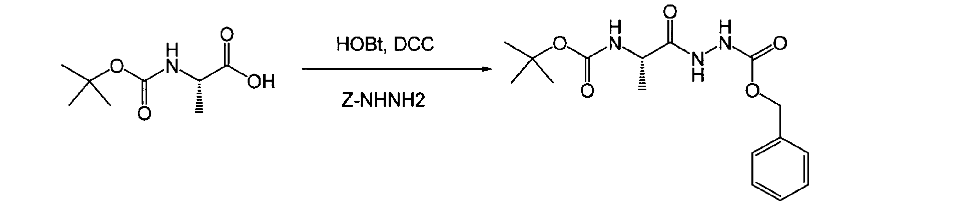

To the solution of Boc-L-alanine (4.0 g, 21.14 mmol) in DCM (50 mL) was added HOBt (3.0 g, 22.2 mmol) followed by DCC (4.58 g, 22.2 mmol) and stirred for 30 min at room temperature. To this Z- hydrazine (3.69 g, 22.2 mmol) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (30 mL),10 % NaHCO₃ (30 mL), brine (30 mL), dried (Na₂SO₄) and concentrated to give 6.9 g (96 %) of product as a white solid; mp. 130-131°C.

R*_{f}* = ( petrol ether/EtOAc)

[α]_{D} -43.6 (*c* 0.5, MeOH)

¹H NMR (200 MHz, CDCl₃ -2 drops DMSO-d₆) δ (ppm) 9.37 (bs, 1H), 8.29 (bs, 1H), 7.3 (s, 5H), 5.72 (bs, 1H), 5.1 (s, 2H), 4.23 (bs, 1H), 1.4 (s, 9H), 1.31 (d, J = 6.64 Hz, 3H).

¹³C NMR (200 MHz, CDCl₃ -2 drops DMSO-d₆) δ (ppm) 177.7, 161.11, 160.02, 140.87, 133.15, 132.84, 132.75, 84.19, 71.77, 53.33, 33.08, 23.55.

### Example 51: N-[(S)-2-(N'-Benzyloxycarbonyl-hydrazino)-1-methyl-2-oxo-ethyl]-oxalamic acid tert-butyl ester (Z-NAlaO-O-t-Bu)

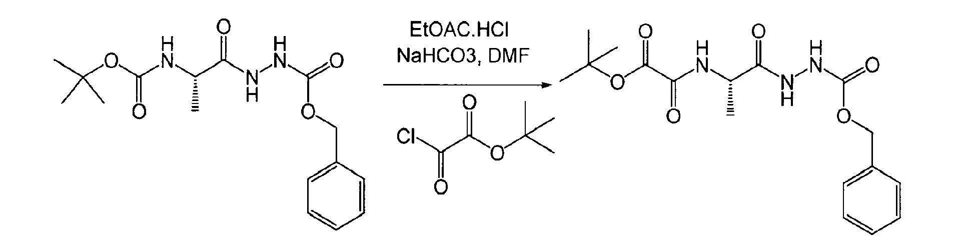

To the solution of N'-((S)-2-tert-Butoxycarbonylamino-propionyl)-hydrazine carboxylic acid benzyl ester (Example 50) (2.99 g, 8.87 mmol) in ethyl acetate (20 mL), ethyl acetate saturated with HCl (20 mL) was added slowly with stirring at 0°C. The reaction mixture was stirred at room temperature for 30 min. Solvent was evaporated and the reaction mixture was well dried under high vacuum. The residue obtained was dissolved in dry DMF (30 mL), NaHCO₃ (5.21 g, 62.08 mmol) was added to the reaction mixture, followed by *tert*-butyl oxalyl chloride (2.19 g, 13.3 mmol) at 0°C under argon. The reaction mixture was stirred at room temperature for 30 min. Solvent was evaporated under high vacuum, the residue obtained was dissolved in water (50 mL) and extracted with ethyl acetate (3 x 30 mL), the combined organic layer was washed with 1N HCl (30 mL) followed by 10 % NaHCO₃ (30 mL) and water (3 x 30 mL) followed by brine (20 mL). The ethyl acetate layer was dried (Na₂SO₄) and concentrated to give 2.8 g (86 %) of product as a white solid; mp. 49-51°C.

R*_{f}* = (1:1 Pentane/EtOAc)

[α]_{D} -44.0 (c 0.5, MeOH)

¹H NMR ( 200 MHz, CDCl₃) δ (ppm) 8.87 (br s, 1H), 7.84 (d, *J* = 7.64 Hz, 1H), 7.3 (s, 5H), 7.21 (s, 1H), 5.1 (s, 2H), 4.51-4.65 (m, 1H), 1.5 (s, 9H), 1.42 (d, *J* = 6.66 Hz, 3H).

¹³C NMR (200 MHz, CDCl₃) δ (ppm) 171.43, 158.65, 157.53, 156.2, 135.52, 128.5, 128.31, 128.12, 84.93, 67.78, 47.83, 27.61, 17.71.

IR (KBr) ν 4000 - 3296 (br.), 2984, 2938, 1733, 1699 (br.), 1517, 1456, 1371, 1297, 1219, 1156, 840, 740, 697 cm⁻¹.

### Example 52: N-[(S)-2-(N'-Benzyloxycarbonyl-hydrazino)-1-methyl-2-oxo-ethyl]-oxalamic acid methyl ester (Z-NAlaO-OMe)

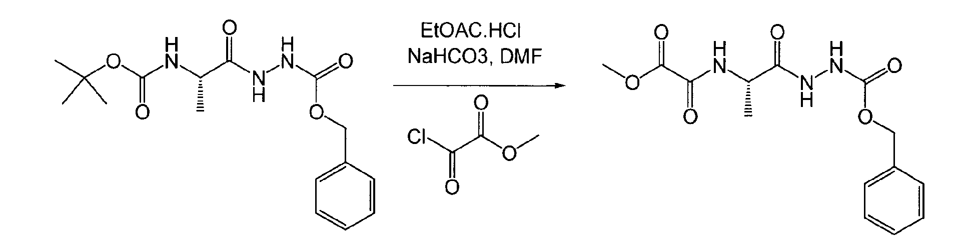

To the solution of N'-((S)-2-tert-Butoxycarbonylamino-propionyl)-hydrazine carboxylic acid benzyl ester (Example 50) (2.98 g, 8.83 mmol) in ethyl acetate (20 mL), ethyl acetate saturated with HCl (20 mL) was added slowly with stirring at 0°C. The reaction mixture was stirred at room temperature for 30 min. Solvent was evaporated and the reaction mixture was well dried under high vacuum. The residue obtained was dissolved in dry DMF (30 mL), NaHCO₃ (5.19 g, 61.79 mmol) was added to the reaction mixture, followed by *mono*-methyl oxalyl chloride (1.62 mL, 17.65 mmol) at 0°C under argon. The reaction mixture was stirred at room temperature for 30 min. Solvent was evaporated under high vacuum, the residue obtained was dissolved in water (50 mL) and extracted with ethyl acetate (3 x 30 mL), the combined organic layer was washed with 1N HCl (30 mL) followed by 10% NaHCO₃ (30 mL) and water (3 x 30 mL) followed by brine (20 mL).The ethyl acetate layer was dried (Na₂SO₄) and concentrated to give 1.7 g of crude compound which was purified by column chromatography to give 640 mg (22 %) of product as a white solid; mp. 51-52°C.

Note: Yield was low due to poor quality of methyl oxalyl chloride.

R*_{f}* = 0.37 (1:1 Pentane/EtOAc)

[α]_{D} -47.6 (c 0.5, MeOH)

¹H NMR (200 MHz, CDCl₃) δ (ppm) 8.93 (s, 1H), 8.0 (d, *J* = 7.24 Hz, 1H), 7.35 (s, 1H), 7.3 (s, 5H), 5.09 (s, 2H), 4.56-4.64 (m, 1H), 3.79 (s, 3H), 1.41 (d, *J*= 6.64 Hz, 3H).

¹³C NMR (200 MHz, CDCl₃) δ (ppm) 171.37, 160.24, 156.39, 156.28, 135.5, 128.5, 128.34, 128.11, 67.81, 53.68, 47.84, 17.65.

IR (KBr) ν 4000 - 3293 (br.), 3035, 2956, 1702, 1689 (br.), 1524, 1456, 1283, 1218, 985, 742, 667 cm⁻¹.

### Example 53: N-[(S)-2-(N'-Benzyloxycarbonyl-hydrazino)-1-methyl-2-oxo-ethyl]-oxalamic acid (Z-NAlaO-OH)

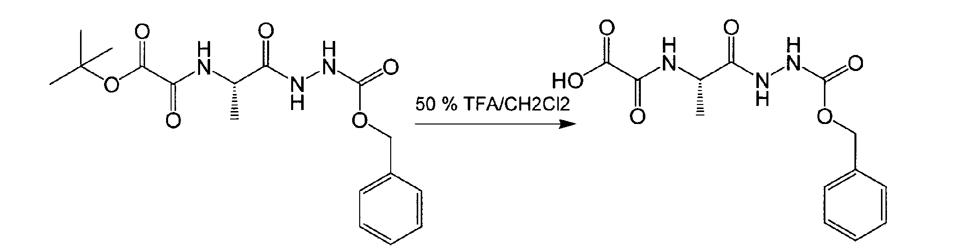

To the solution of N-[(S)-2-(N'-Benzyloxycarbonyl-hydrazino)-1-methyl-2-oxo-ethyl]-oxalamic acid tert-butyl ester (Example 51) (2.6 g, 7.12 mmol) in dichloromethane (15 mL), TFA (15 mL) was added slowly with stirring at 0°C. The reaction mixture was stirred at room temperature for 30 min. Solvent was evaporated and was well dried under high vacuum to give 2.2 g (99 %) of desired compound as a white solid; mp.173-174 °C.

¹H NMR ( 200 MHz, DMSO-d₆) δ (ppm) 9.7 (s, 1H), 9.04 (s, 1H), 8.52 (d, *J* = 7.62 Hz, 1H), 7.14 (s, 5H), 4.86 (s, 2H), 4.08-4.15 (m, 1H), 1.1 (d, *J* = 6.46 Hz, 3H).

¹³C NMR (200 MHz, DMSO-d₆) δ (ppm) 172.27, 162.81, 159.04, 157.05, 137.66, 129.5, 129.11, 128.93, 67.03, 48.36, 18.82.

### Example 54: [N'-(9H-Fluoren-9-ylmethoxycarbonyl)-hydrazino]-oxo-acetic acid tert-butyl ester

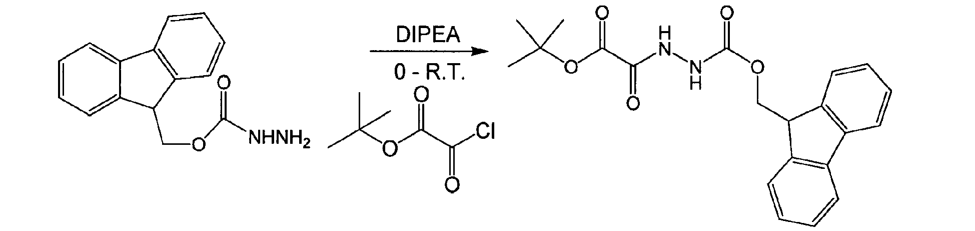

To a mixture of Fmoc-hydrazine (0.100 mg, 0.39 mmol), DIPEA (0.08 mL, 0.43 mmol) and methylenechloride (10 mL) was added t-Bu-oxalylchloride (0.06 mL, 0.39) slowly with stirring and ice-bath cooling under argon. The reaction mixture was stirred further at room temperature for 15 mins. The reaction mixture was then washed with 1N HCL (5 mL), followed by 10% NaHCO₃ (5 mL) and brine (5 mL), dried (Na₂SO₄) and concentrated to give 140 mg of crude compound which was purified by column chromatography to give 94 mg (62%) of pure product as white solid; mp. 79-80°C.

R_{f} = 0.45 (2:1 Pentane/EtOAc)

¹H NMR (200 MHz, CDCl₃) δ (ppm) 8.94 (br s, 1H), 7.75 (d, *J* = 7.42 Hz, 2H), 7.58 (d, *J* = 7.24 Hz, 2H), 7.26-7.43 (m, 5H), 4.44 (d, *J* = 7.22 Hz, 2H), 4.23 (t, *J* = 7.14 Hz, 1H), 1.57 (m, 12H).

¹³C NMR (200 MHz, CDCl₃) δ(ppm) 157.78, 156.38, 155.51, 143.33, 141.25, 127.84, 127.16, 125.1, 120.01, 85.69, 68.3, 46.79, 27.70.

### Example 55: [N'-(9H-Fluoren-9-ylmethoxycarbonyl)-hydrazino]-oxo-acetic acid

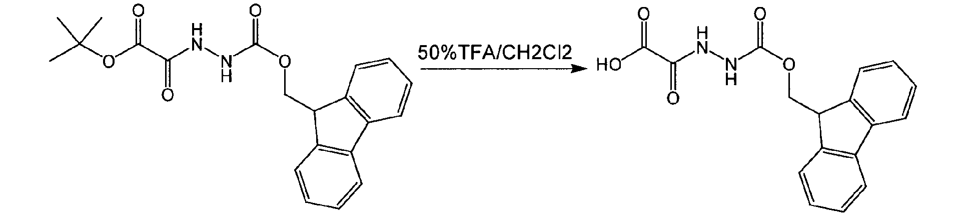

To the solution of N'-(9H-Fluoren-9-ylmethoxycarbonyl)-hydrazino]-oxo-acetic acid tert-butyl ester (Example 54) (90 mg, 0.24 mmol) in dichloromethane (5 mL), TFA (5 mL) was added slowly with stirring at 0°C, the reaction mixture was stirred at room temperature under argon for 2 h. Solvent was evaporated and was well dried under high vacuum to give 76 mg (100%) of product as a white solid; mp. 178-179°C.

¹H NMR (200 MHz, DMSO-d₆) δ (ppm) 10.64 (s, 1H), 9.5 (s, 1H), 7.88 (d, *J* = 7.24 Hz, 2H), 7.72 (d, *J* = 6.64 Hz, 2H), 7.32-7.45 (m, 4H), 4.22-4.35 (m, 3H).

¹³C NMR (200 MHz, DMSO-d₆) δ (ppm) 161.19, 158.19, 155.43, 143.51, 140.67, 127.67, 127.06, 125.18, 120.09, 66.19, 46.38.

### Example 56: N'-((S)-2-tert-Butoxycarbonylamino-3-phenyl-propionyl)-N-methyl-hydrazine carboxylic acid 9H-fluoren-9-ylmethyl ester

### Step 1: 1-Fmoc-1-methylhydrazine:

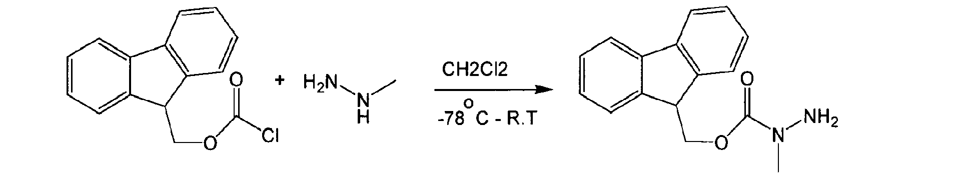

A solution of 9*H*-fluoren-9-ylmethyl chloroformate (5.05 g, 19.54 mmol) in CH₂Cl₂ (15 mL) was slowly added at -78°C to a stirring solution of methyl hydrazine (1.0 g, 21.71 mmol) and DIPEA (5.35 mL, 30.39 mmol) in CH₂Cl₂ (25 mL). The reaction mixture was allowed to warm to room temperature and stirred overnight. The reaction mixture was extracted with water, dried over Na₂SO₄, and concentrated under reduced pressure to give yellow oil which after flash chromatography gives 4.56 g (87%) of pure compound as a white solid: mp. 81.4°C (Lit.:82°C (Gibson et al., Journal of Organic Chemistry (1999), 64(20), 7388-7394)).

R*_{f}* = 0.12 (2:1 Pentane/EtOAc)

¹H NMR ( 200 MHz, CDCl₃) δ (ppm) 7.78 (d, *J* = 7.24 Hz, 2H), 7.6 (d, *J* = 7.22 Hz, 2H), 7.27-7.46 (m, 4H), 4.45 (d, *J* = 6.84 Hz, 2H), 4.26 (t, *J* = 6.84 Hz, 1H), 4.09 (bs, 2H), 3.14 (s, 3H).

¹³C NMR (200 MHz, CDCl₃) δ (ppm) 157.53, 143.85, 141.33, 127.75, 127.07, 124.95, 120.03, 67.86, 47.24, 38.4.

### Step 2:

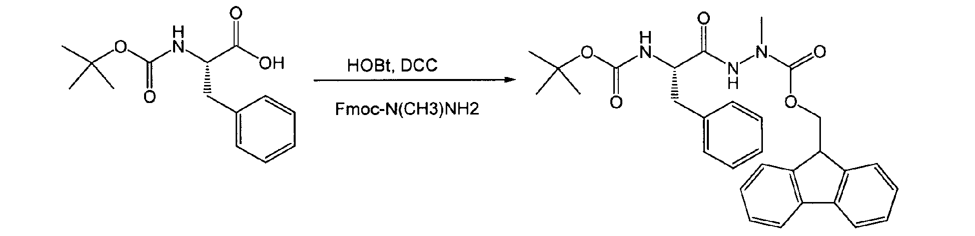

To the solution of Boc-L-phenylalanine (500 mg, 1.89 mmol) in DCM (15 mL) was added HOBt (268 mg, 1.98 mmol) followed by DCC (408 mg, 1.98 mmol) and stirred for 30 min at room temperature. To this 1-Fmoc-1-methylhydrazine (506 mg, 1.89 mmol) was added and the mixture was stirred overnight at room temperature under argon.

The precipitated DCHU was removed by filtration and the filtrate was evaporated. The additional DCHU was removed by subsequent trituration with cold ethyl acetate and filtration. The ethyl acetate solution was washed with 1N HCl (10 mL),10 % NaHCO₃ (10 mL), brine (10 mL), dried (Na₂SO₄) and concentrated to give 1.0 g of crude compound which was purified by column chromatography to give 794 mg (81%) of product as a white solid; mp. 81-82°C.

R*_{f}* = 0.68 (1:1 EtOAc/Pentane)

[α]_{D} -12.0 (c 0.5, MeOH)

¹H NMR ( 200 MHz, CDCl₃) δ (ppm) 8.48 (bs, 1H), 7.75 (d, *J* = 7.22 Hz, 2H), 7.56 (d, *J* = 7.24 Hz, 2H), 7.23-7.43 (m, 9H), 5.24 (bs, 1H), 4.19-4.41 (m, 4H), 3.07 (bs, 5H), 1.37 (s, 9H).

¹³C NMR (200 MHz, CDCl₃) δ (ppm) 170.06, 155.9, 155.56, 143.66, 141.23, 136.35, 129.42, 128.58, 127.73, 127.14, 126.96, 125.06, 119.95, 80.5, 68.31, 54.03, 46.96, 38.32, 37.32, 28.21.

IR (KBr) ν 4000 - 3289 (br.), 3064, 3029, 2977, 1681 (br.), 1478, 1496, 1451, 1392, 1366, 1348, 1249, 1165, 758, 740 cm⁻¹.

### Example 57: N'-((S)-2-tert-Butoxycarbonylamino-3-phenyl-propionyl)-N'-methyl-hydrazine carboxylic acid 9H-fluoren-9-ylmethyl ester

### Step 1: 1-Fmoc-2-methylhydrazine:

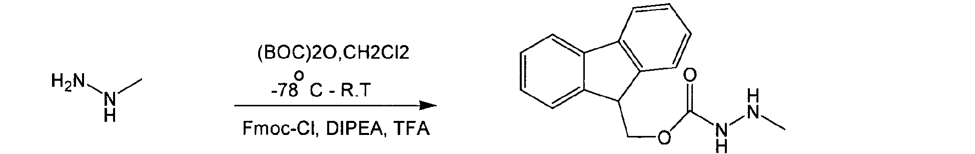

mp. 154.9°C (Lit.:155°C (Gibson et al., 1999, above))

R*_{f}* = 0.19 (1:1 Pentane/EtOAc)

¹H NMR (200 MHz, DMSO-d₆) δ (ppm) 8.66 (s, 1H), 7.87 (d, *J* = 7.24 Hz, 2H), 7.68 (d, *J* = 7.24 Hz, 2H), 7.28-7.44 (m, 4H), 4.21-4.32 (m, 4H), 2.42 (s, 3H).

¹³C NMR (200 MHz, DMSO-d₆) δ (ppm) 156.69, 143.75, 140.67, 127.58, 127.01, 125.15, 120.04, 65.4, 46.63.

### Step 2:

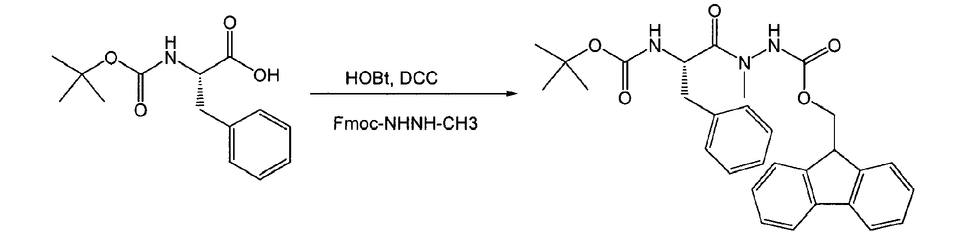

To the solution of Boc-L-phenylalanine (500 mg, 1.89 mmol) in DCM (15 mL ) was added HOBt (268 mg, 1.98 mmol) followed by DCC (408 mg, 1.98 mmol) and stirred for 30 min at room temperature. To this 1-Fmoc-2-methylhydrazine (506 mg, 1.89 mmol) was added and the mixture was stirred overnight at room temperature under argon.

The precipitated DCHU was removed by filtration and the filtrate was evaporated. The additional DCHU was removed by subsequent trituration with cold ethyl acetate and filtration. The ethyl acetate solution was washed with 1N HCl (10 mL),10 % NaHCO₃ (10 mL), brine (10 mL), dried (Na₂SO₄) and concentrated to give 900 mg of crude compound which was purified by column chromatography to give 632 mg (65%) of product as a white solid; mp. 108-109°C.

[α]_{D} + 23.6 (c 0.5, MeOH)

¹H NMR ( 200 MHz, CDCl₃) δ (ppm) 7.79 (d, *J* = 7.24 Hz, 2H), 7.17-7.74 (m, 12H), 5.3 (bs, 1H), 4.91 (bs, 1H), 4.49 (bs, 2H), 4.21 (m, 1H), 2.66-3.31 (m, 5H), 1.39 (s, 9H).

¹³C NMR (200 MHz, CDCl₃) δ (ppm) 173.92, 155.35, 154.71, 143.32, 141.36, 136.53, 129.35, 128.47, 127.9, 127.17, 126.9, 124.91, 120.07, 79.88, 67.77, 51.23, 46.94, 35.64, 28.27.

IR (KBr) ν 4000 - 3383, 3224, 3064, 3004, 2977, 2929, 1742, 1697, 1647, 1516, 1451, 1391, 1366, 1248, 1170, 1115, 1083, 1048, 756, 740 cm⁻¹.

### Example 58: N-{(S)-1-Benzyl-2-[N'-(9H-fluoren-9-ylmethoxycarbonyl)-N'-methyl-hydrazino]-2-oxo-ethyl}-oxalamic acid tert-butyl ester

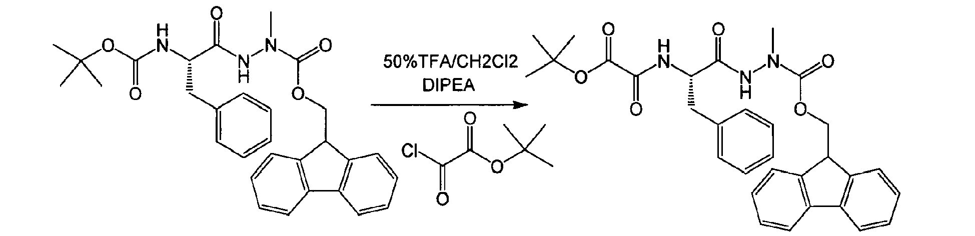

To the solution of N'-((S)-2-tert-Butoxycarbonylamino-3-phenyl-propionyl)-N-methyl-hydrazine carboxylic acid 9H-fluoren-9-ylmethyl ester (Example 56) (687 mg, 1.33 mmol) in dichloromethane (10 mL), TFA (10 mL) was added slowly with stirring at 0°C, the reaction mixture was stirred overnight at room temperature. Solvent was evaporated and was well dried under high vacuum. The residue obtained was dissolved in dry DCM (30 mL), and DIPEA (0.7 mL, 4.0 mmol) was added to the reaction mixture, followed by *tert*-butyl oxalyl chloride (219 mg, 1.33 mmol) at 0°C under argon. The reaction mixture was further stirred at room temperature for 30 min. The mixture was washed with 1N HCl (15 mL) followed by 10% NaHCO₃ (15 mL) and brine (15 mL). The organic phase was dried (Na₂SO₄) and concentrated to give 770 mg of crude product which was purified by column chromatography to give 635 mg (87 %) of product as a white powder.

R*_{f}* = 0.2 (1:2 EtOAc/Pentane)

¹H NMR ( 200 MHz, CDCl₃) δ (ppm) 8.64 (s, 1H), 7.62 (d, *J* = 7.04 Hz, 3H), 7.43 (d, *J* = 7.04 Hz, 2H), 7.13-7.3 (m, 9H), 4.78 (q, *J* = 7.24 Hz, 1H), 3.97-4.27 (m, 3 H), 3.03 (bs, 2H), 2.93 (s, 3H), 1.33 (s, 9H).

¹³C NMR (200 MHz, CDCl₃) δ (ppm) 169.0, 158.29, 157.49, 155.82, 143.61, 141.2, 135.7, 129.42, 128.69, 127.75, 127.23, 127.12, 125.05, 119.95, 85.01, 68.44, 53.08, 46.87, 38.11, 37.51, 27.56.

### Example 59: N-{(S)-1-Benzyl-2-[N'-(9H-fluoren-9-ylmethoxycarbonyl)-N-methyl-hydrazino]-2-oxo-ethyl}-oxalamic acid tert-butyl ester

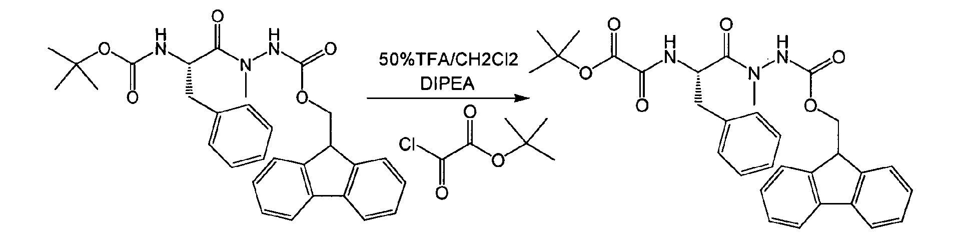

To the solution of N'-((S)-2-tert-Butoxycarbonylamino-3-phenyl-propionyl)-N'-methyl-hydrazine carboxylic acid 9H-fluoren-9-ylmethyl ester (Example 57) (527 mg, 1.02 mmol) in dichloromethane (10 mL), TFA (10 mL) was added slowly with stirring at 0°C, the reaction mixture was stirred overnight at room temperature. Solvent was evaporated and was well dried under high vacuum. The residue obtained was dissolved in dry DCM (30 mL), and DIPEA (0.54 mL, 3.07 mmol) was added to the reaction mixture, followed by *tert*-butyl oxalyl chloride (168 mg, 1.02 mmol) at 0°C under argon. The reaction mixture was further stirred at room temperature for 30 min. The mixture was washed with 1N HCl (15 mL) followed by 10% NaHCO₃ (15 mL) and brine (15 mL). The organic phase was dried (Na₂SO₄) and concentrated to give 575 mg of crude product which was purified by column chromatography to give 463 mg (83 %) of product as a white powder.

*R_{f}* = 0.26 (1:2 EtOAc/Pentane)

¹H NMR ( 200 MHz, CDCl₃) δ (ppm) 7.68 (d, *J* = 7.22 Hz, 2H), 7.07-7.6 (m, 13H), 4.98 (bs, 1H), 4.39 (bs, 2H), 4.11 (bs, 1H), 2.97 (bs, 5H), 1.43 (s, 9H).

¹³C NMR (200 MHz, CDCl₃) δ (ppm) 172.37, 158.6, 157.07, 154.76, 143.29, 141.35, 135.75, 129.32, 128.61, 127.91, 127.23, 127.16, 124.91, 120.07, 84,78, 67.75, 50.73, 46.93, 35.69, 27.68.

### Example 60: N'-{(S)-2-[((S)-1-Dimethylcarbamoyl-ethyl-aminooxalyl)-amino]-3-phenyl-propionyl}-hydrazine carboxylic acid tert-butyl ester

### Step 1:

### ((S)-1-Dimethylcarbamoyl-ethyl)-carbamic acid benzyl ester:

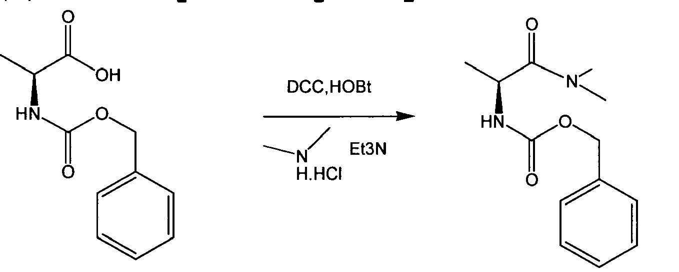

To the solution of Z- L-Alanine (2.0 g, 8.96 mmol) in DCM (10 mL), HOBt (1.211 g, 8.96 mmol) was added, followed by DCC (1.85 g, 8.96 mmol). To this solution a suspension of dimethylamine hydrochloride (731 mg, 8.96 mmol) and triethylamine (1.25 mL, 8.96 mmol) in DCM (10 mL) was added and the mixture was stirred at room temperature for 24 h.

The precipitated DCHU was removed by filtration and the filtrate was evaporated. The additional DCHU was removed by subsequent trituration with cold ethyl acetate and filtration. The ethyl acetate solution was washed with 1N HCl (2 x 15 mL), 10 % NaHCO₃ (15 mL), brine (15 mL) dried (Na₂SO₄) and concentrated to give 2.2 g (98%) of product as a viscous oil.

¹H NMR ( 200 MHz, CDCl₃) δ (ppm) 7.27(s, 5H), 5.78 (d, *J* = 7.44 Hz, 1H), 5.02 (s, 2H), 4.53-4.67 (m, 1H), 2.99 (s, 3H ), 2.89(s, 3H ),1.26 ( d, *J* = 5.86 Hz, 3H ).

### Reference for step 1:

2003 OBC 965: Hill, Roger R.; Birch, David; Jeffs, Graham E.; North, Michael. Enantioselection in peptide bond formation. Organic & Biomolecular Chemistry (2003), 1(6), 965-972.

### Step 2:

### (S)-2-Amino-N,N-dimethyl-propionamide:

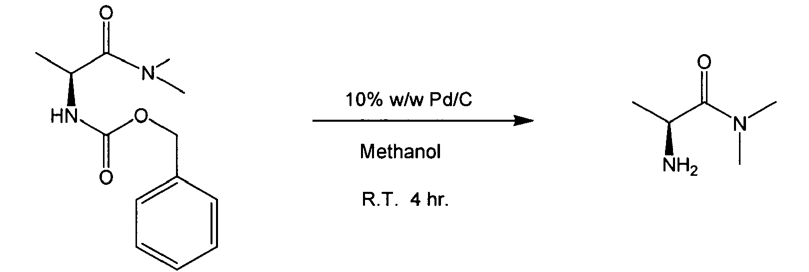

In a Parr apparatus a solution of (S)-1-Dimethylcarbamoyl-ethyl)-carbamic acid benzyl ester (2.2 g, 8.79 mmol) in methanol (50 mL) was hydrogenated using 10% Pd/C (220 mg, 10% w/w) at 60 p.s.i. for 4 h at room temperature. The catalyst was filtered using sintered glass funnel, washed with methanol (15 mL) and the filtrate was evaporated to give 1.0 g (97%) of product as a colourless viscous oil.

¹H NMR (200 MHz, CDCl₃) δ (ppm) 4.6 (br s, 2H), 3.98 (m, 1H), 2.99 (s, 3H), 2.89 (s, 3H), 1.27 (d, *J* = 4.48 Hz, 3H).

### Reference for step 2:

2003 BMC 2715 :Structure-activity relationships of the peptide deformylase inhibitor

BB-3497: modification of the P2' and P3' side chains. Davies, Stephen J.; Ayscough, AndrewP.; Beckett, R. Paul; Clements, John M.; Doel, Sheila; Pratt, Lisa M.; Spavold, Zoe M.; Thomas, S. Wayne; Whittaker, Mark. Bioorganic & Medicinal Chemistry Letters (2003), 13(16), 2715-2718.

### Step 3:

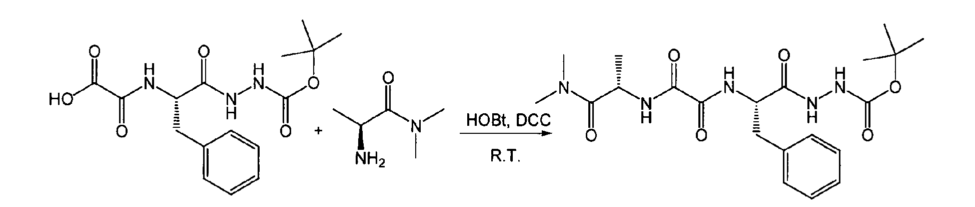

To the solution of *N*-[(S)-1-Benzyl-2-(*N*'-*tert-*butoxycarbonyl-hydrazino)-2-oxo-ethyl]-oxalamic acid (Example 4) (271 mg,0.77 mmol) in DCM (5 mL), HOBt (109 mg,0.81 mmol) was added followed by DCC (167 mg, 0.81 mmol). To this a solution of (S)-2-Amino-N,N-dimethyl-propionamide (Step-2) (94 mg,0.81 mmol) in DCM (5 mL) was added and the mixture was stirred at room temperature under argon for 24 h.

The precipitated DCHU was removed by filtration and the filtrate was evaporated. The additional DCHU was removed by subsequent trituration with cold ethyl acetate and filtration. The ethyl acetate solution was washed with 1N HCl (2 x 5 mL), 10 % NaHCO₃ (5 mL), brine (5 mL) dried (Na₂SO₄) and concentrated to give 250 mg of crude compound which was purified by silica gel column chromatography to give 122 mg (35%) of product as off-white solid; mp 104-107°C.

[α]_{D} - (*c* 0.5, MeOH)

¹H NMR ( 200 MHz, CDCl₃) δ (ppm) 8.6 (br s, 1H), 8.25-8.29 (br, 2H), 7.14-7.2 (m, 5H), 6.59 (br s, 1H), 4.68-4.8 (m, 2H), 3.29 (dd, J = 5.28 Hz, 1H), 2.93-3.07 (m, 4H), 2.88 (s, 3H), 1.35 (s, 9H), 1.26 (d, J = 6.26Hz, 3H).

¹³C NMR (200 MHz, CDCl₃) δ (ppm) 171.17, 169.77, 159.60, 158.42, 155.18, 136.39, 129.25, 128.60, 126.93, 81.75, 53.11, 45.66, 37.29, 36.98, 35.84, 28.08, 17.88.

IR (KBr) ν 4000 - 3292 (br.), 3030, 2980, 2935, 1674 (br.), 1641,1498 cm⁻¹.

### Example 61: {(S)-1-Benzyl-2-[N'-((S)-2-tert-butoxycarbonylamino-propionyl)-hydrazino]-2-oxo-ethyl}-carbamic acid benzyl ester

### Step 1: N-benzyloxycarbonyl-L-phenylalanine hydrazide:

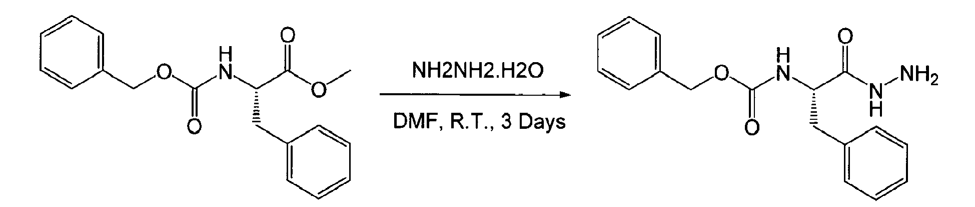

To the solution of Z-L-Phenylalanine methyl ester (6.0 g, 19.15 mmol) in DMF (15 mL) hydrazine hydrate (2.8 mL, 57.44 mmol) was added drop wise with stirring and the reaction mixture was stirred at room temperature for 3 days.

DMF was evaporated on high vacuum and the residue was washed with water and filtered, the resulting crude compound was recrystallized from ethyl acetate- pet ether to give 4.0 g (66%) of product as a white solid, mp. 163 - 164°C (Lit.: 164-166°C).

[α]_{D}-10.4 (c 0.5, DMF) (Lit.: [α]_{D} -9.8 (c 0.8, DMF)).

¹H NMR (200 MHz, DMSO-d₆) δ (ppm) 9.23(s, 1H), 7.5 (d, J = 10.74 Hz, 1H), 7.18-7.33 (m, 10H), 4.92 (s, 2H), 4.14- 4.23 (m, 3H), 2.7-2.96 (m, 2H).

¹³C NMR (200 MHz, DMSO-d₆) δ (ppm) 170.67, 155.66, 137.99, 136.98, 129.12, 128.21, 127.99, 127.6, 127.4, 126.2, 65.12, 54.89, 37.71.

### Reference for step 1:

Eur. Pat. Appl., 5248, 14 Nov 1979 **Tetrapeptidehydrazide derivatives.** Masahiko, Fujino; Susumu, Shinagawa; Kiyohisa, Kawai. Eur. Pat. Appl. (1979), 93 pp. CODEN: EPXXDW EP 5248

This substance was reported in 80% yield.

### Step 2:

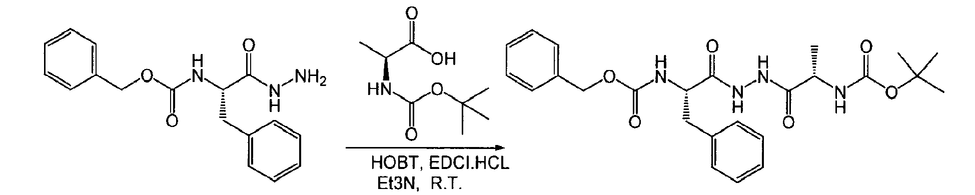

Boc- L-Alanine (1.81 g, 9.57 mmol) was dissolved in DCM (30 mL) and HOBt (1.36 g, 10.05 mmol) was added followed by (1.93 g, 10.05 mmol) EDCI.HCl. To this solution N-benzyloxycarbonyl-L-phenylalanine hydrazide (3.0 g, 9.57 mmol) was added followed by triethylamine (1.47 mL, 10.53 mmol). The reaction mixture was further stirred at room temperature for overnight.

DCM removed by rotary evaporation and the residue obtained was dissolved in ethyl acetate (50 mL). The ethyl acetate solution was washed with 1N HCl (15 mL), 10 % NaHCO₃ (15 mL), brine (15 mL). The ethyl acetate was heated to dissolve the precipitated compound and pet ether (10 mL) was added. The solution was allowed to stand for overnight. The crystallised compound was filtered to give 3.7 g (79 %) of product as a white crystalline solid; mp.197°C-198°C.

¹H NMR ( 200 MHz, DMSO-d₆) δ (ppm) 10.18 (br s,1H), 9.93 (br s, 1H ), 7.54 (d,*J* = 8.4Hz, 1H), 7.17-7.35 (m, 10H), 6.96 (d, *J* = 7.62 Hz, 1H), 4.92 (s, 2H), 4.25-4.36 (m, 1H), 4.0-4.08 (m, 1H), 2.69-3.09 (m, 2H), 1.38 (s, 9H), 1.23 (d, *J* = 7.24 Hz, 3H) .

### Example 62: {(S)-1-[N'-((S)-2-Amino-3-phenyl-propionyl)-hydrazinocarbonyl]-ethyl}-carbamic acid tert-butyl ester

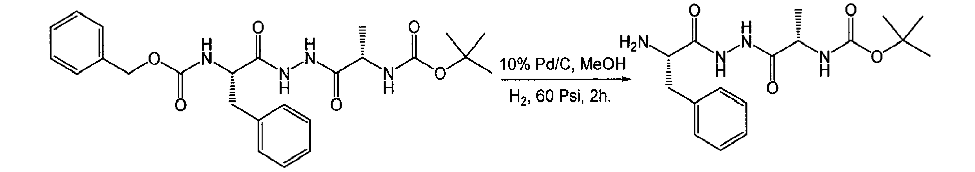

In a Parr apparatus a solution of {(S)-1-Benzyl-2-[*N*'-((S)-2-*tert*-butoxycarbonylamino-propionyl)-hydrazino]-2-oxo-ethyl}-carbamic acid benzyl ester (Example 61) (835 mg, 1.72 mmol) in methanol (15 mL) was hydrogenated using 10% Pd/C (125 mg, 15% w/w) at 60 p.s.i. for 2 h at room temperature. The catalyst was filtered using sintered glass funnel and the filtrate was evaporated to give 568 mg (94%) of the product as an off-white solid; mp. 76 - 77°C.

¹H NMR (200 MHz, DMSO-d₆) δ (ppm) 7.26 (br s, 5H), 6.94 (d, *J* = 9.78 Hz, 1H), 4.02-4.09 (m, 1H), 3.46-3.53 (m, 1H), 2.93-3.02 (m, 1H), 2.57-2.67 (m, 1H), 1.38 (s, 9H), 1.21 (d, *J* = 5.68 Hz, 3H).

### Example 63: N-{(S)-1-Benzyl-2-[N'-((S)-2-tert-butoxycarbonylamino-propionyl)-hydrazino]-2-oxo-ethyl}-oxalamic acid methyl ester (Boc-Ala-NPheO-OMe)

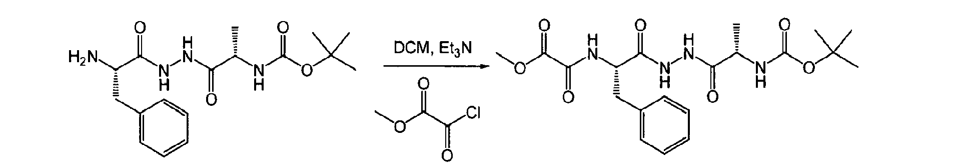

Triethylamine (1.56 mL, 11.13 mmol) was added to a solution of {(S)-1-[N'-((S)-2-Amino-3-phenyl-propionyl)-hydrazinocarbonyl]-ethyl}-carbamic acid tert-butyl ester (Example 62) (2.6 g, 7.42 mmol) in dry DCM (10 mL) at 0° under argon followed by methyl oxalyl chloride (0.72 mL, 7.79 mmol) and the reaction mixture was stirred for 30 min at 0° and at room temperature for 1.5 h. The mixture was diluted with ethylacetate (50 mL) and washed with 1N HCl (10 mL) followed by 10% NaHCO₃ (10 mL) and brine (10 mL). The ethyl acetate layer was dried (Na₂SO₄) and concentrated to give 3.2 g of crude compound, which was crystallised from ethyl acetate-pet ether to give 2.92 g (90%) of the title compound as a white crystalline solid; mp.134-135°C.

¹H NMR ( 200 MHz, DMSO-d₆) δ (ppm) 10.21 (s, 1H), 9.9 (s, 1H), 8.96 (d, *J* = 8.6 Hz, 1H), 7.16-7.29 (m, 5H), 6.97 (d, *J* = 7.64 Hz,1H), 4.54-4.66 (m, 1H), 4.0-4.08 (m, 1H), 3.74 (s, 3H), 2.94-3.18 (m, 2H), 1.38 (s, 9H), 1.22 (d, *J* = 7.64Hz, 3H). NPheO, or generally N-amino acid-O, is the abbreviation for a compound of formula 1, wherein X is an C_{α}-amino acid sidechain. N indicated the hydrazine group and O the oxalic acid.

### Example 64: N-{(S)-1-Benzyl-2-[N'-((S)-2-tert-butoxycarbonylamino-propionyl)-hydrazino]-2-oxo-ethyl}-oxalamic acid (Boc-Ala-NPheO-OH)

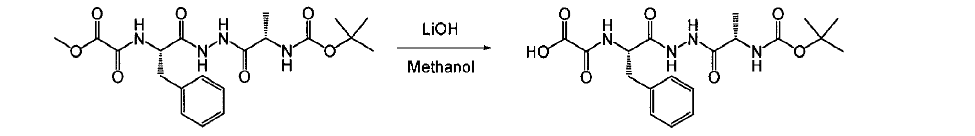

To a solution of N-{(S)-1-Benzyl-2-[N'-((S)-2-tert-butoxycarbonylamino-propionyl)-hydrazino]-2-oxo-ethyl}-oxalamic acid methyl ester (Example 63) (2.9 g, 0.63 mmol) was added drop wise the aqueous solution of LiOH (176 mg, 7.36 mmol) in 0.5 mL of water. The reaction mixture was stirred further for 1 h. Methanol was evaporated and the residue obtained was dissolved in water (15 mL), washed with ether (2 x 15 mL), acidified with 1N HC1 and extracted with ethyl acetate (3 x 15 mL). The combined ethyl acetate layer was washed with brine (2 x 15 mL), dried (Na₂SO₄) and concentrated. The crude compound was triturated with diethyl ether and filtered to give 2.25g (79%) of desired product as a white crystalline solid; mp 162-163°C.

¹H NMR ( 200 MHz, DMSO-d₆) δ (ppm) 10.21 (s, 1H), 9.90 (s, 1H), 8.69 (d, *J =* 10.16 Hz, 1H), 7.15-7.27 (m, 5H), 6.97 (d, *J =* 7.24Hz, 1H), 4.51-4.62 (m, 1H), 3.99-4.07 (m, 1H), 2.93-3.16 (m, 2H), 1.36 (s,9H), 1.21 (d, *J* = 7.24 Hz, 3H).

¹³C NMR ( 200 MHz, DMSO-d₆) δ (ppm) 171.79, 168.99, 161.44, 158.11, 154.92, 137.47, 129.1, 128.05, 126.35, 77.90, 53.07, 48.15, 36.86, 28.15, 18.14.

IR (KBr) ν 4000 - 3331, 3258, 3032, 2980, 2937, 1765, 1686, 1616, 1529, 1481 cm⁻¹.

### Example 65: [(S)-1-(N'-{(S)-2-[((S)-1-Dimethylcarbamoyl-ethyl-aminooxalyl)-amino]-3-phenyl-propionyl}-hydrazinocarbonyl)-ethyl]-carbamic acid tert-butyl ester (Boc-Ala-NPheO-Ala-NMe₂)

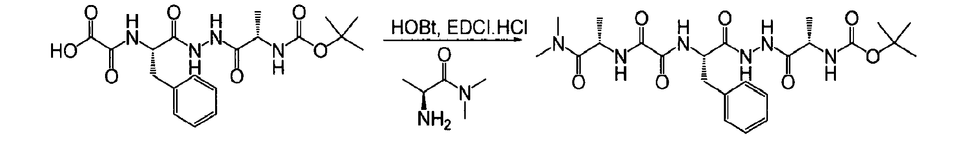

To the solution of N-{(S)-1-Benzyl-2-[N'-((S)-2-tert-butoxycarbonylamino-propionyl)-hydrazino]-2-oxo-ethyl}-oxalamic acid (Example 64) (500 mg, 1.18 mmol) in DCM (10 mL) was added HOBt (167.9 mg, 1.24 mmol) followed by EDCI.HCl (238.2 mg, 1.24 mmol). To this a solution of compound (59) (144.4 mg, 1.24 mmol) in DCM (5mL) was added followed by triethylamine (0.17 mL, 1.24 mmol) and the mixture was stirred overnight at room temperature.

DCM removed by rotary evaporation and the residue obtained was dissolved in ethyl acetate (15 mL). The ethyl acetate solution was washed with 1N HCl (5 mL), 10 % NaHCO₃ (5 mL), brine (5 mL), dried (Na₂SO₄) and concentrated to give 325 mg (52%) of product which was crystallised from ethyl acetate (10 mL) to give white crystalline solid; mp. 199°C.

¹H NMR (200 MHz, DMSO-d₆) δ (ppm) 10.24 (s, 1H), 9.92 (s, 1H), 8.57 (d, *J* = 8.4 Hz, 1H), 8.37 (d, *J* = 7.64 Hz, 1H), 7.15-7.28 (m, 5H), 6.97 (d, *J* = 7.24 Hz, 1H), 4.56-4.72 (m, 2H), 3.99-4.07 (m, 1H), 3.05-3.1 (m, 2H), 2.99( s, 3H ), 2.82 (s, 3H ), 1.37 (s, 9H), 1.18-1.23 (m, 6H).

¹³C NMR (200 MHz, DMSO-d₆) δ (ppm) 171.8, 170.57, 168.89, 159.19, 158.09, 154.94, 137.25, 129.14, 128.08, 126.44, 77.92, 53.04, 48.14, 45.22, 37.02, 36.35, 35.21, 28.15, 18.11, 17.22.

IR (KBr) ν 4000 - 3353, 3280, 3030, 2983, 2936, 1718, 1686, 1656, 1627, 1516 cm⁻¹.

### Example 66: (Boc-Ala-NPheO-O-Merrifield)

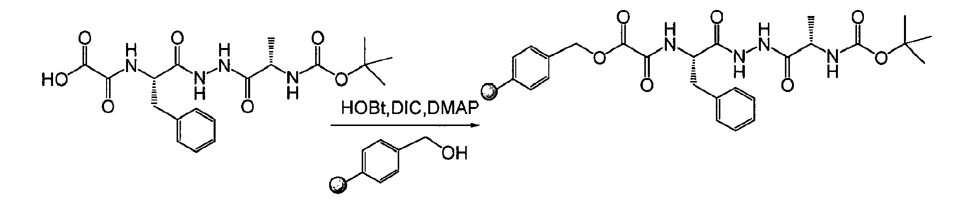

The hydroxymethyl resin (100 mg, 0.104 mmol) was suspended in 9:1 v/v CH₂Cl₂/DMF (1 mL). In a separate flask HOBt (42.16 mg, 0.31 mmol) was added to the solution of N-{(S)-1-Benzyl-2-[N'-((S)-2-tert-butoxycarbonylamino-propionyl)-hydrazino]-2-oxoethyl}-oxalamic acid (Example 64) (131.8 mg, 0.31 mmol) in minimum amount of DMF. The mixture was stirred until the HOBt got dissolved and this solution was added to the resin. DIC (0.05 mL, 0.31 mmol) was then added to the reaction mixture followed by the solution of DMAP (13 mg, 0.11 mmol) in minimum amount of DMF. The reaction mixture was agitated overnight at room temperature with a mechanical shaker under argon. Acetic anhydride and pyridine (2 equivalents relative to the resin) were added to the reaction mixture and agitated for an additional 30 min at room temperature to end-cap any unreacted hydroxyl groups on the resin.

Resin was filtered in a fine sintered glass funnel and washed with DMF (3 x 5 mL), DCM (3 x 5 mL), methanol (3 x 5 mL) and dried in vacuum to a constant weight.

### Example 67: (Ala-NPheO-O-Merrifield)

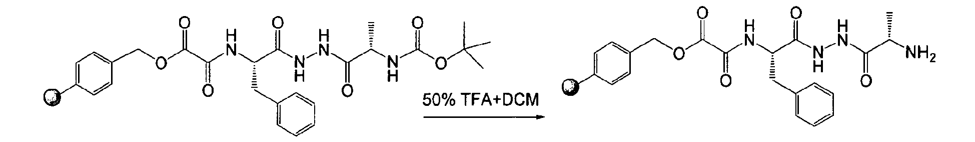

The suspension of resin (Example 66) (0.104 mmol) in 50% (v/v) TFA/DCM (1 mL) was agitated at room temperature using a mechanical shaker for 30 min. Resin was filtered in a fine sintered glass funnel and washed with DCM (3 x 5 mL) followed by 5% (v/v) DIPEA (2 mL) to remove TFA and dried in vacuum to a constant weight.

### Example 68: (Boc-Phe-Ala-NPheO-O-Merrifield)

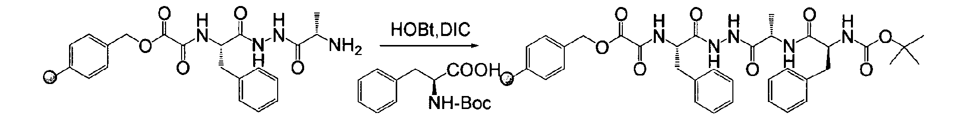

The resin (Example 67) (0.104 mmol) was suspended in 9:1 v/v CH₂Cl₂/DMF (1 mL). In a separate flask HOBt (42.16 mg, 0.31 mmol) was added to the solution of Boc-L-Phenylalanine (82.8 mg, 0.31 mmol) in minimum amount of DMF. The mixture was stirred until the HOBt got dissolved and this solution was added to the resin. DIC (0.05 mL, 0.31 mmol) was then added and the reaction mixture was agitated overnight at room temperature with a mechanical shaker under argon.

Resin was filtered in a fine sintered glass funnel and washed with DMF (3 x 5 mL), DCM (3 x 5 mL), methanol (3 x 5 mL) and dried in vacuum to a constant weight.

### Example 69: N-((S)-1-Benzyl-2-{N'-[(S)-2-((S)-2-tert-butoxycarbonylamino-3-phenyl-propionylamino)-propionyl]-hydrazino}-2-oxoethyl)-oxalamic acid methyl ester (Boc-Phe-Ala-NPheO-OMe)

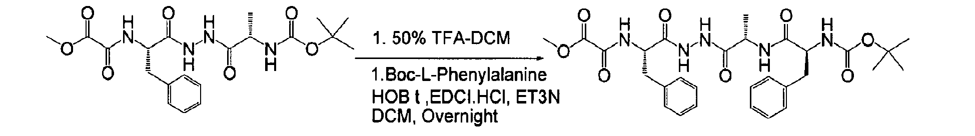

To the solution of N-{(S)-1-Benzyl-2-[N'-((S)-2-tert-butoxycarbonylamino-propionyl)-hydrazino]-2-oxo-ethyl}-oxalamic acid methyl ester (Example 63) (178 mg, 0.41 mmol) in DCM (5 mL), TFA (5 mL) was added slowly with stirring at 0°C. The reaction mixture was stirred at room temperature for 30 min. DCM and TFA were evaporated and the reaction mixture was well dried under high vacuum, the residue obtained was dissolved in dry DCM (5 mL) and was added to another flask containing the solution of Boc-L-Phenylalanine (113.6 mg, 0.43 mmol), HOBt (57.86 mg, 0.43 mmol), EDCI.HCl (82.09 mg, 0.43 mmol) in DCM (5 mL). Triethylamine (0.17 mL 1.22 mmol) was added and the reaction mixture was stirred at room temperature for overnight.

DCM removed by rotary evaporation and the residue obtained was dissolved in ethyl acetate (10 mL). The ethyl acetate solution was washed with 1N HCl (5 mL), 10 % NaHCO₃ (5 mL), brine (5 mL), dried (Na₂SO₄) and concentrated to give 95 mg (39%) of desired product as a white solid.

¹H NMR ( 200 MHz, DMSO-d₆) δ (ppm) 10.29 (br s, 1H), 10.05 (br s, 1H), 9.04 (d, J = 9.78 Hz, 1H), 8.17 (d, J = 8.42 Hz, 1H), 7.27-7.35 (m, 10H), 6.95 (d, J = 9.78 Hz, 1H), 4.61-4.72 (m, 1H), 4.41-4.49 (m, 1H), 4.07-4.32 (m, 1H), 3.8 (s, 3H), 2.94-3.25 (m, 3H), 2.68-2.82 (m, 1H), 1.34 (m, 12H).

### Example 70: N-((S)-1-Benzyl-2-N'{(S)-2-((S)-2-tert-butoxycarbonylamino-3-phenyl-propionylamino)-propionyl]-hydrazino}-2-oxoethyl)-oxalamic acid (Boc-Phe-Ala-NPheO-OH)

### a) via solid phase synthesis:

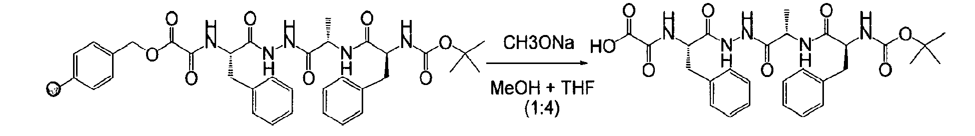

To the suspension of resin (Example 68) (0.104 mmol) in 1:4 v/v methanol - THF (2 mL) was added sodium methoxide (0.6 mg, 0.01 mmol). The reaction mixture was stirred at 70°C for 18 h. Water (3 drops) was added and it was stirred for additional 30 min at 70°C. The reaction mixture was filtered through a fine glass sintered funnel, washed with methanol (5 mL). The filtrate was concentrated to remove methanol, the residue obtained was dissolved in 10% NaHCO₃ aqueous solution (5 mL) and washed with ether (2 x 5 mL). The aqueous layer was cooled and acidified with 1N HCL and extracted with ethyl acetate (3 x 5 mL). The combined organic layer was washed with brine (2 x 5 mL), dried (Na₂SO₄) and concentrated to give 9 mg (15%) of the product as an off-white crystalline solid.

### b) via liquid phase synthesis

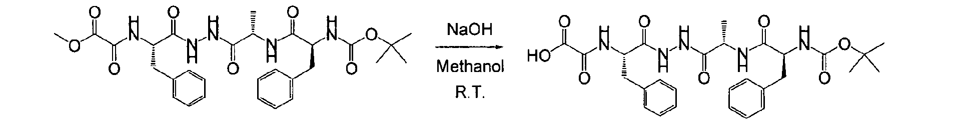

To the solution of N-((S)-1-Benzyl-2-{N'-[(S)-2-((S)-2-tert-butoxycarbonylamino-3-phenyl-propionylamino)-propionyl]-hydrazino}-2-oxo-ethyl)-oxalamic acid methyl ester (Example 69) (85 mg, 0.15 mmol) in methanol (2 mL) was added sodium hydroxide (6 mg, 0.15 mmol), and stirred at room temperature for 1 h. Methanol was evaporated and the residue obtained was dissolved in water (10 mL), washed with ether (2 x 5 mL). The aqueous layer was cooled in ice bath, acidified (pH = 4) with 1N HCl and extracted with ethyl acetate (3 x 10 mL). The combined organic layer was washed with brine (10 mL), dried (Na₂SO₄) and concentrated to give 26 mg (31%) of desired compound as a off-white crystalline solid; mp. 155 - 156°C

¹H NMR ( 200 MHz, DMSO-d₆) δ (ppm) 10.28 (br s, 1H), 10.04 (br s, 1H ), 8.79 (br s, 1H), 8.15 (br s, 1H), 6.5-7.27 (m, 10H), 4.39 (m, 2H), 2.76-3.2 (m, 5H), 1.29 (br s, 12H).

IR (KBr) ν 4000 - 3299 (broad), 3030, 2979, 2933, 1702, 1696, 1687, 1674, 1652, 1508 cm⁻¹.

### Example 71: [(S)-2-Methyl-1-((S)-3-methyl-1-methylcarbamoyl-butylcarbamoyl)-butyl]-carbamic acid tert-butyl ester

### Step 1:

### ((S)-3-Methyl-1-methylcarbamoyl-butyl)-carbamic acid tert-butyl ester:

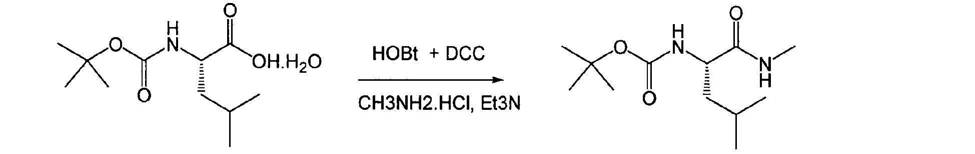

To the solution of Boc-L-Leucine monohydrate (2.0 g, 8.02 mmol) in DCM (30 mL), HOBt (1.14 g, 8.42 mmol) was added followed by DCC (1.74 g, 8.42 mmol). To this solution a suspension of methylamine hydrochloride (596 mg, 8.82 mmol) and triethylamine (1.68 mL, 12.03 mmol) in DCM (15 mL) was added and the mixture was stirred at room temperature for 24 h.

The precipitated DCHU was removed by filtration and the filtrate was evaporated. The additional DCHU was removed by subsequent trituration with cold ethyl acetate and filtration. The ethyl acetate solution was washed with 1N HCl (2 x 15 mL), 10 % NaHCO₃ (15 mL), brine (15 mL), dried (Na₂SO₄) and concentrated to give 1.5 g (76%) of desired product as a white solid.

¹H NMR (200 MHz, CDCl₃) δ (ppm) 6.73 (br s, 1H), 5.2 (br s, 1H), 4.07-4.1 (m, 1H), 2.71 (d, *J* = 4.7 Hz, 3H), 1.36-1.67 (m, 3H), 1.3 (s, 9H), 0.86 (dd, *J* = 2.34, 2.14 Hz, 6H).

¹³C NMR (200 MHz, CDCl₃) δ (ppm) 173.46, 155.81, 79.76, 53, 41.62, 28.26, 26.03, 24.68, 22.88, 21.91.

### Step 2:

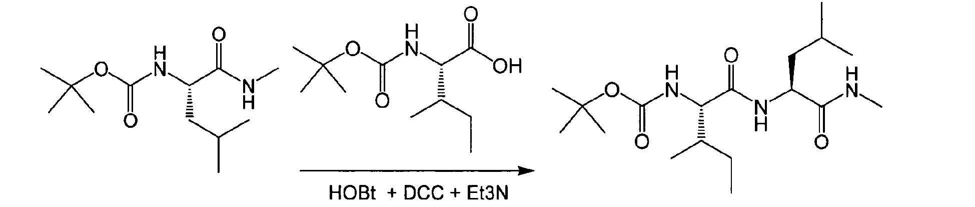

To the solution of ((S)-3-Methyl-1-methylcarbamoyl-butyl)-carbamic acid tert-butyl ester (1.47 g, 6.02 mmol) in dichloromethane (15 mL), TFA (15 mL) was added slowly with stirring at 0°C. The reaction mixture was stirred at room temperature for 30 min. Solvent was evaporated and was well dried under high vacuum, the residue obtained was dissolved in dichloromethane (30 mL). To this solution was added Boc-Isoleucine (compound 71) (1.53 g, 6.62 mmol), HOBt (0.89 g, 6.62 mmol), DCC (1.74 g, 6.62 mmol ) followed by triethylamine (2.53 mL, 18.05 mmol) and the mixture was stirred overnight at room temperature.

The precipitated DCHU was removed by filtration and the filtrate was evaporated. The additional DCHU was removed by subsequent trituration with cold ethyl acetate and filtration.The ethyl acetate solution was washed with 1N HCl (2 x 15 mL), 10 % NaHCO₃ (15 mL), brine (15 mL), dried (Na₂SO₄) and concentrated to give 2.1 g ( 97%) of desired product as a white solid; mp. 151-152°C.

R*_{f}* = (1:1 EtOAc/Pentane)

¹H NMR (200 MHz, CDCl₃) δ (ppm) 7.19 (br s, 1H), 6.95 (br s, 1H), 5.42 (d, *J* = 8.2 Hz, 1H), 4.37-4.48 (m, 1H), 3.92-4.0 (m, 1H), 2.69 (d, *J* = 4.7 Hz, 3H), 1.36-1.81 (m, 5H), 1.27 (s, 9H), 0.97-1.25 (m, 1H), 0.77-0.86 (m, 12H).

¹³C NMR (200 MHz, CDCl₃) δ (ppm) 172.51, 172.18, 155.97, 79.74, 59.36, 51.69, 40.44, 37.08, 28.25, 26.03, 24.92, 24.71, 22.79, 21.98, 15.39, 11.22.

### Example 72: {(S)-1-[(S)-2-Methyl-1-((S)-3-methyl-1-methylcarbamoyl-butylcarbamoyl)-butylcarbamoyl]-2-phenyl-ethyl}-carbamic acid tert-butyl ester

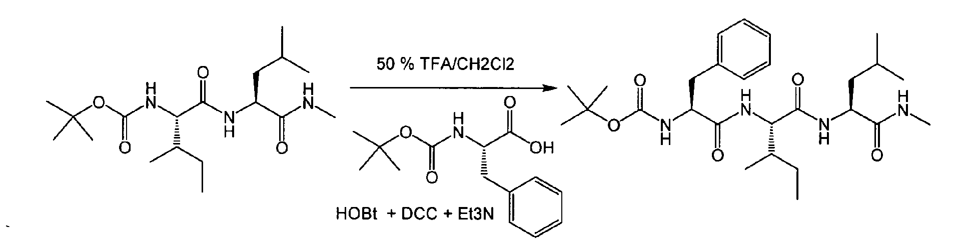

To the solution of [(S)-2-Methyl-1-((S)-3-methyl-1-methylcarbamoyl-butylcarbamoyl)-butyl]-carbamic acid tert-butyl ester (Example 71) (2.1 g, 6.02 mmol) in dichloromethane (15 mL), TFA (15 mL) was added slowly with stirring at 0°C. The reaction mixture was stirred at room temperature for 30 min. Solvent was evaporated and was well dried under high vacuum, the residue obtained was dissolved in dichloromethane (30 mL). To this solution was added JP-102 (1.53 g, 6.62 mmol), HOBt (0.89 g, 6.62 mmol), DCC (1.74 g, 6.62 mmol ) followed by triethylamine (2.53 mL, 18.05 mmol) and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (2 x 15 mL), 10 % NaHCO₃ (15 mL), brine (15 mL), dried (Na₂SO₄) and concentrated to give 2.1 g (97%) of desired product as a white solid; mp. 151-152°C.

R*_{f}* = (1:1 EtOAc/Pentane)

¹H NMR (200 MHz, DMSO-d₆) δ (ppm) 7.98 (d, *J* = 7.82 Hz, 1H), 7.78 (br s, 2H), 7.23 (s, 5H), 6.97 (d, *J* = 7.82 Hz, 1H), 4.19-4.23 (m, 3H), 2.53-2.97 (m, 5H), 1.01-1.72 (m, 15H), 0.79-0.82 (m, 12H).

### Example 73: Synthesis of a beta-turn peptide motif using a compound of formula 1 as substructure Step 1:

2-[[(2-ethoxy-2-oxoethyl)amino]carbonyl]-hydrazinecarboxylic acid, 1,1-dimethylethyl ester (Boc-Aza-Gly-OEt)

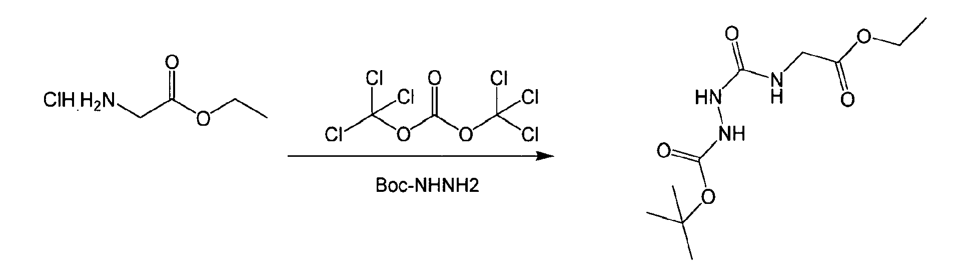

To the solution of triphosgene (3.4 g, 11.46 mmol) in CH₂Cl₂ (10 mL), a mixture of glycine ethyl ester hydrochloride (4.0 g, 28.66 mmol) and DIEA (14.87 mL, 85.97 mmol) in CH₂Cl₂ (15 mL) was added slowly over a period of 30 min. After a further 15 min of stirring, a solution of Boc-Hydrazine (3.79 g, 28.66 mmol) in CH₂Cl₂ (15 mL) was added in one portion. The reaction mixture was further refluxed for 2 h, evaporated to dryness, diluted with ethyl acetate, washed with 10% aq NaHCO₃ and brine, dried over Na₂SO₉ and evaporated to give 5.6 g of crude compound which was purified by column chromatography to give 3.63 g (48%) of product as a sticky mass which after overnight standing at rt give off white solid; mp. 137-138°C.

R*_{f}* = 0.29 (5% methanol in CH2Cl₂)

¹H NMR (200 MHz, CDCl₃) δ (ppm) 7.2 (bs, 1H), 7.05 (s, 1H), 6.2 (t, *J* = 5.46 Hz, 1H), 4.11 (q, *J* = 7.04 Hz, 2H), 3.92 (d, *J* = 5.66 Hz, 2H), 1.39 (s, 9H), 1.2 (t, *J* = 7.04 Hz, 3H).

¹³C NMR (200 MHz, CDCl₃) δ (ppm) 171.07, 158.95, 156.43, 81.51, 61.25, 41.79, 28.09, 14.04.

### References for this step:

This compound was prepared as described in Gacel, G.; Zajac, J. M.; Delay-Goyet, P.; Dauge, V.; Roques, B. P. Investigation of the structural parameters involved in the µ and δ opioid receptor discrimination of linear enkephalin-related peptides. Journal of Medicinal Chemistry (1988), 31(2), 374-83.

The use of triphosgene for related reactions is described in: Majer, Pavel; Randad, Ramnarayan S. A Safe and Efficient Method for Preparation of N,N'-Unsymmetrically Disubstituted Ureas Utilizing Triphosgene. Journal of Organic Chemistry (1994), 59(7), 1937-8.

### Step 2: Boc-Pro-Aza-Gly-OEt

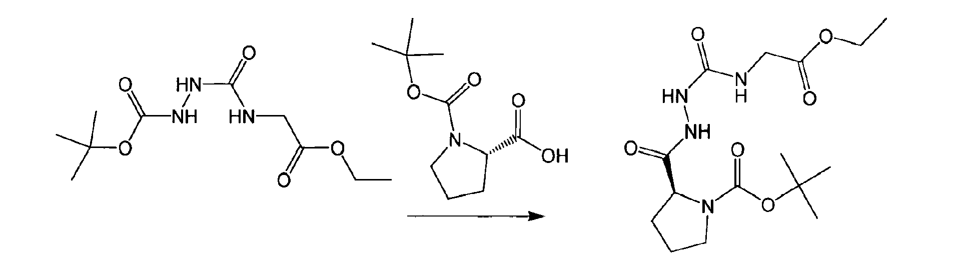

To the solution of Boc-Aza-Gly-OEt (3.63 g, 13.89 mmol) in dichloromethane (15 mL), TFA (15 mL) was added slowly with stirring at 0°C, the reaction mixture was stirred at room temperature for 30 min. Solvent was evaporated and was well dried under high vacuum, the residue obtained was dissolved in dichloromethane (15 mL). In another flask Boc-proline (2.99 g, 13.89 mmol), HOBt (1.97 g, 14.59 mmol) and DCC (3.09 g, 14.59 mmol) were dissolved in dichloromethane (15 mL) and stirred for 15 min at rt, to this a mixture of above TFA salt of JP-120 and triethylamine (4.77 mL, 34.73 mmol) in dichloromethane (15 mL) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (2 x 15 mL), 10 % NaHCO₃ (15 mL), brine (15 mL), dried (Na₂SO₄) and concentrated to give 5.2 g of crude compound which was purified by column chromatography to give 3.2 g (48%) of product as a brown solid; mp. 58-59°C.

¹H NMR ( 200 MHz, CDCl₃) δ (ppm) 9.03 (bs, 1H), 7.47 (bs, 1H), 6.65 (bs, 1H), 3.76-4.2 (m, 5H), 3.35-3.49 (m, 2H), 1.67-2.08 (m, 4H), 1.35 (s, 9H), 1.19 (t, *J* = 7.04 Hz, 3H).

¹³C NMR (200 MHz, CDCl₃) δ (ppm) 173.1, 172.7, 171.07, 155.48, 80.56, 61.19, 59.02, 47.13, 41.78, 29.38, 28.33, 24.62, 14.07.

### Example 74: EtO-Gly-NProO-O-tBu (Step 3)

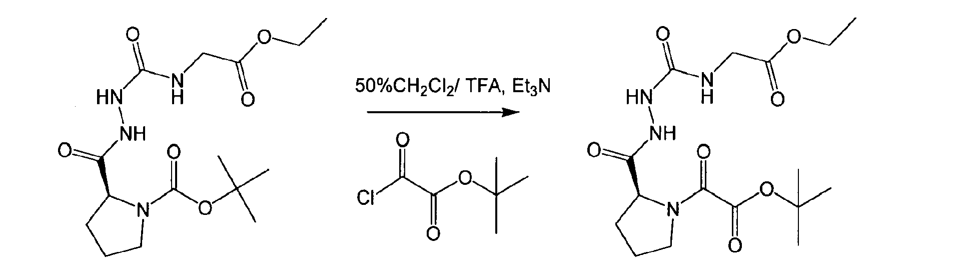

To the solution of Boc-Pro-Aza-Gly-OEt (Example 73) (2.63 g, 7.34 mmol) in dichloromethane (15 mL), TFA (15 mL) was added slowly with stirring at 0°C, the reaction mixture was stirred overnight at room temperature. Solvent was evaporated and was well dried under high vacuum. The residue obtained was dissolved in dichloromethane (30 mL), to that was added slowly with stirring triethylamine (4.1 mL, 29.35 mmol) at 0°C, followed by t-butyl oxalyl chloride (1.27 g, 7.71 mmol) and the reaction mixture was further stirred under argon at rt for 30 min. The solvent was evaporated and the crude compound obtained was purified by column chromatography to give 2.2 g (77%) of product as a sticky mass.

¹H NMR ( 200 MHz, CDCl₃) δ (ppm) 9.1 (bs, 1H), 7.54 (bs, 1H), 6.46 (t, *J* = 5.48 Hz, 1H), 4.38 (t, *J* = 5.48 Hz, 1H), 4.09 (q, *J* = 7.04 Hz, 2H) 3.58-4.0 (m, 4H), 1.73-2.16 (m, 4H), 1.47 (s, 9H), 1.18 (t, *J* = 7.04 Hz, 3H).

¹³C NMR (200 MHz, CDCl₃) δ (ppm) 171.26, 171.16, 160.59, 160.02,158.76, 84.6, 61.29, 59.08, 48.57, 41.85, 28.62, 27.83, 27.68, 25.1, 14.05.

### Example 75: Boc-Aza-Gly-N(Me)₂ (Step 4)

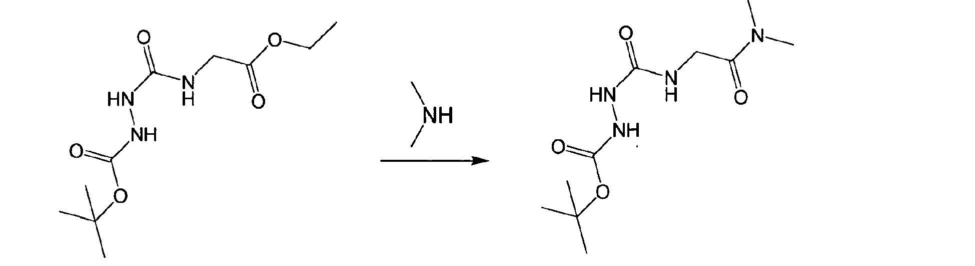

The solution of Boc-Aza-Gly-OEt (73 step-1) (3.8 g, 14.54 mmol) in 33% solution of dimethylamine in ethanol (20 mL) was stirred overnight at room temperature. Solvent was evaporated and was well dried under high vacuum to give 3.78 g (100%) of product as an off-white solid; mp.71-72°C.

R*_{f}* = 0.24 (5% methanol in CH₂Cl₂)

¹H NMR (200 MHz, CDCl₃) δ (ppm) 7.35 (bs, 1H), 7.07 (s, 1H), 6.48 (t, *J* = 4.3 Hz, 1H), 4.0 (d, *J* = 4.3 Hz, 2H), 2.92 (s, 3H), 2.89 (s, 3H), 1.38 (s, 9H).

¹³C NMR (200 MHz, CDCl₃) δ (ppm) 169.15, 158.74, 156.33, 81.08, 41.8, 36.01, 35.64, 28.13.

### Example 76: Boc-Pro-Aza-Gly-N(Me)2 (Step 5)

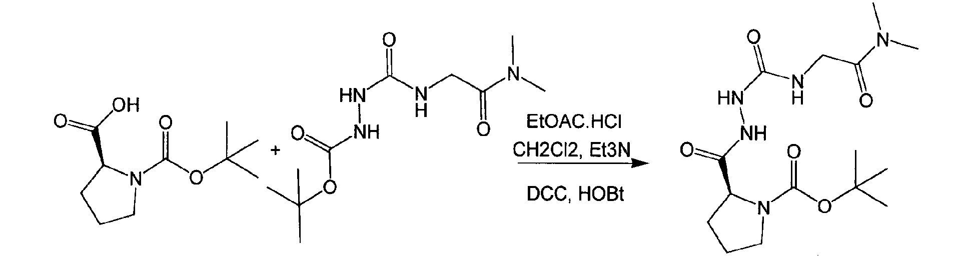

To the solution of Boc-Aza-Gly-N(Me)₂ (Example 75) (1.87 g, 7.2 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. In another flask Boc-proline (1.55 g, 7.2 mmol), HOBt (1.02 g, 7.56 mmol) and DCC (1.56 g, 7.56 mmol) were dissolved in dichloromethane (15 mL) and stirred for 15 min at rt, to this a mixture of above hydrochloride salt of JP-129 and triethylamine (3.0 mL, 21.6 mmol) in dichloromethane (15 mL) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was concentrated. The crude compound was purified by column chromatography to give 2.1 g (81%) of product as a white solid: mp. 74-75°C.

R*_{f}* = 0.51 (10% methanol/CH₂Cl₂)

¹H NMR ( 200 MHz, CDCl₃) δ (ppm) 8.89 (bs, 1H), 7.75 (bs, 1H), 6.56 (bs, 1H), 3.87-4.22 (m, 3H), 3.41 (m, 2H), 2.91 (s, 3H), 2.88 (s, 3H), 1.68-2.1 (m, 4H), 1.36 (s, 9H).

¹³C NMR (200 MHz, CDCl₃) δ (ppm) 172.46, 169.06, 158.28, 155.33, 80.33, 58.72, 53.44, 47.07, 41.83, 36.05, 35.71, 28.33, 24.49.

### Example 77: t-BuO-Oxalyl-Pro-Aza-Gly-(NMe)₂ ((Me)2N-Gly-NProO-OtBu)

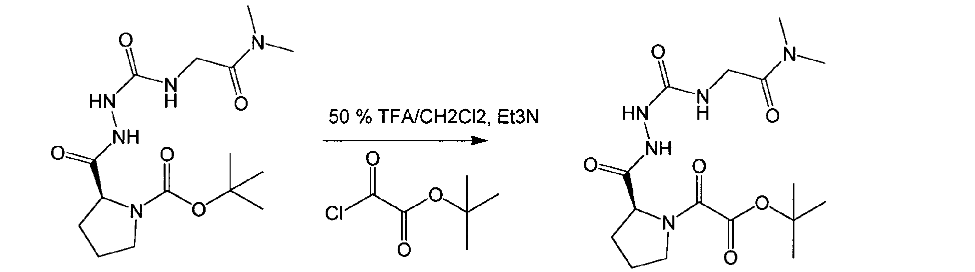

To the solution of Boc-Pro-Aza-Gly-N(Me)₂ (Example 76) (0.72 g, 2.02 mmol) in dichloromethane (10 mL), TFA (10 mL) was added slowly with stirring at 0°C, the reaction mixture was further stirred under argon at room temperature for 30 min. Solvent was evaporated and was well dried under high vacuum. The residue obtained was dissolved in dichloromethane (25 mL), to that was added slowly with stirring triethylamine (0.85 mL, 6.04 mmol) at 0°C, followed by *tert*-butyl oxalyl chloride (0.33 g, 2.02 mmol) and the reaction mixture was further stirred under argon at rt for 30 min. The solvent was evaporated and the crude compound obtained was purified by column chromatography to give 250 mg (32%) of product as white solid. mp. 172-173°C.

R*_{f}* = 0.36 (10% methanol/CH₂Cl₂)

¹H NMR (200 MHz, DMSO-d₆) δ (ppm) 9.78 (bs, 1H), 8.15 (s, 1H), 6.28 (m, 1H), 4.41 (m, 1H), 3.97 (m, 2H), 3.56 (m, 2H), 2.91 (s, 3H), 2.83 (s, 3H), 1.8-2.35 (m, 4H), 1.46 (s, 9H)

### Example 78: (Me)₂N-Gly-NProO-NCH(CH₃)₂

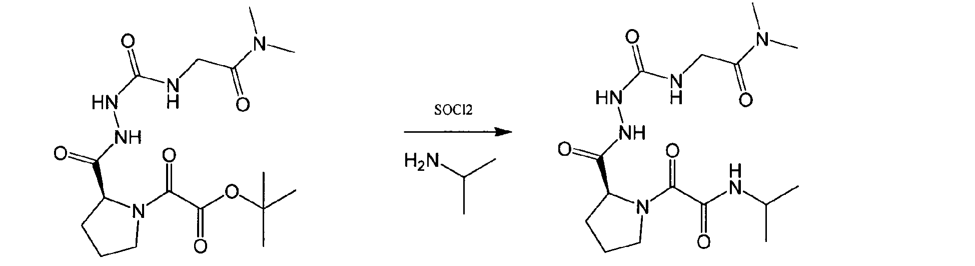

To the solution of *t*-BuO-Oxalyl-Pro-Aza-Gly-(NMe)₂ (Example 77) (220 mg, 0.57 mmol) in dichloromethane (5 mL), TFA (5 mL) was added slowly with stirring at 0°C, the reaction mixture was stirred overnight at room temperature. Solvent was evaporated and was well dried under high vacuum. The residue obtained was dissolved DCM (15 mL), to that was added with stirring thionyl chloride (0.21 mL, 2.85 mmol) followed by catalytic DMF (2 drops). The reaction mixture was further stirred overnight under argon at room temp. Solvent was evaporated on rotavap, the excess of thionyl chloride was removed under high vacuum. The residue obtained was dissolved in dichloromethane (15 mL), to this was added slowly with stirring isopropyl amine (0.15 mL, 1.72 mmol) at 0°C. The mixture was diluted with ethyl acetate (30 mL) and washed with 1N HCL (10 mL), followed by 10% NaHCO₃ (10 mL) and brine (10 mL). The ethyl acetate layer was over Na₂SO₄ and concentrated to give 220 mg of crude compound which was purified by column chromatography to give 150 mg (70%) of the desired product as a white solid.

¹H NMR ( 200 MHz, CDCl₃) δ (ppm) 9.71 (bs, 1H), 9.56 (bs, 1H), 8.11 (bs, 1H), 6.28 (bs, 1H), 4.34 (m, 1H), 4.05 (m, 2H), 3.94 (m, 1H), 3.6 (m, 2H), 2.92 (s, 3H), 2.89 (s, 3H), 1.87-2.39 (m, 4H), 1.18- 1.3 (m, 6H)

¹³C NMR (200 MHz, CDCl₃) δ (ppm) 170.5, 164.9, 162.1, 162.0, 158.5, 62.7, 46.57, 43.1, 41.1, 36.8, 37.2, 30.1, 25.27, 23.63, 24.1.

### Example 79: (S)-2-(tert-Butoxyoxalyl-ethyl-amino)-butyric acid methyl ester (Step 6)

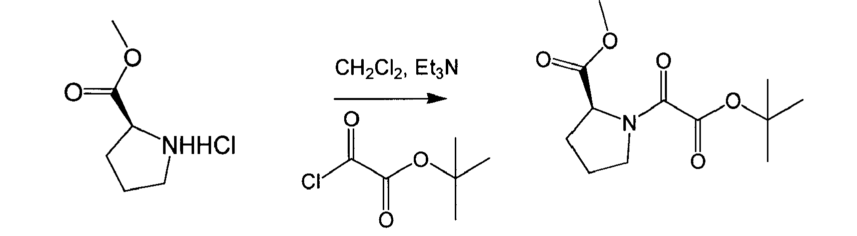

To a solution of L-proline methyl ester hydrochloride (3.02 g, 18.22 mmol) in DCM (30 mL) was added slowly with stirring triethylamine (7.65 mL,54.67 mmol) at 0°C, followed by t-butyl oxalyl chloride (3.15 g, 19.13 mmol). The reaction mixture was further stirred under argon at rt for 30 min.

The mixture was diluted with ethyl acetate (50 mL) and washed with 1N HCL (10 mL), followed by 10% NaHCO₃ (10 mL) and brine (10 mL). The ethyl acetate layer was dried (Na₂SO₄) and concentrated to give 4.21 g (89%) of pure product as a dark brown viscous oil.

R*_{f}* = 0.38 (1:2 EtOAc/Pentane)

¹H NMR (200 MHz, CDCl₃) δ (ppm) 4.76 (dd, *J* = 3.72 Hz, 1H), 4.46 (dd, 3.7, 3.12 Hz, 1H), 3.5-3.82 (m, 10H), 1.78-2.33 (m, 8H), 1.51 (s, 9H), 1.47 (s, 9H). (two rotamers visable in the spectra)

### Example 80: (S)-2-(tert-Butoxyoxalyl-ethyl-amino)-butyric acid (Step 7)

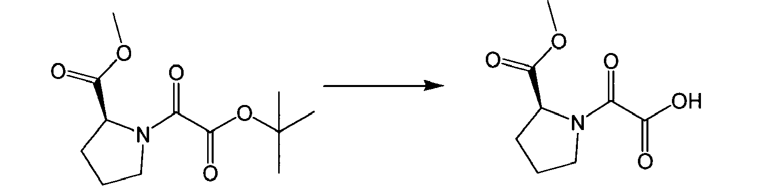

To the solution of compound (S)-2-(tert-Butoxyoxalyl-ethyl-amino)-butyric acid methyl ester (Example 79) (4.1 g, 15.94 mmol) in dichloromethane (15 mL), TFA (15 mL) was added slowly with stirring at 0°C, the reaction mixture was stirred overnight at room temperature. Solvent was evaporated and was well dried under high vacuum to give 3.2 g (100%) of product as a brown viscous oil.

¹H NMR (200 MHz, CDCl₃) δ (ppm) 5.09 (dd, *J* = 3.52, 3.32 Hz, 1H), 4.53 (dd, *J* = 3.52 Hz, 1H), 3.52-4.02 (m, 10H), 1.85-2.36 (m, 8H).

¹³C NMR (200 MHz, CDCl₃) δ(ppm) 170.58, 169.73, 157.76, 155.99, 155.87, 59.74, 59.22, 51.2, 48.1, 47.46, 29.85, 26.97, 23.43, 20.54, 20.28. (two rotamers visable in the spectra)

The synthesis of the compound of example 80 has been described in: Kraus, George A.; Melekhov, Alex. A direct route to acylhydroquinones from α-keto acids and α-carboxamido acids. Tetrahedron Letters (1998), 39(23), 3957-3960.

### Example 81: (S)-1-Isopropylaminooxalyl-pyrrolidine-2-carboxylic acid methyl ester (Step 7)

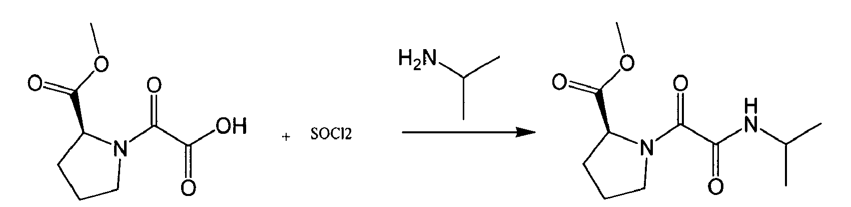

To a solution of (S)-2-(tert-Butoxyoxalyl-ethyl-amino)-butyric acid (Example 80) (3.2 g, 15.91 mmol) in DCM (30 mL) was added with stirring thionyl chloride (5.81 mL, 79.53 mmol) followed by catalytic DMF (2 drops), the reaction mixture was further stirred overnight under argon at room temp. Solvent was evaporated on rotavap, the excess of thionyl chloride was removed under high vacuum. The residue obtained was dissolved in dichloromethane (30 mL), to this was added slowly with stirring isopropyl amine (3.4 mL, 39.77 mmol) at 0°C. The mixture was diluted with ethyl acetate (50 mL) and washed with 1N HCL (10 mL), followed by 10% NaHCO₃ (10 mL) and brine (10 mL). The ethyl acetate layer was dried (Na₂SO₄) and concentrated to give 3.4 g (88%) of desired compound as a dark brown viscous oil.

R*_{f}* = 0.32 (1:2 EtOAc/Pentane )

¹H NMR ( 200 MHz, CDCl₃) δ (ppm) 7.35 (bs, 2H), 5.17 (dd, *J* = 3.92, 3.7 Hz,1H), 4.4 (dd, *J =* 4.3, 4.1Hz, 1H), 3.48-4.12 (m, 10H), 1.66-2.34 (m, 7H), 1.1-1.22 (m, 10H).

¹³C NMR (200 MHz, CDCl₃) δ (ppm) 172.75, 171.88, 159.74, 159.68, 159.1 158.95, 60.75, 52.25, 49.24, 48.61, 41.50, 41.45, 31.82, 28.33, 25.49, 22.27, 22.24, 22.19, 21.71.

two rotamers visable in the spectra

### Example 82: (2-Phenylamino-ethyl)-carbamic acid 9H-fluoren-9-yl-methyl ester (Step 8)

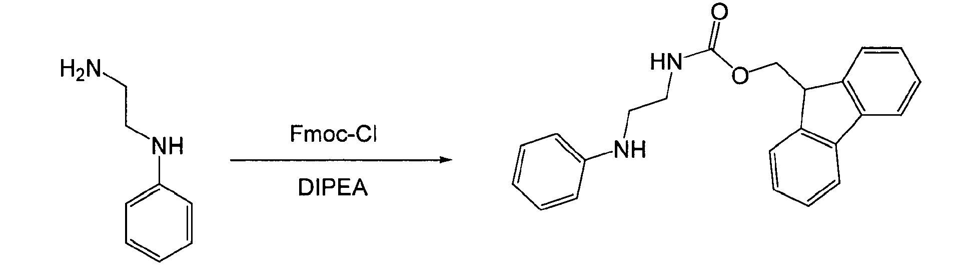

To a mixture of phenyl ethylenediamine (3.0 g, 22.12 mmol), DIPEA (4.65 mL, 26.54 mmol) and DCM (25 mL) was added slowly with stirring and ice-bath cooling a solution of 9-fluorenylmethyl chloroformate (5.72 g, 22.12 mmol) in methylene chloride (25 mL) under argon for a period of 30 min, the reaction mixture was stirred further at room temperature for 15 min. The reaction mixture was then washed with 1N HCL (15 mL), followed by 10% NaHCO₃(15 mL) and brine (15 mL), dried (Na₂SO₄) and concentrated to give 7.8 g of crude compound which was crystallised from ethyl acetate to give 6.5 g (82%) of pure product as white solid; mp. 174-175°C.

R*_{f}* = 0.54 (1:2 EtOAc/Pentane)

¹H NMR (200 MHz, DMSO-d₆) δ (ppm) 7.87 (d, J = 7.24 Hz, 2H), 7.67 (m, 3H), 7.27-7.43 (m, 9H), 4.2-4.31 (m, 3H), 3.31 (bs, 4H) .

¹³C NMR (200 MHz, DMSO-d₆) δ (ppm) 156.07, 143.74, 140.66, 138.6, 138.18, 129.63, 127.58, 127.03, 125.16, 121.09, 120.06, 65.56, 48.46, 46.6, 36.97.

### Example 83: Boc-Aza-(2-Phenylamino-ethyl)-carbamic acid 9H-fluoren-9-ylmethyl ester

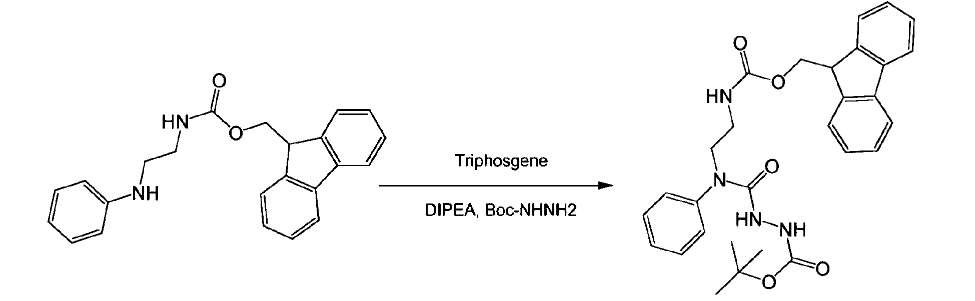

To the solution of triphosgene (578 mg, 1.95 mmol) in CH₂Cl₂ (10 mL), a mixture of (2-Phenylamino-ethyl)-carbamic acid 9H-fluoren-9-ylmethyl ester ((Example 82) (1.79 g, 4.99 mmol) and DIEA (1.0 mL, 5.99 mmol) in CH₂Cl₂ (10 mL) was added slowly over a period of 30 min. After a further 15 min of stirring, a solution of Boc-Hydrazine (990 mg, 7.49 mmol) in CH₂Cl₂ (10 mL) was added in one portion. The reaction mixture was further refluxed for 2 h, evaporated to dryness, diluted with ethyl acetate, washed with 10% aq NaHCO₃ and brine, dried over Na₂SO₄ and evaporated to give 1.9 g of crude compound which was purified by column chromatography to give 1.1 g (42%) of product as a white solid; mp.69-70°C.

R*_{f}* = 0.51 (5% methano in CH₂Cl₂)

¹H NMR (200 MHz, CDCl₃) δ (ppm) 7.67 (d, *J* = 7.22 Hz, 2H), 7.53 (d, *J* = 7.24 Hz, 2H), 7.18-7.42 (m, 9H), 6.33 (bs, 1H), 5.96 (bs, 1H), 5.63 (bs, 1H), 4.23 (d, *J* = 6.84 Hz, 2H), 4.11 (t, *J* = 6.44 Hz, 1H) , 3.77 (m, 2H), 3.33 (m, 2H), 1.37 (s, 9H).

¹³C NMR (200 MHz, CDCl₃) δ (ppm) 157.77, 156.61, 156.28, 144.02, 141.23, 140.22, 130.41, 128.52, 128.27, 127.63, 127.04, 125.23, 119.91, 81.39, 66.85, 49.44, 47.18, 40.3, 28.17.

### Example 84: Preparation of (S)-1-Isopropylaminooxalyl-pyrrolidine-2-carboxylic acid methyl ester

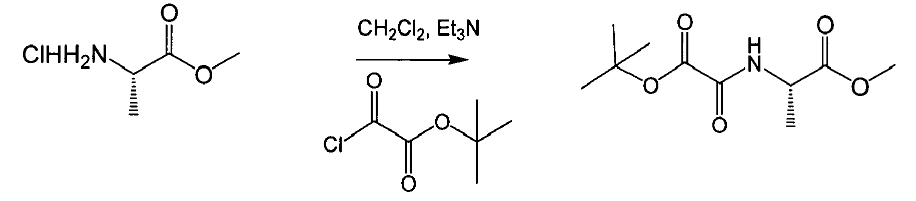

To a solution of L-alanine methylester hydrochloride (3.02 g, 21.62 mmol) in DCM (30 mL) was added slowly with stirring triethylamine (7.15 mL, 64.87 mmol) at 0°C, followed by t-butyl oxalyl chloride (3.74 g, 22.7 mmol). The reaction mixture was further stirred under argon at rt for 30 min. The mixture was diluted with ethyl acetate (50 mL) and washed with 1N HCL (10 mL), followed by 10% NaHCO₃ (10 mL) and brine (10 mL). The ethyl acetate layer was dried (Na₂SO₄) and concentrated to give 4.92 g (98%) of pure product as a dark brown viscous oil.

R_{f} = 0.51 (1:2 EtOAc/Pentane )

¹H NMR (200 MHz, CDCl₃) δ (ppm) 7.58 (bs, 1H), 4.55 (m, 1H), 3.74 (s, 3H), 1.53 (s, 9H), 1.44 (d, *J* = 7.24 Hz, 3H).

¹³C NMR (200 MHz, CDCl₃) δ (ppm) 172.33, 159.02, 156.8, 84.62, 52.61, 48.42, 27.63, 17.97.

### Example 85: (S)-2-(tert-Butylaminooxalyl-amino)-propionic acid methyl ester

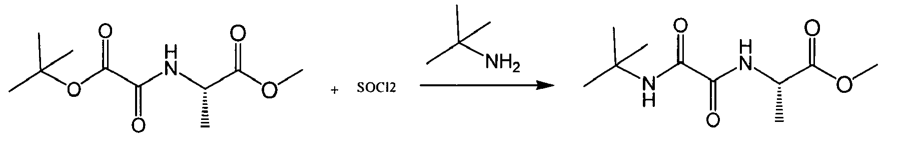

To the solution of preparation of (S)-1-Isopropyl-aminooxalyl-pyrrolidine-2-carboxylic acid methyl ester (Example 84) (1.71 g, 7.4 mmol) in dichloromethane (10 mL), TFA (10 mL) was added slowly with stirring at 0°C, the reaction mixture was stirred overnight at room temperature. Solvent was evaporated and was well dried under high vacuum to give 1.3 g (100%) of product as a brown viscous oil. The residue obtained was dissolved DCM (30 mL), to that was added with stirring thionyl chloride (2.7 mL, 36.97 mmol) followed by catalytic DMF (2 drops). The reaction mixture was further stirred overnight under argon at room temp. Solvent was evaporated on rotavap, the excess of thionyl chloride was removed under high vacuum. The residue obtained was dissolved in dichloromethane (30 mL), to this was added slowly with stirring isopropyl amine (2.33 mL, 22.18 mmol) at 0°C. The mixture was diluted with ethyl acetate (50 mL) and washed with 1N HCL (10 mL), followed by 10% NaHCO₃ (10 mL) and brine (10 mL). The ethyl acetate layer was over Na₂SO₄ and concentrated to give 1.37 g (80%) of desired compound as an off-white solid; mp. 80-81°C.

R*_{f}* = 0.67 (1:2 EtOAc/Pentane)

¹H NMR ( 200 MHz, CDCl₃) δ (ppm) 7.94 (bs, 1H), 7.25 (bs, 1H), 4.47 (m, 1H), 3.69 (s, 3H), 1.4 (d, *J* = 7.24 Hz, 3H), 1.33 (s, 9H).

¹³C NMR (200 MHz, CDCl₃) δ (ppm) 172.03, 160.5, 158.24, 52.53, 51.46, 48.34, 29.87, 28.2, 17.89.

### Example 86: (S)-2-(tert-Butylaminooxalyl-amino)-propionic acid

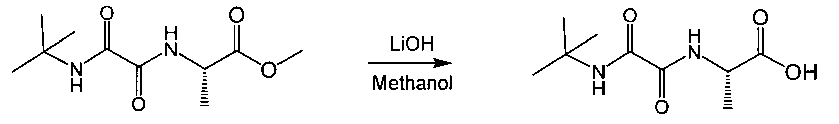

To a solution of (S)-2-(tert-butylaminooxalyl-amino)-propionic acid methyl ester (Example 85) (500 mg, 2.17 mmol) in methanol (5 mL )was added drop wise the aqueous solution of LiOH (62 mg, 2.61 mmol) in 0.5 mL of water. The reaction mixture was stirred further for 20 min at room temperature. Methanol was evaporated and the residue obtained was dissolved in water (10 mL), washed with ether (2 x 5 mL), acidified (pH = 4) with 1N HCl and extracted with ethyl acetate (3 x 105 mL). The combined ethyl acetate layer was washed with brine (2 x 5 mL), dried (Na₂SO₄) and concentrated to give 400 mg (85%) of desired product as a white crystalline solid; mp 118-119°C.

¹H NMR (200 MHz, CDCl₃) δ (ppm) 11.1 (bs, 1H), 8.22 (d, *J* = 7.04 Hz, 1H), 7.43 (s, 1H), 4.5 (m, 1H), 1.45 (d, *J* = 7.24 Hz, 3H), 1.33 (s, 9H).

¹³C NMR (200 MHz, CDCl₃) δ (ppm) 175.64, 160.17, 158.4, 51.79, 48.32, 28.17, 17.44.

### Example 87: peptide mimetic

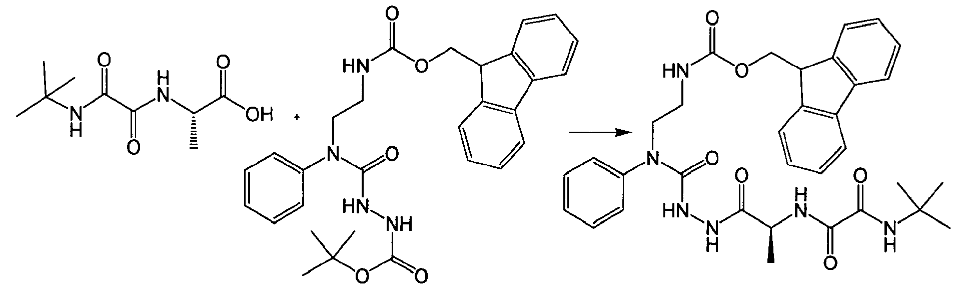

To the solution of Boc-Aza-(2-Phenylamino-ethyl)-carbamic acid 9H-fluoren-9-ylmethyl ester (Example 83) (900 mg, 1.7 mmol) in dichloromethane (10 mL), TFA (10 mL) was added slowly with stirring at 0°C, the reaction mixture was further stirred under argon at room temperature for 30 min (after overnight stirring there is formation of by-product so stirring only for 30 min. is crucial). The solvent was evaporated and was well dried under high vacuum, the residue obtained was dissolved in dichloromethane (15 mL). In another flask (S)-2-(tert-butylaminooxalyl-amino)-propionic acid (Example 86) (377 mg, 1.74 mmol), HOBt (247 mg, 1.83 mmol) and DCC (377 mg, 1.83 mmol) were dissolved in dichloromethane (15 mL) and stirred for 15 min at rt, to this a mixture of above TFA salt of JP-139 and DIPEA (0.34 mL, 1.92 mmol) in dichloromethane (15 mL) was added and the mixture was stirred overnight at room temperature.

The precipitated DCHU was removed by filtration and the filtrate was evaporated. The additional DCHU was removed by subsequent trituration with cold ethyl acetate and filtration.The ethyl acetate solution was washed with 1N HC1 (15 mL), 10 % NaHCO₃ (15 mL), brine (15 mL), dried (Na₂SO₄) and concentrated to give 1.1 g of crude compound which was purified by column chromatography to give 974 mg (90%) of product as a white solid; mp. 48-49°C

R*_{f}* = 0.42 (5% methanol in CH₂Cl₂)

¹H NMR ( 200 MHz, CDCl₃) δ (ppm) 9.25 (bs, 1H), 8.35 (d, *J* = 8.6 Hz, 1H), 7.75 (d, *J* = 7.22 Hz, 2H), 7.58 (d, *J* = 7.24 Hz, 2H), 7.25-7.45 (m, 10H), 6.48 (bs, 1H), 5.88 (bs, 1H), 4.55-4.75 (m, 1H), 4.14-4.29 (m, 3H), 4.25 (m, 2H), 3.4 (m, 2H), 1.45 (d, *J* = 7.04 Hz, 3H),1.35 (s, 9H).

¹³C NMR (200 MHz, CDCl₃) δ (ppm) 170.52, 160.5, 158.29, 157.26, 156.57, 143.95, 141.22, 139.94, 130.46, 128.74, 128.1, 127.63, 127.02, 125.16, 119.92, 66.78, 51.53, 49.64, 47.74, 47.16, 39.78, 28.26, 17.67.

### Example 88: peptide mimetic

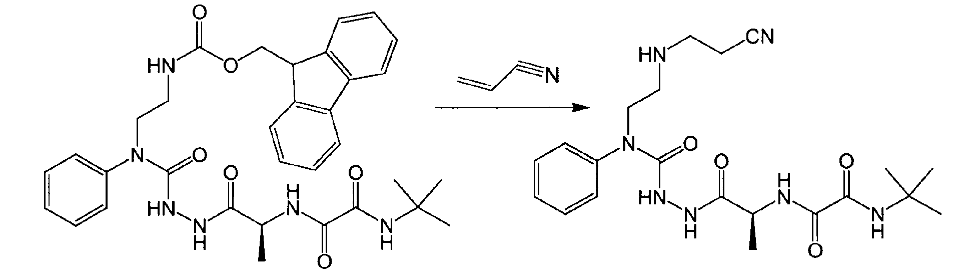

The solution of compound (Example 87) (915 mg, 1.49 mmol) in 10% piperidine in CH₂Cl₂ (15 mL) was stirred at room temperature for 20 min. Solvent was evaporated and the reaction mixture was well dried under high vacuum to remove the traces of piperidine. The residue obtained was crystallised from ethylacetate - pentane to give 575 mg (98%) of free amine. The free amine was dissolved in methanol (15 mL), to that was added acrylonitrile (0.15 mL, 2.23 mmol) and the reaction mixture was stirred overnight at room temperature under argon. The solvent was evaporated and the crude compound was purified by column chromatography to give 333 mg (50%) of product as a white solid; mp. 62-63°C.

R*_{f}* = 0.42 (5% methanol in CH₂Cl₂)

¹H NMR ( 200 MHz, CDCl₃) δ (ppm) 8.14 (d, *J*= 8.4 Hz, 1H), 7.21-7.43 (m, 6H), 4.4-4.55 (m, 1H), 3.72 (t, *J*= 6.06 Hz, 2H), 2.84 (t, *J* = 6.54 Hz, 2H), 2.71 (t, *J* = 6.06 Hz, 2H), 2.41 (t, *J* = 6.65 Hz, 2H), 1.37 (d, *J =* 7.04 Hz, 3H),1.3 (s, 9H).

¹³C NMR (200 MHz, CDCl₃) δ (ppm) 170.51, 160.4, 158.23, 157.09, 140.11, 130.27, 128.48, 128.23, 118.81, 51.51, 49.52, 47.69, 47.01, 44.83, 28.25, 18.61, 17.8.

### Example 89: peptide mimetic

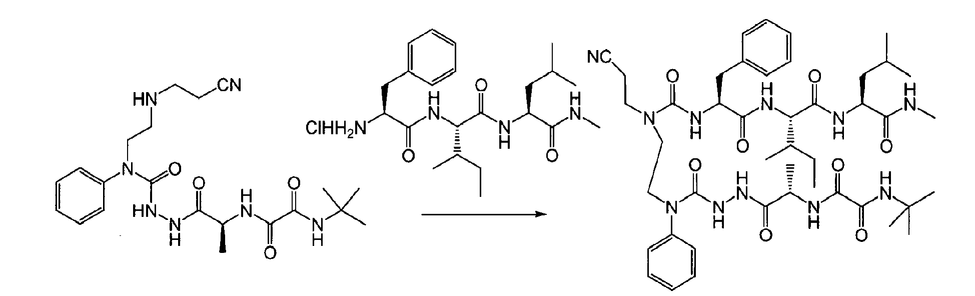

A 25 mL, one necked, round-bottomed flask, was charged with hydrochloride salt of compound (Example 88) (363 mg, 0.82 mmol), 10 mL of CH₂Cl₂, and 10 mL of saturated aqueous NaHCO₃. The biphasic mixture was cooled to 0°C in an ice bath. Stirring was stopped, the layers were allowed to separate, and 1.93 M solution of phosgene in toluene (0.85 mL, 1.64 mmol) was added in a single portion via syringe to the lower (organic) phase. Stirring was resumed immediately, and the ice-cooled reaction mix was stirred for 10 min at 600 rpm. The layers were then separated, the aqueous phase was extracted with CH₂Cl₂ (3 x 5 mL), and the combined organic layer was dried over Na₂SO₄, filtered, and concentrated by rotary evaporation. The residue obtained was dissolved in toluene (15 mL), to that was added compound (111) (333 mg, 0.75 mmol). The reaction mixture was further refluxed overnight, evaporated to dryness, diluted with ethyl acetate, washed with 10% aq NaHCO₃ and brine, dried over Na₂SO₄ and evaporated to give crude compound which was purified by column chromatography to give (68%) of desired compound as a white solid;

¹H NMR (200 MHz, CDCl₃) δ (ppm) 9.12 (bs, 1H), 8.04 (d, *J* = 7.82 Hz, 1H), 7.56 (bs, 1H), 7.15-7.37 (m, 12H), 6.91 (bs, 1H), 6.42 (bs, 1H), 6.23 (bs, 1H), 2.28-4.52 (m, 17H), 1.29-1.69 (m, 14H), 0.6-0.77 (m, 16H).

### Example 90: Synthesis of a beta turn model peptide

### Step 1: Boc-Aza-Gly-OEt (Step 1 of Example 73):

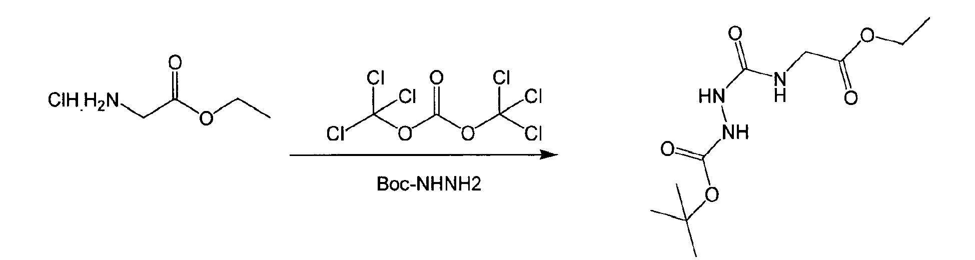

To the solution of triphosgene (3.4 g, 11.46 mmol ) in CH₂Cl₂ (10 mL), a mixture of glycine ethyl ester hydrochloride (4.0 g, 28.66 mmol) and DIEA (14.87 mL, 85.97 mmol) in CH₂Cl₂ (15 mL) was added slowly over a period of 30 min. After a further 15 min of stirring, a solution of Boc-Hydrazine (3.79 g, 28.66 mmol) in CH₂Cl₂ (15 mL) was added in one portion. The reaction mixture was further refluxed for 2 h, evaporated to dryness, diluted with ethyl acetate, washed with 10% aq NaHCO₃ and brine, dried over Na₂SO₄ and evaporated to give 5.6 g of crude compound which was purified by column chromatography to give 3.63 g (48%) of product as a sticky mass which after overnight standing at rt gives off white solid; mp. 137-138°C.

R*_{f}* = 0.29 (5% methanol in CH₂Cl₂)

¹H NMR (200 MHz, CDCl₃) δ (ppm) 7.2 (bs, 1H), 7.05 (s, 1H), 6.2 (t, *J* = 5.46 Hz, 1H), 4.11 (q, *J* = 7.04 Hz, 2H), 3.92 (d, *J* = 5.66 Hz, 2H), 1.39 (s, 9H), 1.2 (t, *J* = 7.04 Hz, 3H).

¹³C NMR (200 MHz, CDCl₃) δ (ppm) 171.07, 158.95, 156.43, 81.51, 61.25, 41.79, 28.09, 14.04.

### Reference:

1994 JOC 1937: Majer, Pavel; Randad, Ramnarayan S. A Safe and Efficient Method for Preparation of N,N'-Unsymmetrically Disubstituted Ureas Utilizing Triphosgene.Journal of Organic Chemistry (1994), 59(7), 1937-8.

### Step 2:

### Boc-Aza-Gly-N(Me)₂ (as in Example 75):

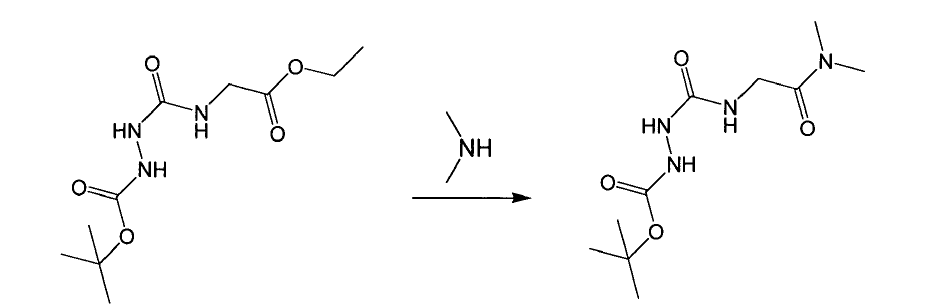

The solution of Boc-Aza-Gly-OEt (Step-1 of Example 73) (3.8 g, 14.54 mmol) in 33% solution of dimethylamine in ethanol (20 mL) was stirred overnight at room temperature. Solvent was evaporated and was well dried under high vacuum to give 3.78 g (100%) of product as an off-white solid; mp. 71-72°C.

R*_{f}* = 0.24 (5% methanol in CH₂Cl₂)

¹H NMR (200 MHz, CDCl₃) δ (ppm) 7.35 (bs, 1H), 7.07 (s, 1H), 6.48 (t, *J* = 4.3 Hz, 1H), 4.0 (d, *J* = 4.3 Hz, 2H), 2.92 (s, 3H), 2.89 (s, 3H), 1.38 (s, 9H).

¹³C NMR (200 MHz, CDCl₃) δ (ppm) 169.15, 158.74, 156.33, 81.08, 41.8, 36.01, 35.64, 28.13.

### Step 3:

### Boc-Pro-Aza-Gly-N(Me)₂ (as in Example 76):

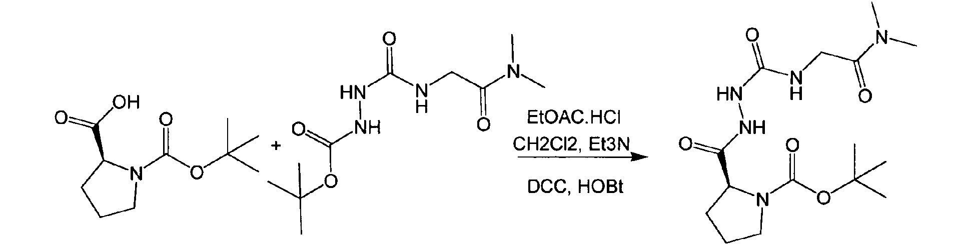

To the solution of Boc-Aza-Gly-N(Me)₂ (Example 75) (1.87 g, 7.2 mmol) in ethyl acetate (10 mL), ethyl acetate saturated with HCl (10 mL) was added slowly with stirring at 0°C. The reaction mixture was stirred at room temperature for 30 min. Solvent was evaporated and the reaction mixture was well dried under high vacuum. In another flask Boc-proline (1.55 g, 7.2 mmol), HOBt (1.02 g, 7.56 mmol) and DCC (1.56 g, 7.56 mmol) were dissolved in dichloromethane (15 mL) and stirred for 15 min at rt, to this a mixture of above hydrochloride salt of JP-129 and triethylamine (3.0 mL, 21.6 mmol) in dichloromethane (15 mL) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was concentrated. The crude compound was purified by column chromatography to give 2.1 g (81%) of product as a white solid; mp. 74-75°C.

R*_{f}* = 0.51 (10% methanol/CH₂Cl₂)

¹H NMR ( 200 MHz, CDCl₃) δ (ppm) 8.89 (bs, 1H), 7.75 (bs, 1H), 6.56 (bs, 1H), 3.87-4.22 (m, 3H), 3.41 (m, 2H), 2.91 (s, 3H), 2.88 (s, 3H), 1.68-2.1 (m, 4H), 1.36 (s, 9H).

¹³C NMR (200 MHz, CDCl₃) δ (ppm) 172.46, 169.06, 158.28, 155.33, 80.33, 58.72, 53.44, 47.07, 41.83, 36.05, 35.71, 28.33, 24.49.

### Step 4:

### t-BuO-Oxalyl-Pro-Aza-Gly-(NMe)₂ (as in Example 77):

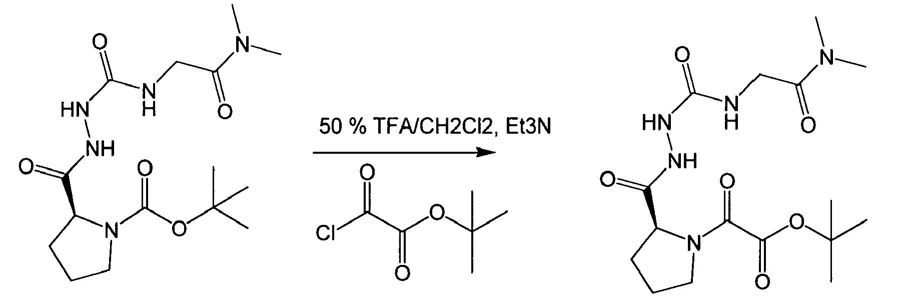

To the solution of Boc-Pro-Aza-Gly-N(Me)₂ (Example 76) (0.72 g, 2.02 mmol) in dichloromethane (10 mL), TFA (10 mL) was added slowly with stirring at 0°C, the reaction mixture was further stirred under argon at room temperature for 30 min. Solvent was evaporated and was well dried under high vacuum. The residue obtained was dissolved in dichloromethane (25 mL), to that was added slowly with stirring triethylamine (0.85 mL, 6.04 mmol) at 0°C, followed by *tert*-butyl oxalyl chloride (0.33 g, 2.02 mmol) and the reaction mixture was further stirred under argon at rt for 30 min. The solvent was evaporated and the crude compound obtained was purified by column chromatography to give 250 mg (32%) of product as white solid. 172-173°C.

R*_{f}* = 0.36 (10% methanol/CH₂Cl₂)

¹H NMR (200 MHz, DMSO-d₆) δ (ppm) 9.78 (bs, 1H), 8.15 (s, 1H), 6.28 (m, 1H), 4.41 (m, 1H), 3.97 (m, 2H), 3.56 (m, 2H), 2.91 (s, 3H), 2.83 (s, 3H), 1.8-2.35 (m, 4H), 1.46 (s, 9H)

### Step 5: (Me)2N-Gly-NProO-NCH(CH₃)₂ (as in Example 78):

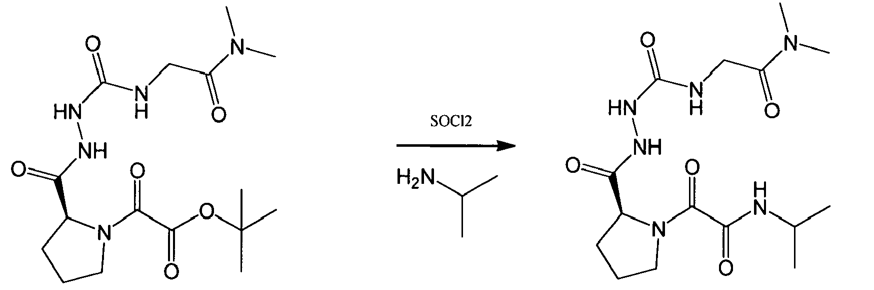

To the solution of *t*-BuO-Oxalyl-Pro-Aza-Gly-(NMe)₂(Example 77) (220 mg, 0.57 mmol) in dichloromethane (5 mL), TFA (5 mL) was added slowly with stirring at 0°C, the reaction mixture was stirred overnight at room temperature. Solvent was evaporated and was well dried under high vacuum. The residue obtained was dissolved DCM (15 mL), to that was added with stirring thionyl chloride (0.21 mL, 2.85 mmol) followed by catalytic DMF (2 drops). The reaction mixture was further stirred overnight under argon at room temp. Solvent was evaporated on rotavap, the excess of thionyl chloride was removed under high vaccum. The residue obtained was dissolved in dichloromethane (15 mL), to this was added slowly with stirring isopropyl amine (0.15 mL, 1.72 mmol) at 0°C. The mixture was diluted with ethyl acetate (30 mL) and washed with 1N HCL (10 mL), followed by 10% NaHCO₃ (10 mL) and brine (10 mL). The ethyl acetate layer was over Na₂SO₄ and concentrated to give 220 mg of crude compound which was purified by column chromatography to give 150 mg (70) of the desired product as a white solid.

¹H NMR ( 200 MHz, CDCl₃) δ (ppm) 9.71 (bs, 1H), 9.56 (bs, 1H), 8.11 (bs, 1H), 6.28 (bs, 1H), 4.34 (m, 1H), 4.05 (m, 2H), 3.94 (m, 1H), 3.6 (m, 2H), 2.92 (s, 3H), 2.89 (s, 3H), 1.87-2.39 (m, 4H), 1.18- 1.3 (m, 6H)

¹³C NMR (200 MHz, CDCl₃) δ (ppm) 170.5, 164.9, 162.1, 162.0, 158.5, 62.7, 46.57, 43.1, 41.1, 36.8, 37.2, 30.1, 25.27, 23.63, 24.1.

### Example 91: Synthesis of Beta sheet mimic

### Step 1: (2-Phenylamino-ethyl)-carbamic acid 9H-fluoren-9-yl-methyl ester (as in Example 82)

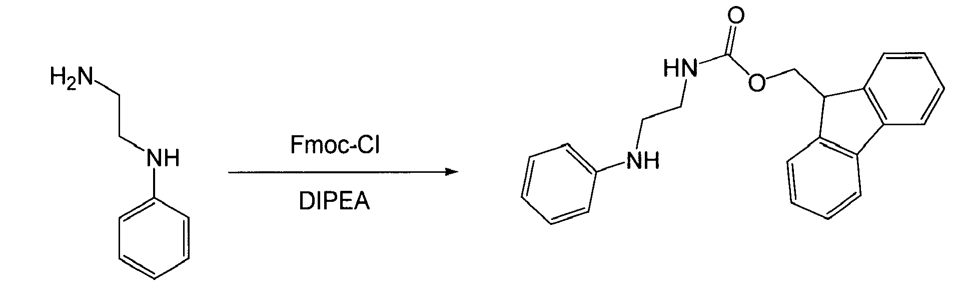

To a mixture of phenyl ethylenediamine (3.0 g, 22.12 mmol), DIPEA (4.65 mL, 26.54 mmol) and DCM (25 mL) was added slowly with stirring and ice-bath cooling a solution of 9-fluorenylmethyl chloroformate (5.72 g, 22.12 mmol) in methylene chloride (25 mL) under argon for a period of 30 min, the reaction mixture was stirred further at room temperature for 15 min. The reaction mixture was then washed with 1N HCL (15 mL), followed by 10% NaHCO₃ (15 mL) and brine (15 mL), dried (Na₂SO₄) and concentrated to give 7.8 g of crude compound which was crystallised from ethyl acetate to give 6.5 g (82%) of pure product as white solid; mp. 174-175°C.

R_{f} = 0.54 (1:2 EtOAc/Pentane)

¹H NMR (200 MHz, DMSO-d₆) δ (ppm) 7.87 (d, *J =* 7.24 Hz, 2H), 7.67 (m, 3H), 7.27-7.43 (m, 9H), 4.2-4.31 (m, 3H), 3.31 (bs, 4H).

¹³C NMR (200 MHz, DMSO-d₆) δ (ppm) 156.07, 143.74, 140.66, 138.6, 138.18, 129.63, 127.58, 127.03, 125.16, 121.09, 120.06, 65.56, 48.46, 46.6, 36.97.

### Step 2: Boc-Aza-(2-Phenylamino-ethyl)-carbamic acid 9H-fluoren-9-ylmethyl ester (as in Example 83):

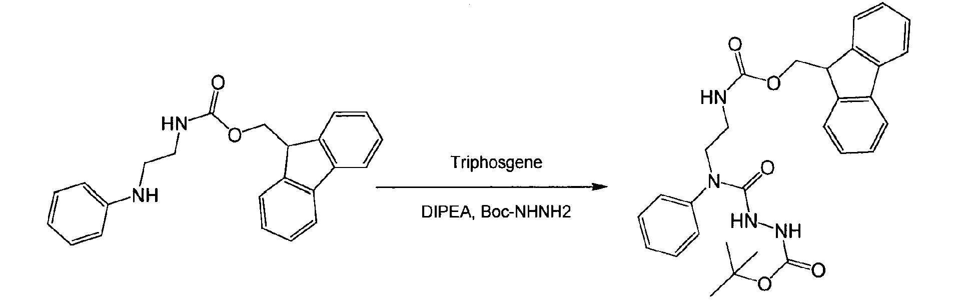

To the solution of triphosgene (578 mg, 1.95 mmol) in CH₂Cl₂ (10 mL), a mixture of (2-Phenylamino-ethyl)-carbamic acid 9H-fluoren-9-ylmethyl ester ((Example 82) (1.79 g, 4.99 mmol) and DIEA (1.0 mL, 5.99 mmol) in CH₂Cl₂ (10 mL) was added slowly over a period of 30 min. After a further 15 min of stirring, a solution of Boc-Hydrazine (990 mg, 7.49 mmol) in CH₂Cl₂ (10 mL) was added in one portion. The reaction mixture was further refluxed for 2 h, evaporated to dryness, diluted with ethyl acetate, washed with 10% aq NaHCO₃ and brine, dried over Na₂SO₄ and evaporated to give 1.9 g of crude compound which was purified by column chromatography to give 1.1 g (42%) of product as a white solid; mp. 69-70°C.

R*_{f}* = 0.51 (5% methano in CH₂Cl₂)

¹H NMR ( 200 MHz, CDCl₃) δ (ppm) 7.67 (d, *J* = 7.22 Hz, 2H), 7.53 (d, *J* = 7.24 Hz, 2H), 7.18-7.42 (m, 9H), 6.33 (bs, 1H), 5.96 (bs, 1H), 5.63 (bs, 1H), 4.23 (d, *J* = 6.84 Hz, 2H), 4.11 (t, J= 6.44 Hz, 1H), 3.77 (m, 2H), 3.33 (m, 2H), 1.37 (s, 9H).

¹³C NMR (200 MHz, CDCl₃) δ (ppm) 157.77, 156.61, 156.28, 144.02, 141.23, 140.22, 130.41, 128.52, 128.27, 127.63, 127.04, 125.23, 119.91, 81.39, 66.85, 49.44, 47.18, 40.3, 28.17.

### Step 3: Preparation of (S)-1-Isopropylaminooxalyl-pyrrolidine-2-carboxylic acid methyl ester (as in Example 84):

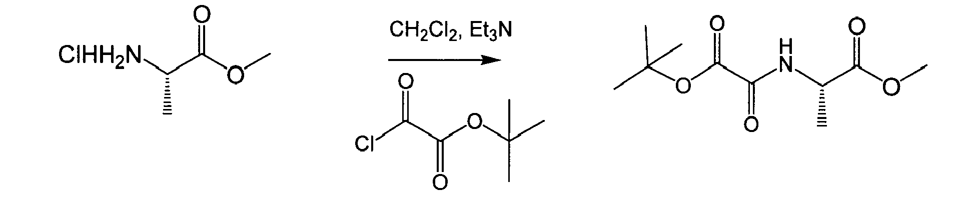

To a solution of L-alanine methylester hydrochloride (3.02 g, 21.62 mmol) in DCM (30 mL) was added slowly with stirring triethylamine (7.15 mL, 64.87 mmol) at 0°C, followed by t-butyl oxalyl chloride (3.74 g, 22.7 mmol). The reaction mixture was further stirred under argon at rt for 30 min. The mixture was diluted with ethyl acetate (50 mL) and washed with 1N HCL (10 mL), followed by 10% NaHCO₃(10 mL) and brine (10 mL). The ethyl acetate layer was dried (Na₂SO₄) and concentrated to give 4.92 g (98%) of pure product as a dark brown thick liquid.

R*_{f}* = 0.51 (1:2 EtOAc/Pentane )

¹H NMR (200 MHz, CDCl₃) δ (ppm) 7.58 (bs, 1H), 4.55 (m, 1H), 3.74 (s, 3H), 1.53 (s, 9H), 1.44 (d, *J* = 7.24 Hz, 3H).

¹³C NMR (200 MHz, CDCl₃) δ (ppm) 172.33, 159.02, 156.8, 84.62, 52.61, 48.42, 27.63, 17.97.

### Step 4: (S)-2-(tert-Butylaminooxalyl-amino)-propionic acid methyl ester (as in Example 85):

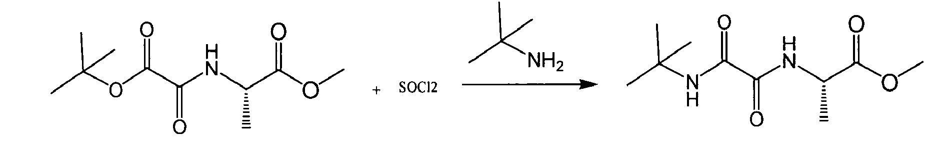

To the solution of Preparation of (S)-1-Isopropyl-aminooxalyl-pyrrolidine-2-carboxylic acid methyl ester (Example 84) (1.71 g, 7.4 mmol) in dichloromethane (10 mL), TFA (10 mL) was added slowly with stirring at 0°C, the reaction mixture was stirred overnight at room temperature. Solvent was evaporated and was well dried under high vacuum to give 1.3 g (100%) of product as a brown thick liquid. The residue obtained was dissolved DCM (30 mL), to that was added with stirring thionyl chloride (2.7 mL, 36.97 mmol) followed by catalytic DMF (2 drops). The reaction mixture was further stirred overnight under argon at room temp. Solvent was evaporated on rotavap, the excess of thionyl chloride was removed under high vacuum. The residue obtained was dissolved in dichloromethane (30 mL), to this was added slowly with stirring isopropyl amine (2.33 mL, 22.18 mmol) at 0°C. The mixture was diluted with ethyl acetate (50 mL) and washed with 1N HCL (10 mL), followed by 10% NaHCO₃ (10 mL) and brine (10 mL). The ethyl acetate layer was over Na₂SO₄ and concentrated to give 1.37 g (80%) of desired compound as an off-white solid; mp. 80-81°C.

R*_{f}* = 0.67 (1:2 EtOAc/Pentane )

¹H NMR ( 200 MHz, CDCl₃) δ (ppm) 7.94 (bs, 1H), 7.25 (bs, 1H), 4.47 (m, 1H), 3.69 (s, 3H), 1.4 (d, *J* = 7.24 Hz, 3H), 1.33 (s, 9H).

¹³C NMR (200 MHz, CDCl₃) δ (ppm) 172.03, 160.5, 158.24, 52.53, 51.46, 48.34, 29.87, 28.2, 17.89.

### Step 5: (S)-2-(tert-Butylaminooxalyl-amino)-propionic acid (as in Example 86):

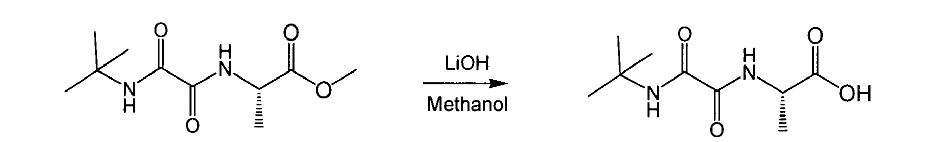

To a solution of (S)-2-(tert-butylaminooxalyl-amino)-propionic acid methyl ester (Example 85) (500 mg, 2.17 mmol) in methanol (5 mL )was added dropwise the aqueous solution of LiOH (62 mg, 2.61 mmol) in 0.5 mL of water. The reaction mixture was stirred further for 20 min at room temperature. Methanol was evaporated and the residue obtained was dissolved in water (10 mL), washed with ether (2 x 5 mL), acidified (pH = 4) with 1N HCl and extracted with ethyl acetate (3 x 105 mL). The combined ethyl acetate layer was washed with brine (2 x 5 mL), dried (Na₂SO₄) and concentrated to give 400 mg (85%) of desired product as a white crystalline solid:; mp 118-119°C.

¹H NMR (200 MHz, Fon+CDCl₃) δ (ppm) 11.1 (bs, 1H), 8.22 (d, J = 7.04 Hz, 1H), 7.43 (s, 1H), 4.5 (m, 1H), 1.45 (d, J = 7.24 Hz, 3H), 1.33 (s, 9H).

¹³C NMR (200 MHz, CDCl₃) δ (ppm) 175.64, 160.17, 158.4, 51.79, 48.32, 28.17, 17.44.

### Step 6: (as in Example 87):

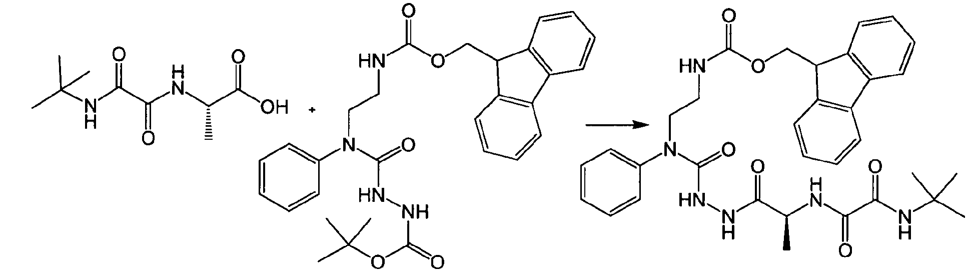

To the solution of Boc-Aza-(2-Phenylamino-ethyl)-carbamic acid 9H-fluoren-9-ylmethyl ester (Example 83) (900 mg, 1.7 mmol) in dichloromethane (10 mL), TFA (10 mL) was added slowly with stirring at 0°C, the reaction mixture was further stirred under argon at room temperature for 30 min (after overnight stirring there is formation of by-product so stirring only for 30 min. is crucial). Solvent was evaporated and was well dried under high vacuum, the residue obtained was dissolved in dichloromethane (15 mL). In another flask (S)-2-(tert-butylaminooxalyl-amino)-propionic acid (Example 86) (377 mg, 1.74 mmol), HOBt (247 mg, 1.83 mmol) and DCC (377 mg, 1.83 mmol ) were dissolved in dichloromethane (15 mL) and stirred for 15 min at rt, to this a mixture of above TFA salt of JP-139 and DIPEA (0.34 mL, 1.92 mmol) in dichloromethane (15 mL) was added and the mixture was stirred overnight at room temperature.

The precipitated DCHU was removed by filtration and the filtrate was evaporated. The additional DCHU was removed by subsequent trituration with cold ethyl acetate and filtration. The ethyl acetate solution was washed with 1N HCl (15 mL), 10 % NaHCO₃ (15 mL), brine (15 mL), dried (Na₂SO₄) and concentrated to give 1.1 g of crude compound which was purified by column chromatography to give 974 mg (90%) of product as a white solid; mp. 48-49°C.

R_{f} = 0.42 (5% methanol in CH₂Cl₂)

¹H NMR ( 200 MHz, CDCl₃) δ (ppm) 9.25 (bs, 1H), 8.35 (d, *J* = 8.6 Hz, 1H), 7.75 (d, *J* = 7.22 Hz, 2H), 7.58 (d, *J* = 7.24 Hz, 2H), 7.25-7.45 (m, 10H), 6.48 (bs, 1H), 5.88 (bs, 1H), 4.55-4.75 (m, 1H), 4.14-4.29 (m, 3H), 4.25 (m, 2H), 3.4 (m, 2H), 1.45 (d, *J* = 7.04 Hz, 3H),1.35 (s, 9H).

¹³C NMR (200 MHz, CDCl₃) δ (ppm) 170.52, 160.5, 158.29, 157.26, 156.57, 143.95, 141.22, 139.94, 130.46, 128.74, 128.1, 127.63, 127.02, 125.16, 119.92, 66.78, 51.53, 49.64, 47.74, 47.16, 39.78, 28.26, 17.67.

### Step 7: (as in Example 88):

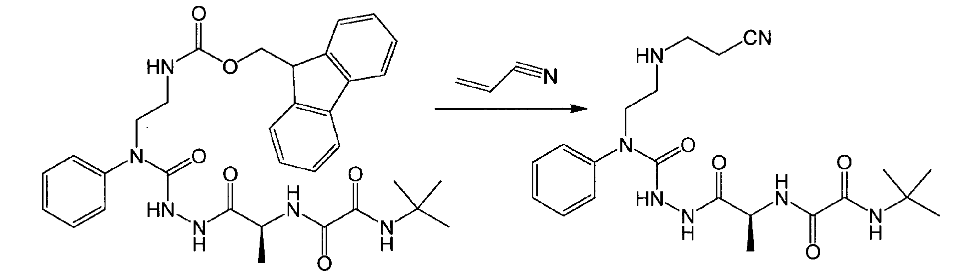

The solution of compound (Example 87) (915 mg, 1.49 mmol) in 10% piperidine in CH₂Cl₂ (15 mL) was stirred at room temperature for 20 min. Solvent was evaporated and the reaction mixture was well dried under high vacuum to remove the traces of piperidine. The residue obtained was crystallised from ethylacetate - pentane to give 575 mg (98%) of free amine. The free amine was dissolved in methanol (15 mL), to that was added acrylonitrile (0.15 mL, 2.23 mmol) and the reaction mixture was stirred overnight at room temperature under argon. The solvent was evaporated and the crude compound was purified by column chromatography to give 333 mg (50%) of product as a white solid; mp. 62-63°C.

R*_{f}* = 0.42 (5% methanol in CH₂Cl₂)

¹H NMR ( 200 MHz, CDCl₃) δ (ppm) 8.14 (d, *J* = 8.4 Hz, 1H), 7.21-7.43 (m, 6H), 4.4-4.55 (m, 1H), 3.72 (t, *J* = 6.06 Hz, 2H), 2.84 (t, *J* = 6.54 Hz, 2H), 2.71 (t, *J* = 6.06 Hz, 2H), 2.41 (t, *J* = 6.65 Hz, 2H), 1.37 (d, *J* = 7.04 Hz, 3H),1.3 (s, 9H).

¹³C NMR (200 MHz, CDCl₃) δ (ppm) 170.51, 160.4, 158.23, 157.09, 140.11, 130.27, 128.48, 128.23, 118.81, 51.51, 49.52, 47.69, 47.01, 44.83, 28.25, 18.61, 17.8.

### Step 8: (as in Example 89):

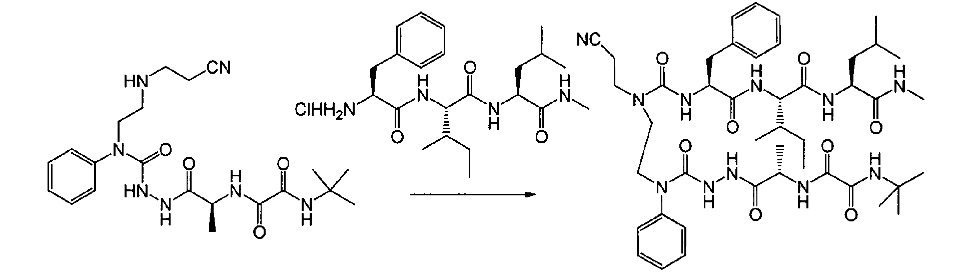

A 25 mL, one necked, round-bottomed flask, was charged with hydrochloride salt of compound (Example 88) (363 mg, 0.82 mmol), 10 mL of CH₂Cl₂, and 10 mL of saturated aqueous NaHCO₃. The biphasic mixture was cooled to 0°C in an ice bath. Stirring was stopped, the layers were allowed to separate, and 1.93 M solution of phosgene in toluene (0.85 mL, 1.64 mmol) was added in a single portion via syringe to the lower (organic) phase. Stirring was resumed immediately, and the ice-cooled reaction mix was stirred for 10 min at 600 rpm. The layers were then separated, the aqueous phase was extracted with CH₂Cl₂ (3 x 5 mL), and the combined organic layer was dried over Na₂SO₄, filtered, and concentrated by rotary evaporation. The residue obtained was dissolved in toluene (15 mL), to that was added compound (111) (333 mg, 0.75 mmol). The reaction mixture was further refluxed overnight, evaporated to dryness, diluted with ethyl acetate, washed with 10% aq NaHCO₃ and brine, dried over Na₂SO₄ and evaporated to give crude compound which was purified by column chromatography to give (68%) of desired compound as a white solid.

¹H NMR ( 200 MHz, CDCl₃) δ (ppm) 9. 12 (bs, 1H), 8.04 (d, *J* = 7.82 Hz, 1H), 7.56 (bs, 1H), 7.15-7.37 (m, 12H), 6.91 (bs, 1H), 6.42 (bs, 1H), 6.23 (bs, 1H), 2.28-4.52 (m, 17H), 1.29-1.69 (m, 14H), 0.6-0.77 (m, 16H).

## Claims

1. Compound of the general formula 1, 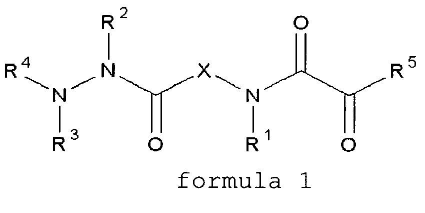 wherein X is the connection between the CO-hydrazine and the NR¹-oxalic acid, oxalic ester or oxalic amide group and is either a bond, 5-20-membered heteroaryl, or an optionally substituted group selected from C₃₋₂₀-cycloalkyl, 3-20-membered heterocyclyl, and linear or branched C₁₋₂₀-alkyl, C₂₋₂₀-alkenyl or C₂₋₂₀-alkinyl; and
R⁵ is selected from -OR¹⁰ and -NR¹⁰R¹¹, or R⁵ can cooperate with R² or R³ to form a bond or a 8 to 10 membered heterocyclic ring; and
R³ and R⁴ together may constitute a double bond to a group R¹²;
R¹, R², R³, R⁴, R¹⁰, R¹¹ and R¹² are optionally substituted and independently selected from H, C₃₋₁₄-cycloalkyl, C₅₋₁₄-aryl, 3-14-membered heterocyclyl or heteroaryl, linear or branched C₁₋₁₄-alkyl, C₂₋₁₄-alkenyl, C₂₋₁₄-alkinyl; and
optionally at least two of R¹, R², R³, R⁴ and X can cooperate to form a 3 to 20-membered cycloalkyl or heterocycloalkyl ring; or an ester, amide, salt, stereoisomer or racemate therefrom;
with the proviso that in the case of X being CH₂ R³ and R⁴ each are bound by single bonds and R² is selected from H, C₃₋₂₀-cycloalkyl, C₅₋₂₀-aryl, 3-20-membered heterocyclyl or heteroaryl, linear or branched C₂₋₂₀-alkenyl, C₂₋₂₀-alkinyl, or unsubstituted C₁₋₄-alkyl, and in the case of X being a bond one of R³ or R⁴ forms an amide, and in the case of X being heteroaryl X does not cooperate to form a heterocycloalkyl ring with R³, R⁴ or R⁵, and in the case of X and R¹ cooperating to form a cycloalkyl ring R⁵ does not comprise a further hydrazine group, R² does not form an aromatic ring with neither R³ or R⁴ and neither R³ nor R⁴ comprise sulphur.

2. Compound according to claim 1, **characterized in that** X is a chemical group of one of formulas 2-6, 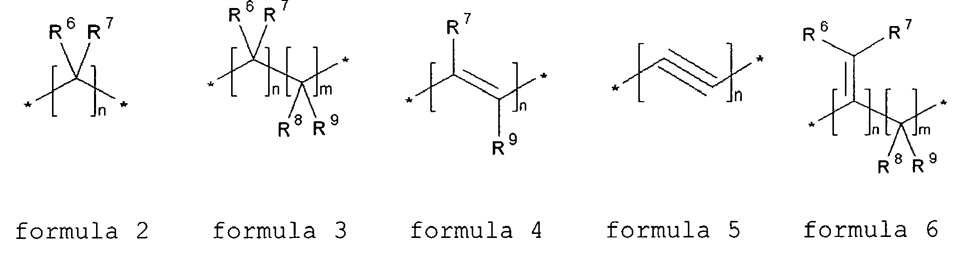 wherein R⁶, R⁷, R⁸ and R⁹ are optionally substituted and independently selected from the groups of H, C₃₋₁₄-cycloalkyl, C₅₋₁₂ aryl, 3-12-membered heterocyclyl or heteroaryl and linear or branched C₁₋₁₄-alkyl, C₂₋₁₄-alkenyl or C₂₋₁₄-alkynyl; and
optionally at least two of R⁶, R⁷, R⁸ and R⁹ can cooperate to form a 3 to 12-membered cycloalkyl or heterocycloalkyl ring;
and n and m are independent integers between 0 and 5.

3. Compound according to claim 1 or 2, **characterized in that** X is an preferably alpha, beta or gamma NR¹-oxalic acid or ester bound group, selected from C₁₋₂-alkyl, guanidinylbutyl, 2-methyl-butyl, phenylethyl, p-hydroxyphenylethyl, indole-3-ylethyl, hydroxyethyl, methylthiopropyl, thioethyl, C₂₋₃-alkyl acid, C₂₋₃-alkyl acidamide, aminopentyl, , 4-imidazolylethyl, or X and R¹ cooperate to form a butyl group, forming a pyrrolidine ring with the nitrogen of the NR¹-oxalic acid or ester group.

4. Compound according to any one of claims 1 to 3, **characterized in that** X is an alpha -CHR¹³- group, wherein R¹³ is either H or the side chain of an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine and valine, preferably the C-alpha L-enantiomer therefrom.

5. Compound according to claim 1, **characterized in that** X is selected from O-, S-, N- or P- heterosubstituted 3-20-membered O-, S-, N- or P- heterosubstituted heteroaryl or from the group of optionally substituted heterocycloalkyl, and linear or branched 1-20-membered heteroalkyl, 2-20-membered heteroalkenyl or heteroalkinyl.

6. Compound according to claim 1, wherein R³ and R⁵ form a bond resulting in a heterocylic compound of the general formula 10, 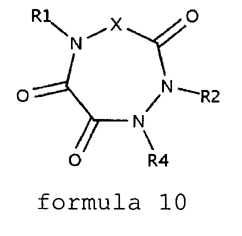 wherein X, R¹, R² and R⁴ are defined as in claim 1.

7. Compound according to any one of claims 1 to 6, **characterized in that** the ester or amide is with a protecting or activating group, preferably selected from Boc, Fmoc, CBz, Alloc, carbonyl, sulfonyl, sulfinyl, phosphoryl, phosphinyl and an amino acid.

8. Compound according to claim 7, **characterized in that** the protecting group replaces R³, R⁴, R⁵ or R¹⁰.

9. Method for the manufacture of a compound according to any one of claims 1 to 8, **characterized in that** a compound according to formula 7 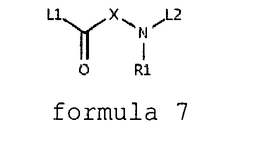 is contacted by a hydrazine or hydrazine derivative according to formula 8 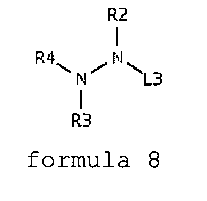 and an oxalic acid, ester or derivative according to formula 9, 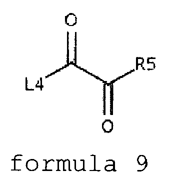 in any order, wherein X, R¹, R², R³, R⁴ and R⁵ are defined as in claim 1 and L1, L2, L3 and L4 are independent arbitrary leaving groups or hydrogen, preferably L4 is Cl or OH, an activated ester or an anhydride.

10. Method according to claim 9, **characterized in that** one of R³ or R⁴ is a protecting group, preferably Fmoc.

11. Peptide mimetic comprising at least a compound according to the general formula 1, 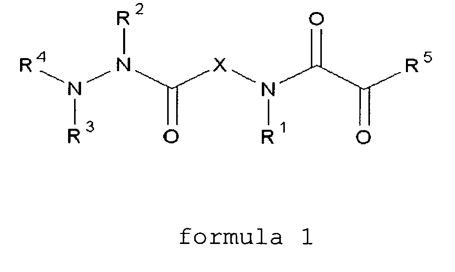 wherein X is the connection between the CO-hydrazine and the NR¹-oxalic acid or ester group and is either a bond, 5-20-membered heteroaryl or aryl, or an optionally substituted group selected from C₃₋₂₀-cycloalkyl, 3-20-membered heterocyclyl, and linear or branched C₁₋₂₀-alkyl, C₂₋₂₀-alkenyl or C₂₋₂₀-alkinyl; and
R⁵ is selected from -OR¹⁰ and -NR¹⁰R¹¹, or R⁵ can cooperate with R² or R³ to form a bond or a 8 to 10 membered heterocyclic ring; and
R³ and R⁴ together may constitute a double bond to a group R¹²;
R¹, R², R³, R⁴, R¹⁰, R¹¹ and R¹² are optionally substituted and independently selected from H, C₃₋₁₄-cycloalkyl, C₅₋₁₄-aryl, 3-14-membered heterocyclyl or heteroaryl, linear or branched C₁₋₁₄-alkyl, C₂₋₁₄-alkenyl, C₂₋₁₄-alkinyl; and
optionally at least two of R¹, R², R³, R⁴ and X can cooperate to form a 3 to 12 membered cycloalkyl or heterocycloalkyl ring; or an ester, amide, salt, stereoisomer or racemate therefrom;
as a molecular part, and a natural or unnatural amino acid or an additional amino acid mimetic, preferably bound by an amide bond.

12. Peptide mimetic according to claim 11, which mimics the three dimensional structure of an alpha-helix, beta-sheet or turn motif.

13. Method for the synthesis of a peptide mimetic according to claim 11 or 12 using a compound of the general formula 1, as defined in claim 11, wherein a reaction target comprising an amine is contacted with the compound according to formula 1, and R³ or R⁴ constitutes an amide with a protecting group, preferably Fmoc.

14. Method according to claim 13 further comprising the step of removing the R³ or R⁴ protecting group.

15. Method according to claim 14, wherein an amino acid or amino acid mimetic, preferably with a protected amino group, is contacted with the compound according to formula 1, preferably after the step of removing the R³ or R⁴ protecting group.

16. Method according to any one of claims 13 to 15, **characterized in that** the reaction target is solid, preferably a solid resin.

17. Peptide, protein or protein fragment comprising the compound of the general formula 1, as defined in claim 11, as covalently bound insert or replacement at any position of the protein amino acid sequence.

18. Method for the manufacture of a peptide, protein or protein fragment according to claim 17, comprising the step of ligating a compound of formula 1, as defined in claim 11, to a peptide, protein or protein fragment.

19. Method according to claim 18 further comprising the step of ligating a further amino acid, peptide, protein or protein fragment to the compound of formula 1, as defined in claim 11.

20. Use of a compound according to any one of claims 1 to 8 as spacer moiety.
